(19) Europäisches Patentamt
European Patent Office
Office européen des brevets

(11) **EP 2 270 113 A1**

(12) **EUROPEAN PATENT APPLICATION**
published in accordance with Art. 153(4) EPC

(43) Date of publication:
**05.01.2011 Bulletin 2011/01**

(21) Application number: **09727136.5**

(22) Date of filing: **30.03.2009**

(51) Int Cl.:
***C09K 3/00*** *(2006.01)* ***C07D 409/14*** *(2006.01)*
***C07D 413/14*** *(2006.01)* ***C08K 5/45*** *(2006.01)*
***C08L 101/00*** *(2006.01)*

(86) International application number:
**PCT/JP2009/056542**

(87) International publication number:
**WO 2009/123142 (08.10.2009 Gazette 2009/41)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **31.03.2008 JP 2008091837**

(71) Applicant: **FUJIFILM Corporation**
**Tokyo 106-8620 (JP)**

(72) Inventors:
- **FURUKAWA, Kazushi**
**Odawara-shi**
**Kanagawa**
**2500001 (JP)**
- **KIMURA, Keizo**
**Odawara-shi**
**Kanagawa 250-0001 (JP)**
- **TAKESHIMA, Youichiro**
**Odawara-shi**
**Kanagawa 250-0001 (JP)**
- **AMASAKI, Ichiro**
**Odawara-shi**
**Kanagawa 250-0001 (JP)**

(74) Representative: **HOFFMANN EITLE**
**Patent- und Rechtsanwälte**
**Arabellastraße 4**
**81925 München (DE)**

(54) **ULTRAVIOLET ABSORBENT COMPOSITIONS**

(57) An ultraviolet absorbent composition, comprising:

at least one kind of ultraviolet absorbent (A) that is a compound represented by the following Formula (1); and at least one kind of ultraviolet absorbent (B) that is a compound where absorbance at 320 nm is 20% or more of absorbance at absorption maximum wavelength in the range of from 270 nm to 400 nm and the absorption maximum wavelength is 380 nm or less:

Formula (1)

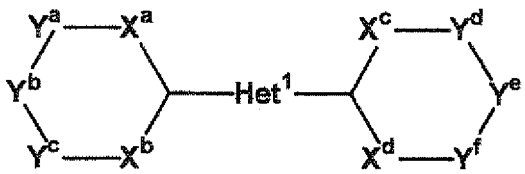

wherein $Het^1$ represents a bivalent five- or six-membered aromatic heterocyclic residue; the aromatic heterocyclic residue may have a substituent;
$X^a$, $X^b$, $X^c$ and $X^d$ each independently represent a heteroatom; $X^a$ to $X^d$ may have a substituent;
$Y^a$, $Y^b$, $Y^c$, $Y^d$, $Y^e$ and $Y^f$ each independently represent a heteroatom or a carbon atom; $Y^a$ to $Y^f$ may have a substituent; and the rings bound to $Het^1$ may have a double bond at any position.

EP 2 270 113 A1

[Fig.1]

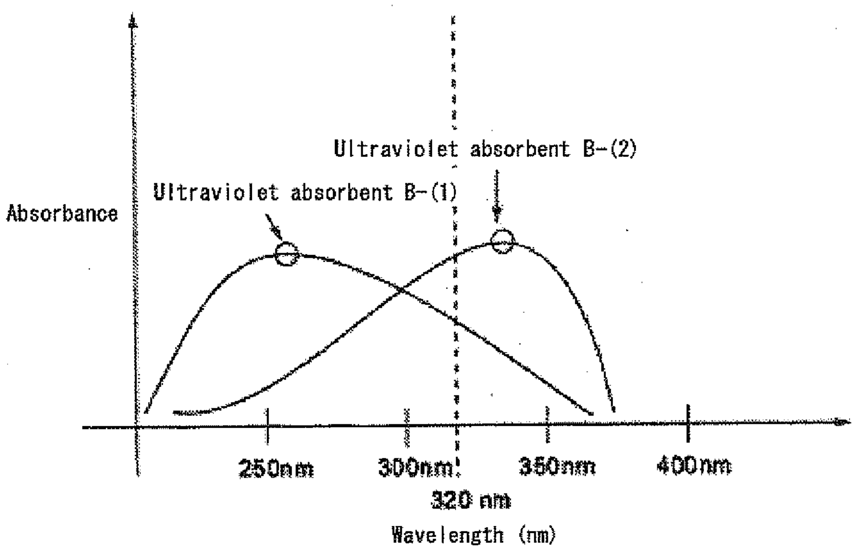
Ultraviolet absorbent B-(2)
Ultraviolet absorbent B-(1)
Absorbance
250nm
300nm
350nm
400nm
320 nm
Wavelength (nm)

## Description

### TECHNICAL FIELD

The present invention relates to an ultraviolet absorbent composition.

### BACKGROUND ART

Ultraviolet absorbents have been used in combination with various resins for providing the resins with ultraviolet-absorptivity. The ultraviolet absorbent is conventionally added to a resin for the purpose of improving stability of the resin, and the significance of cutting the light in a long-wavelength ultraviolet (UV-A) range is scarcely known. Both inorganic and organic ultraviolet absorbents are used as the ultraviolet absorbent. The inorganic ultraviolet absorbents (see, for example, Patent Documents 1 to 3) are superior in durability such as weather resistance and heat resistance. However, a freedom in selecting the compound is limited, because the absorption wavelength is determined by the band gap of the compound. In addition, there is no inorganic absorbent that absorbs the light in a long-wavelength ultraviolet (UV-A) range of 320 to 400 nm. And any such absorbent that absorbs long-wavelength ultraviolet would have color because it would have absorption also in the visible range. It is known that a film having a shielding effect over a wide ultraviolet range can be obtained by coating a cerium oxide-based ultraviolet-shielding agent that blocks the UV-A range onto the surface of a specific titanic acid having a UV-B range blocking property (see, for example, Patent Document 4). In contrast, a freedom in designing a structure of absorbents is much wider for organic ultraviolet absorbents, and thus, it is possible to obtain absorbents having various absorption wavelengths by devising the structure of the absorbents.

So far, various organic ultraviolet absorbent systems have been studied, and for absorption in the long-wavelength ultraviolet range, it is conceivable either to use an absorbent having the wavelength of maximal absorption in the long-wavelength ultraviolet range or to use an absorbent in a high concentration. However, the absorbents described in, for example, Patent Documents 5 and 6, which have the wavelength of maximal absorption in the long-wavelength ultraviolet range are inferior in light stability, and their absorption capacity declines over time.

In contrast, benzophenone- and benzotriazole-based ultraviolet absorbents have relatively higher light stability, and increase in concentration or film thickness leads to relatively clean blocking of the light in the longer-wavelength range (see, for example, Patent Documents 7 and 8). However, when such an ultraviolet absorbent is applied as mixed with a resin or the like, a film thickness is limited to several tens of μm at the most. For utilizing the film thickness to block the light in the longer-wavelength range, it is necessary to add the ultraviolet absorbent to a considerably high concentration. However, simple increase in concentration of ultraviolet absorbent only results in problems of precipitation of the absorbent and bleed-out of the absorbent during long-term use. In addition, an ultraviolet absorbent having the wavelength of maximal absorption in the long-wavelength ultraviolet range but also having absorption in the range of 400 nm or more becomes yellowish, and a tone of a color image after transmission is deteriorated. This phenomenon become distinct problem in the case of ading the absorbent in a high concentration. Therefore, there is a need for an ultraviolet absorbent that blocks the light in a wide ultraviolet range and yet has no absorption in the visible range.

[Patent Document 1] JP-A-5-339033 ("JP-A" means unexamined published Japanese patent application)
[Patent Document 2] JP-A-5-345639
[Patent Document 3] JP-A-6-56466
[Patent Document 4] JP-A-2006-316107
[Patent Document 5] JP-A-6-145387
[Patent Document 6] JP-A-2003-177235
[Patent Document 7] JP-T-2005-517787 ("JP-T" means published Japanese translation of PCT application)
[Patent Document 8] JP-A-7-285927

### SUMMARY OF INVENTION

The present invention addresses to provide an ultraviolet absorbing dye mixture that has an ultraviolet absorptive capacity in a wide wavelength range, and that has a stain (color) suppressed as low as possible while cutting even an UV light of a longer wavelength, and that is significantly excellent in light resistance (light stability), and that is able to impart an ultraviolet absorptive capacity in a wide wavelength-range and other properties to a resin or the like in an effective manner when added to the resin or the like.

The inventors of the present invention conducted intensive studies on compounds having absorption in the ultraviolet range, and also studies into making the compounds block the light in the wider ultraviolet range and the UV light in the long-wavelength ultraviolet range more effectively and yet have no absorption in the visible range. As a result, the inventors found that although it is theoretically possible to achieve a balance between the above-described both

tasks in the case of using a linked ultraviolet absorbent in which plural ultraviolet absorbents are linked, it is not easy to solve the above problems with a single molecular compound that does not have a plurality of ultraviolet-absorbing structures. Namely, when attempting to block an entire ultraviolet renge with a single molecule ultraviolet absorbent, the absorption intensity mostly becomes smaller, and thus, increase in addition amount of the absorbent is needed to block the ultraviolet light effectively. This lead to bleed-out, and is undesirable. The compound having absorption in a wide wavelength range has broad absorption spectra with its wavelength of maximal absorption at the center. Thus, when a wavelength range to be blocked reliably and a wavelength range to be transmitted reliably are close to each other, it is quite difficult to satisfy both requirements at the same time. On the contrary, the compound having sharp absorption blocks the light only in a narrow range with its wavelength of maximal absorption at the center.

Based on these findings, it might be thought that it is possible to provide an ultraviolet absorbent composition blocking the light in the entire ultraviolet range and the light in the long-wavelength ultraviolet range effectively, by using an ultraviolet absorbent that does not have absorption on the long-wavelength side of the visible range but has sufficient absorption in the ultraviolet range, i.e., an ultraviolet absorbent having a steep spectrum in the long-wavelength range for absorption in the long-wavelength ultraviolet range, and additionally another ultraviolet absorbent for absorption in the other range where absorption is insufficient. From the past, plural ultraviolet absorbents have been used in combination. However, it is not known that an effective blocking of light in the ultraviolet range can be achieved by using plural ultraviolet absorbents each having a specifically shaped absorption spectrum in combination. Further, combination use of different kinds of UV agents may form a complex, which sometimes causes a problem such as deterioration of light resistance and discoloration.

The present invention was completed based on the above described findings.

The present invention provides the following means:

<1> An ultraviolet absorbent composition, comprising:

at least one kind of ultraviolet absorbent (A) that is a compound represented by the following Formula (1); and at least one kind of ultraviolet absorbent (B) that is a compound where absorbance at 320 nm is 20% or more of absorbance at absorption maximum wavelength in the range of from 270 nm to 400 nm and the absorption maximum wavelength is 380 nm or less:

Formula (1)

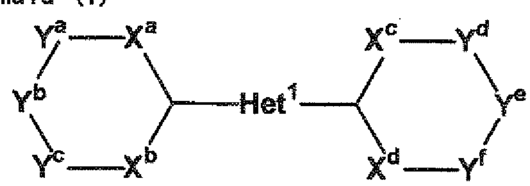

wherein $Het^1$ represents a bivalent five- or six-membered aromatic heterocyclic residue; the aromatic heterocyclic residue may have a substituent;

$X^a$, $X^b$, $X^c$ and $X^d$ each independently represent a heteroatom; $X^a$ to $X^d$ may have a substitutuent;

$Y^a$, $Y^b$, $Y^c$, $Y^d$, $Y^e$ and $Y^f$ each independently represent a heteroatom or a carbon atom; $Y^a$ to $Y^f$ may have a substitutuent; and

the rings bound to $Het^1$ may have a double bond at any position.

<2> The ultraviolet absorbent composition described in the above item <1>, wherein, in the above Formula (1), at least one of the ring formed by $X^a$, $X^b$, $Y^a$ to $Y^c$ and the carbon atom and the ring formed by $X^c$, $X^d$, $Y^d$ to $Y^f$ and the carbon atom is a fused ring.

<3> The ultraviolet absorbent composition described in the above item <1> or <2>, wherein, in the above Formula (1), at least one of the ring formed by $X^a$, $X^b$, $Y^a$ to $Y^c$ and the carbon atom and the ring formed by $X^c$, $X^d$, $Y^d$ to $Y^f$ and the carbon atom is not a perimidine ring.

<4> The ultraviolet absorbent composition described in any one of the above items <1> to <3>, wherein the compound represented by the above Formula (1) is a compound represented by the following Formula (2):

Formula (2)

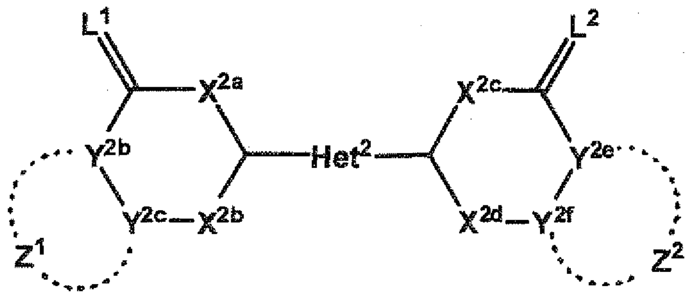

wherein $Het^2$ is the same as $Het^1$ in the above Formula (1);
$X^{2a}$, $X^{2b}$, $X^{2c}$ and $X^{2d}$ are the same as $X^a$, $X^b$, $X^c$ and $X^d$ in the above Formula (1), respectively;
$Y^{2b}$, $Y^{2c}$, $Y^{2e}$ and $Y^{2f}$ are the same as $Y^b$, $Y^c$, $Y^e$ and $Y^f$ in the above Formula (1), respectively;
$L^1$ and $L^2$ each independently represent an oxygen atom, a sulfur atom or $=NR^a$ ($R^a$ represents a hydrogen atom or a monovalent substituent); and
$Z^1$ and $Z^2$ each independently represent a group of atoms needed to form a four- to eight-membered ring together with $Y^{2b}$ and $Y^{2c}$ or $Y^{2e}$ and $Y^{2f}$.

<5> The ultraviolet absorbent composition described in the above item <4>, wherein the compound represented by the above Formula (2) is a compound represented by the following Formula (3):

Formula (3)

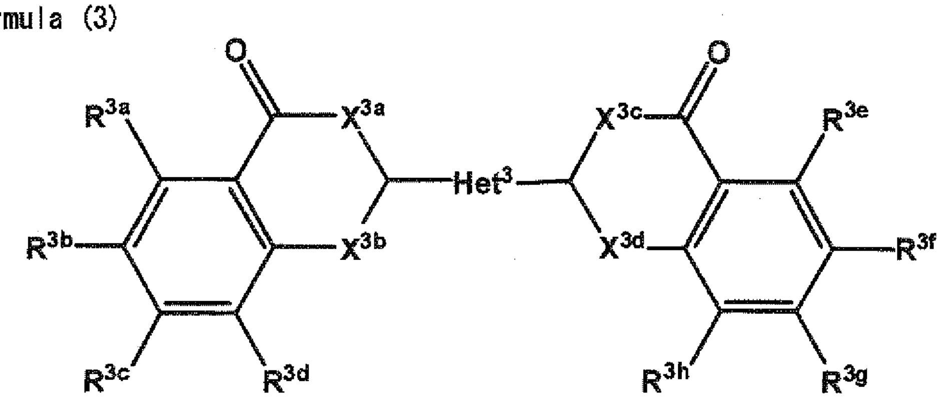

wherein $Het^3$ is the same as $Het^2$ in the above Formula (2);
$X^{3a}$, $X^{3b}$, $X^{3c}$ and $X^{3d}$ are the same as $X^{2a}$, $X^{2b}$, $X^{2c}$ and $X^{2d}$ in the above Formula (2), respectively; and
$R^{3a}$, $R^{3b}$, $R^{3c}$, $R^{3d}$, $R^{3e}$, $R^{3f}$, $R^{3g}$ and $R^{3h}$ each independently represent a hydrogen atom or a monovalent substituent.

<6> The ultraviolet absorbent composition described in the above item <5>, wherein the compound represented by the above Formula (3) is a compound represented by the following Formula (4):

Formula (4)

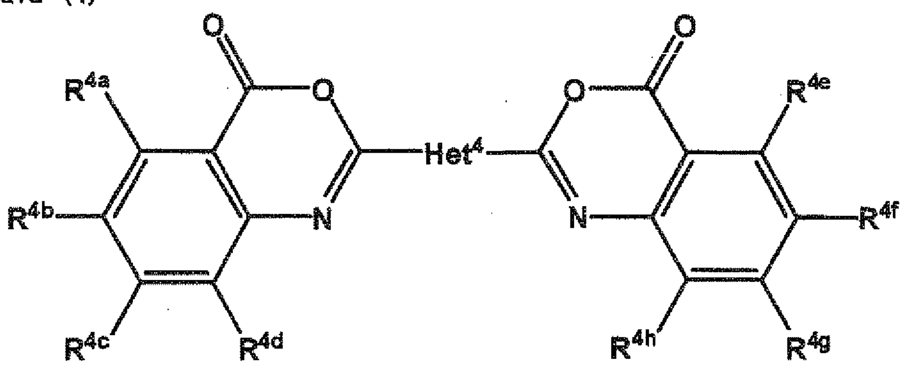

wherein $Het^4$ is the same as $Het^3$ in the above Formula (3); and $R^{4a}$, $R^{4b}$, $R^{4c}$, $R^{4d}$, $R^{4e}$, $R^{4f}$, $R^{4g}$ and $R^{4h}$ are the same as $R^{3a}$, $R^{3b}$, $R^{3c}$, $R^{3d}$, $R^{3e}$, $R^{3f}$, $R^{3g}$ and $R^{3h}$ in the above Formula (3), respectively.

<7> The ultraviolet absorbent composition described in the above item <6>, wherein the compound represented by the above Formula (4) is a compound represented by the following Formula (5):

Formula (5)

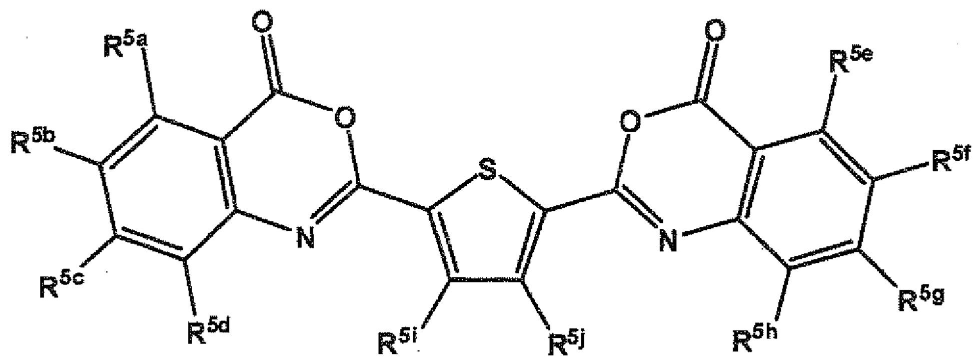

wherein $R^{5a}$, $R^{5b}$, $R^{5c}$, $R^{5d}$, $R^{5e}$, $R^{5f}$, $R^{5g}$ and $R^{5h}$ are the same as $R^{4a}$, $R^{4b}$, $R^{4c}$, $R^{4d}$, $R^{4e}$, $R^{4f}$, $R^{4g}$ and $R^{4h}$ in the above Formula (4), respectively; and $R^{5i}$ and $R^{5j}$ each independently represent a hydrogen atom or a monovalent substituent.

<8> The ultraviolet absorbent composition described in any one of the above items <1> to <7>, wherein a ratio of the ultraviolet absorbent (A) and the ultraviolet absorbent (B) is in the range of from 10:1 to 1:10.
<9> An ultraviolet absorbent dispersion, comprising the ultraviolet absorbent composition described in any one of the above items <1> to <8>.
<10> An ultraviolet absorbent solution, comprising the ultraviolet absorbent composition described in any one of the above items <1> to <8>.
<11> A polymer material, comprising the ultraviolet absorbent composition described in any one of the above items <1> to <8>.

The ultraviolet absorbent composition of the present invention is excellent in an ultraviolet absorptive capacity in a wide wavelength range. Further, the ultraviolet absorbent composition of the present invention is able to impart an ultraviolet absorptive capacity in a wide wavelength range to the resin or the like in an effective manner when added to the resin or the like. Further, the ultraviolet absorbent composition of the present invention cuts (blocks) a long-wavelength ultraviolet with a good sharp shape at a bottom of absorption spectrum. As a result, a balance between cutting of the long-wavelengthrange of ultraviolet and minimal stain is achieved. Further, a combination of specific ultraviolet absorbents

makes it possible to improve compatibility with a resin whereby a problem of bleed-out that is ordinarily caused when an ultraviolet absorbent is added to the resin can be prevented. As a result, the ultraviolet absorbent composition of the present invention is significantly excellent in light resistance of a resin to which the ultraviolet absorbents have been added as well as the ultraviolet absorbents themselves.

BRIEF DESCRIPTION OF THE DRAWINGS

[Fig. 1] Fig. 1 shows preferred absorption spectrum of the ultraviolet absorbent (B) for use in the present invention.

Other and further features and advantages of the present invention will appear more fully from the following description, taking the accompanying drawing into consideration.

DETAILED DESCRIPTION OF THE INVENTION

The present invention is explained in detail below.
In the present specification, the aliphatic group means an alkyl group, a substituted alkyl group, an alkenyl group, a substituted alkenyl group, an alkynyl group, a substituted alkynyl group, an aralkyl group, and a substituted aralkyl group. The aforementioned alkyl group may have a branch or may form a ring (i.e. a cycloalkyl group). The alkyl group preferably has 1 to 20 carbon atoms, and more preferably 1 to 18 carbon atoms. The alkyl moiety in the aforementioned substituted alkyl group is the same as the above mentioned alkyl group. The aforementioned alkenyl group may have a branch or may form a ring (i.e. a cycloalkenyl group). The alkenyl group has preferably 2 to 20 carbon atoms, and more preferably 2 to 18 carbon atoms. The alkenyl moiety in the aforementioned substituted alkenyl group is the same as the above mentioned alkenyl group. The aforementioned alkynyl group may have a branch or may form a ring (i.e. a cycloalkynyl group). The alkynyl group has preferably 2 to 20 carbon atoms, and more preferably 2 to 18 carbon atoms. The alkynyl moiety in the aforementioned substituted alkynyl group is the same as the above mentioned alkynyl group. The alkyl moiety in the aforementioned aralkyl group and substituted aralkyl group is the same as the above mentioned alkyl group. The aryl moiety in the aforementioned aralkyl group and substituted aralkyl group is the same as the aryl group mentioned below.
Specific examples of the substituent in the alkyl moiety of the substituted alkyl group, the substituted alkenyl group, the substituted alkynyl group, and the substituted aralkyl group include: a halogen atom (e.g. a chlorine atom, a bromine atom, or an iodine atom); an alkyl group which represents a substituted or unsubstituted linear, branched, or cyclic alkyl group, and which includes an alkyl group (preferably an alkyl group having 1 to 30 carbon atoms (a methyl group, an ethyl group, an n-propyl group, an isopropyl group, a t-butyl group, an n-octyl group, an eicosyl group, a 2-chloroethyl group, a 2-cyanoethyl group, or a 2-ethylhexyl group), a cycloalkyl group (preferably a substituted or unsubstituted cycloalkyl group having 3 to 30 carbon atoms, e.g. a cyclohexyl group, a cyclopentyl group, or a 4-n-dodecylcyclohexyl group), a bicycloalkyl group (preferably a substituted or unsubstituted bicycloalkyl group having 5 to 30 carbon atoms, i.e. a monovalent group obtained by removing one hydrogen atom from a bicycloalkane having 5 to 30 carbon atoms, e.g. a bicyclo[1,2,2]heptan-2-yl group or a bicyclo[2,2,2]octan-3-yl group), and a higher ring structure such as tricyclo are included; and an alkyl group in a substituent explained below (e.g. an alkyl group in an alkylthio group) represents such an alkyl group of the above concept];
an alkenyl group [a substituted or unsubstituted linear, branched, or cyclic alkenyl group, and which includes an alkenyl group (preferably a substituted or unsubstituted alkenyl group having 2 to 30 carbon atoms, e.g. a vinyl group, an allyl group, a prenyl group, a geranyl group, or an oleyl group), a cycloalkenyl group (preferably a substituted or unsubstituted cycloalkenyl group having 3 to 30 carbon atoms, i.e. a monovalent group obtained by removing one hydrogen atom from a cycloalkene having 3 to 30 carbon atoms, e.g. a 2-cyclopenten-1-yl group or a 2-cyclohexen-1-yl group), and a bicycloalkenyl group (which represents a substituted or unsubstituted bicycloalkenyl group, preferably a substituted or unsubstituted bicycloalkenyl group having 5 to 30 carbon atoms, i.e. a monovalent group obtained by removing one hydrogen atom from a bicycloalkene having one double bond, e.g. a bicyclo[2,2,1]hept-2-en-1-yl group or a bicyclo[2,2,2]oct-2-en-4-yl group)]; an alkynyl group (preferably a substituted or unsubstituted alkynyl group having 2 to 30 carbon atoms, e.g. an ethynyl group, a propargyl group, or a trimethylsilylethynyl group);
an aryl group (preferably a substituted or unsubstituted aryl group having 6 to 30 carbon atoms, e.g. a phenyl group, a p-tolyl group, a naphthyl group, an m-chlorophenyl group, or an o-hexadecanoylaminophenyl group); a heterocyclic group (preferably a monovalent group obtained by removing one hydrogen atom from a substituted or unsubstituted 5- or 6-membered aromatic or nonaromatic heterocyclic compound; more preferably a 5- or 6-membered aromatic heterocyclic group having 3 to 30 carbon atoms, e.g. a 2-furyl group, a 2-thienyl group, a 2-pyrimidinyl group, a 2-benzothiazolyl group); a cyano group; a hydroxy group; a nitro group; a carboxyl group; an alkoxy group (preferably a

substituted or unsubstituted alkoxy group having 1 to 30 carbon atoms, e.g. a methoxy group, an ethoxy group, an isopropoxy group, a t-butoxy group, an n-octyloxy group, or a 2-methoxyethoxy group); an aryloxy group (preferably a substituted or unsubstituted aryloxy group having 6 to 30 carbon atoms, e.g. a phenoxy group, a 2-methylphenoxy group, a 4-t-butylphenoxy group, a 3-nitrophenoxy group, or a 2-tetradecanoylaminophenoxy group); a silyloxy group (preferably a silyloxy group having 3 to 20 carbon atoms, e.g. a trimethylsilyloxy group or a t-butyldimethylsilyloxy group); a heterocyclic oxy group (preferably a substituted or unsubstituted heterocyclic oxy group having 2 to 30 carbon atoms, e.g. a 1-phenyltetrazol-5-oxy group or a 2-tetrahydropyranyloxy group);

an acyloxy group (preferably a formyloxy group, a substituted or unsubstituted alkylcarbonyloxy group having 2 to 30 carbon atoms, or a substituted or unsubstituted arylcarbonyloxy group having 6 to 30 carbon atoms, e.g. a formyloxy group, an acetyloxy group, a pivaloyloxy group, a stearoyloxy group, a benzoyloxy group, or a p-methoxyphenylcarbonyloxy group); a carbamoyloxy group (preferably a substituted or unsubstituted carbamoyloxy group having 1 to 30 carbon atoms, e.g. an N,N-dimethylcarbamoyloxy group, an N,N-diethylcarbamoyloxy group, a morpholinocarbonyloxy group, an N,N-di-n-octylaminocarbonyloxy group, or an N-n-octylcarbamoyloxy group); an alkoxycarbonyloxy group (preferably a substituted or unsubstituted alkoxycarbonyloxy group having 2 to 30 carbon atoms, e.g. a methoxycarbonyloxy group, an ethoxycarbonyloxy group, a t-butoxycarbonyloxy group, or an n-octylcarbonyloxy group); an aryloxycarbonyloxy group (preferably a substituted or unsubstituted aryloxycarbonyloxy group having 7 to 30 carbon atoms, e.g. a phenoxycarbonyloxy group, a p-methoxyphenoxycarbonyloxy group, or a p-n-hexadecyloxyphenoxycarbonyloxy group); an amino group (preferably an amino group, a substituted or unsubstituted alkylamino group having 1 to 30 carbon atoms, or a substituted or unsubstituted anilino group having 6 to 30 carbon atoms, e.g. an amino group, a methylamino group, a dimethylamino group, an anilino group, an N-methyl-anilino group, or a diphenylamino group);

an acylamino group (preferably a formylamino group, a substituted or unsubstituted alkylcarbonylamino group having 1 to 30 carbon atoms, or a substituted or unsubstituted arylcarbonylamino group having 6 to 30 carbon atoms, e.g. a formylamino group, an acetylamino group, a pivaloylamino group, a lauroylamino group, a benzoylamino group, or a 3,4,5-tri-n-octyloxyphenylcarbonylamino group); an aminocarbonylamino group (preferably a substituted or unsubstituted aminocarbonylamino group having 1 to 30 carbon atoms, e.g. a carbamoylamino group, an N,N-dimethylaminocarbonylamino group, an N,N-diethylaminocarbonylamino group, or a morpholinocarbonylamino group); an alkoxycarbonylamino group (preferably a substituted or unsubstituted alkoxycarbonylamino group having 2 to 30 carbon atoms, e.g. a methoxycarbonylamino group, an ethoxycarbonylamino group, a t-butoxycarbonylamino group, an n-octadecyloxycarbonylamino group, or an N-methyl-methoxycarbonylamino group); an aryloxycarbonylamino group (preferably a substituted or unsubstituted aryloxycarbonylamino group having 7 to 30 carbon atoms, e.g. a phenoxycarbonylamino group, a p-chlorophenoxycarbonylamino group, or an m-n-octyloxyphenoxycarbonylamino group);

a sulfamoylamino group (preferably a substituted or unsubstituted sulfamoylamino group having 0 to 30 carbon atoms, e.g. a sulfamoylamino group, an N,N-dimethylaminosulfonylamino group, or an N-n-octylaminosulfonylamino group); an alkyl- or aryl-sulfonylamino group (preferably a substituted or unsubstituted alkylsulfonylamino group having 1 to 30 carbon atoms, or a substituted or unsubstituted arylsulfonylamino group having 6 to 30 carbon atoms, e.g. a methylsulfonylamino group, a butylsulfonylamino group, a phenylsulfonylamino group, a 2,3,5-trichlorophenylsulfonylamino group, or a p-methylphenylsulfonylamino group); a mercapto group; an alkylthio group (preferably a substituted or unsubstituted alkylthio group having 1 to 30 carbon atoms, e.g. a methylthio group, an ethylthio group, or an n-hexadecylthio group); an arylthio group (preferably a substituted or unsubstituted arylthio group having 6 to 30 carbon atoms, e.g. a phenylthio group, a p-chlorophenylthio group, or an m-methoxyphenylthio group); a heterocyclic thio group (preferably a substituted or unsubstituted heterocyclic thio group having 2 to 30 carbon atoms, e.g. a 2-benzothiazolylthio group or a 1-phenyltetrazol-5-ylthio group); a sulfamoyl group (preferably a substituted or unsubstituted sulfamoyl group having 0 to 30 carbon atoms, e.g. an N-ethylsulfamoyl group, an N-(3-dodecyloxypropyl)sulfamoyl group, an N,N-dimethylsulfamoyl group, an N-acetylsulfamoyl group, an N-benzoylsulfamoly group, or an N-(N'-phenylcarbamoyl) sulfamoyl group);

a sulfo group; an alkyl- or aryl-sulfinyl group (preferably a substituted or unsubstituted alkylsulfinyl group having 1 to 30 carbon atoms, or a substituted or unsubstituted arylsulfinyl group having 6 to 30 carbon atoms, e.g. a methylsulfinyl group, an ethylsulfinyl group, a phenylsulfinyl group, or a p-methylphenylsulfinyl group); an alkyl- or aryl-sulfonyl group (preferably a substituted or unsubstituted alkylsulfonyl group having 1 to 30 carbon atoms, or a substituted or unsubstituted arylsulfonyl group having 6 to 30 carbon atoms, e.g. a methylsulfonyl group, an ethylsulfonyl group, a phenylsulfonyl group, or a p-methylphenylsulfonyl group); an acyl group (preferably a formyl group, a substituted or unsubstituted alkylcarbonyl group having 2 to 30 carbon atoms, a substituted or unsubstituted arylcarbonyl group having 7 to 30 carbon atoms, or a substituted or unsubstituted heterocyclic carbonyl group having 4 to 30 carbon atoms and being bonded to said carbonyl group through a carbon atom, e.g. an acetyl group, a pivaloyl group, a 2-chloroacetyl group, a stearoyl group, a benzoyl group, a p-n-octyloxyphenylcarbonyl group, a 2-pyridylcarbonyl group, or a 2-furylcarbonyl group); an aryloxycarbonyl group (preferably a substituted or unsubstituted aryloxycarbonyl group having 7 to 30 carbon atoms, e.g. a phenoxycarbonyl group, an o-chlorophenoxycarbonyl group, an m-nitrophenoxycarbonyl group, or a p-t-butylphenoxycarbonyl group); an alkoxycarbonyl group (preferably a substituted or unsubstituted alkoxycarbonyl group having

2 to 30 carbon atoms, e.g. a methoxycarbonyl group, an ethoxycarbonyl group, a t-butoxycarbonyl group, or an n-octadecyloxycarbonyl group); a carbamoyl group (preferably a substituted or unsubstituted carbamoyl group having 1 to 30 carbon atoms, e.g. a carbamoyl group, an N-methylcarbamoyl group, an N,N-dimethylcarbamoyl group, an N,N-di-n-octylcarbamoyl group, or an N-(methylsulfonyl)carbamoyl group);

an aryl- or heterocyclic-azo group (preferably a substituted or unsubstituted aryl azo group having 6 to 30 carbon atoms, or a substituted or unsubstituted heterocyclic azo group having 3 to 30 carbon atoms, e.g. a phenylazo group, a p-chlorophenylazo group, or a 5-ethylthio-1,3,4-thiadiazol-2-ylazo group); an imido group (preferably an N-succinimido group or an N-phthalimido group); a phosphino group (preferably a substituted or unsubstituted phosphino group having 2 to 30 carbon atoms, e.g. a dimethylphosphino group, a diphenylphosphino group, or a methylphenoxyphosphino group); a phosphinyl group (preferably a substituted or unsubstituted phosphinyl group having 2 to 30 carbon atoms, e.g. a phosphinyl group, a dioctyloxyphosphinyl group, or a diethoxyphosphinyl group); a phosphinyloxy group (preferably a substituted or unsubstituted phosphinyloxy group having 2 to 30 carbon atoms, e.g. a diphenoxyphosphinyloxy group or a dioctyloxyphosphinyloxy group); a phosphinylamino group (preferably a substituted or unsubstituted phosphinylamino group having 2 to 30 carbon atoms, e.g. a dimethoxyphosphinylamino group or a dimethylaminophosphinylamino group); and a silyl group (preferably a substituted or unsubstituted silyl group having 3 to 30 carbon atoms, e.g. a trimethylsilyl group, a t-butyldimethylsilyl group, or a phenyldimethylsilyl group).

Among the above functional groups, those having a hydrogen atom may further be substituted with any of the above groups at the position from which the hydrogen atom is removed. Examples of such a functional group include an alkylcarbonylaminosulfonyl group, an arylcarbonylaminosulfonyl group, an alkylsulfonylaminocarbonyl group, and an arylsulfonylaminocarbonyl group. Specific examples of these groups include a methylsulfonylaminocarbonyl, a p-methylphenylsulfonylaminocarbonyl, an acetylaminosulfonyl, and a benzoylaminosulfonyl group.

Examples of a substituent of the aryl portion of the substituted aralkyl group are similar to the examples of a substituent of the substituted aryl groups mentioned later.

In this specification, the aromatic groups refer to aryl groups and substituted aryl groups. To the aromatic groups, an aliphatic ring, another aromatic ring, or a heterocycle may be condensed. The aromatic group preferably has 6 to 40 carbon atoms, more preferably 6 to 30 carbon atoms, and even more preferably 6 to 20 carbon atoms. Among the above, phenyl or naphthyl is preferable as an aryl group, and phenyl is particularly preferable.

The aryl portion of the substituted aryl group is similar to the above-mentioned aryl groups. Examples of a substituent of the substituted aryl groups are similar to the above-mentioned examples of the substituent of the alkyl portions of a substituted alkyl group, a substituted alkenyl group, a substituted alkynyl group, and a substituted aralkyl group.

In this specification, the heterocyclic groups preferably contain a 5-membered or 6-membered, saturated or unsaturated heterocycle. To the heterocycle, an aliphatic ring, an aromatic ring, or another heterocycle may be condensed. Examples of a heteroatom of the heterocycle include B, N, O, S, Se, and Te. As the heteroatom, N, O, and S are preferable. It is preferable that a carbon atom of the heterocycle has a free valence (monovalent) (the heterocyclic group is preferably to be bonded at a carbon atom thereof). The heterocyclic group preferably has 1 to 40 carbon atoms, more preferably 1 to 30 carbon atoms, and even more preferably 1 to 20 carbon atoms. Examples of the saturated heterocycle include a pyrrolidine ring, a morpholine ring, a 2-bora-1,3-dioxolane ring, and 1,3-thiazolidine ring. Examples of the unsaturated heterocycles include an imidazole ring, a thiazole ring, a benzothiazole ring, a benzoxazole ring, a benzotriazole ring, a benzoselenazole ring, a pyridine ring, a pyrimidine ring, and a quinoline ring. The heterocyclic groups may have a substituent. Examples of the substituent are similar to the previously-mentioned examples of the substituent of the alkyl portions of the substituted alkyl group, the substituted alkenyl group, the substituted alkynyl group, and the substituted aralkyl group.

A solution for confirming the spectral absorption maximum wavelength is obtained by dissolving the ultra absorbent compositions (A) and (B) in an organic or inorganic solvent or water, either singly or as a mixture.

Examples of the organic solvent include amide solvents (e.g., N,N-dimethylformamide, NN-dimethylacetamide, and 1-methyl-2-pyrrolidone), sulfone solvents (e.g., sulfolane), sulfoxide solvents (e.g., dimethyl sulfoxide), ureido solvents (e.g., tetramethylurea), ether solvents (e.g., dioxane, tetrahydrofuran, and cyclopentyl methyl ether), ketone solvents (e.g., acetone and cyclohexanone), hydrocarbon solvents (e.g., toluene, xylene, and n-decane), halogen solvents (e.g., tetrachloroethane, chlorobenzene, and chloronaphthalene), alcohol solvents (e.g., methanol, ethanol, isopropyl alcohol, ethylene glycol, cyclohexanol, and phenol), pyridine solvents (e.g., pyridine, γ-picoline, and 2,6-lutidine), ester solvents (e.g., ethyl acetate and butyl acetate), carboxylic acid solvents (e.g., acetic acid and propionic acid), nitrile solvents (e.g., acetonitrile), sulfonic acid solvents (e.g., methanesulfonic acid), and amine solvents (e.g., triethylamine and tributylamine).

As the inorganic solvent, sulfuric acid and phosphoric acid can be used.

From the viewpoint of solubility of ultra absorbent compositions (A) and (B), amide solvents, sulfone solvents, sulfoxide solvents, ureido solvents, ether solvents, ketone solvents, halogen solvents, hydrocarbon solvents, alcohol solvents, ester solvents, or nitrile solvents are preferable.

The concentrations of the ultra absorbent compositions (A) and (B) for measurement are not particularly limited insofar as the maximum wavelength of spectral absorption can be confirmed, and are preferably in a range of from $1 \times 10^{-7}$ mol/L to $1 \times 10^{13}$ mol/L.
The measurement temperatures are not particularly limited, and are preferably from 0°C to 80°C.
There is no particular limitation on a spectral absorption measurement apparatus, and a common spectral absorption measurement apparatus (e.g., U-4100 spectrophotometer, trade name, manufactured by Hitachi High-Technologies Corp.) can be used.
The ultraviolet absorbent composition of the present invention is **characterized in that** the composition includes at least one ultraviolet absorbent (A) and at least one ultraviolet absorbent (B) having a specific absorption-spectral shape. The ultraviolet absorbent (A) is preferably composed of two kinds or less. It is especially preferable that the ultraviolet absorbent (A) is only one kind. The ultraviolet absorbent (B) is preferably three kinds or less, and more preferably two kinds. It is especially preferable that the ultraviolet absorbent (B) is only one kind.
A mixing ratio of the ultraviolet absorbent (A) and the ultraviolet absorbent (B) may be arbitrary, except that the ratios of both 1:0 and 0:10 are excluded. The ratio is preferably in the range of from 10:1 to 1:10, more preferably from 5:1 to 1:5, and most preferably from 4:1 to 1:4. The mixing ratio in this case is represented by molar ratio.
Detailed explanation of the ultraviolet absorbent (A) is followed. The ultraviolet absorbent (A) is a compound represented by the above-described Formula (1).
In Formula (1) above, $Het^1$ represents a bivalent five- or six-membered aromatic heterocyclic residue having at least one hetero atom. $Het^1$ may be a fused ring.
Examples of the hetero atoms include boron, nitrogen, oxygen, silicon, phosphorus, sulfur, selenium, tellurium, and the like, preferably, nitrogen, oxygen and sulfur atoms, more preferably nitrogen and sulfur atoms, and particularly preferably a sulfur atom. If the ring has two or more hetero atoms, the hetero atoms may be the same as or different from each other.
Examples of the aromatic heterocycles prepared by adding two hydrogen atoms to a bivalent aromatic heterocyclic residue include pyrrole, pyrazole, imidazole, 1,2,3-triazole, 1,2,4-triazole, pyridine, pyridazine, pyrimidine, pyrazine, 1,3,5-triazine, furan, thiophene, oxazole, isoxazole, thiazole, isothiazole, 1,2,3-oxadiazole, 1,3,4-thiadiazole, and the like. The aromatic heterocycle is preferably pyrrole, pyridine, furan, or thiophene, more preferably pyridine or thiophene, and particularly preferably thiophene. The site of the aromatic heterocycle where the hydrogen atom is abstracted is arbitrary. For example, in the case of a five-membered heterocyclic compound pyrrole, the sites are, for example, 2- and 3-sites, 2- and 4-sites, 2- and 5-sites, 3- and 4-sites, and 3- and 5-sites; and in the case of a six-membered heterocyclic compound pyridine, the sites are, for example, 2- and 3-sites, 2- and 4-sites, 2- and 5-sites, 2- and 6-sites, 3- and 4-sites, 3- and 5-sites and 3- and 6-sites.
The aromatic heterocyclic residue may have a substituent group(s). The substituent group is, for example, a monovalent substituent group. Examples of the monovalent substituent groups (hereinafter, referred to as R) include halogen atoms (e.g., fluorine atom, chlorine atom, bromine atom, and iodine atom), alkyl groups having 1 to 20 carbon atoms (e.g., methyl and ethyl), aryl groups having 6 to 20 carbon atoms (e.g., phenyl and naphthyl), a cyano group, a carboxyl group, alkoxycarbonyl groups (e.g., methoxycarbonyl), aryloxycarbonyl groups (e.g., phenoxycarbonyl), substituted or unsubstituted carbamoyl groups (e.g., carbamoyl, N-pheylcarbamoyl and N,N-dimethylcarbamoyl), alkylcarbonyl groups (e.g., acetyl), arylcarbonyl groups (e.g., benzoyl), a nitro group, substituted or unsubstituted amino groups (e.g., amino, dimethylamino and anilino), acylamino groups (e.g., acetamido and ethoxycarbonylamino), sulfonamido groups (e.g., methane sulfonamide), imido groups (e.g., succinimido and phthalimido), imino groups (e.g., benzylideneamino), a hydroxy group, alkoxy groups having 1 to 20 carbon atoms (e.g., methoxy), aryloxy groups (e.g., phenoxy), acyloxy groups (e.g., acetoxy), alkylsulfonyloxy groups (e.g., methanesulfonyloxy), arylsulfonyloxy groups (e.g., benzenesulfonyloxy), a sulfo group, substituted or unsubstituted sulfamoyl groups (e.g., sulfamoyl and N-phenylsulfamoyl), alkylthio groups (e.g., methylthio), arylthio groups (e.g., phenylthio), alkylsulfonyl groups (e.g., methanesulfonyl), arylsulfonyl groups (e.g., benzenesulfonyl), heterocyclic groups having 6 to 20 carbon atoms (e.g., pyridyl, morpholino), and the like. The substituent group may be further substituted, and the multiple substituent groups, if present, may be the same as or different from each other. The substituent groups then are, for example, the monovalent substituents R described above. The substituent groups may bind to each other to form a ring.
The substituent group is preferably an alkyl group, an alkoxy group, or an aryl group, more preferably an alkyl or aryl group, and particularly preferably an alkyl group.
$X^a$, $X^b$, $X^c$ and $X^d$ each independently represent a heteroatom. Examples of the hetero atoms include boron, nitrogen, oxygen, silicon, phosphorus, sulfur, selenium, tellurium, and the like, preferably, nitrogen, oxygen and sulfur atoms, more preferably nitrogen and oxygen atoms. $X^a$ to $X^d$ may have a substituent group(s). The substituent groups then are, for example, the monovalent substituents R described above.
$Y^a$, $Y^b$, $Y^c$, $Y^d$, $Y^e$ and $Y^f$ each independently represent a heteroatom or a carbon atom. The atoms constituting $Y^a$ to $Y^f$ include, for example, carbon atom, nitrogen atom, oxygen atom, sulfur atom and the like. The atoms constituting $Y^a$ to $Y^f$ are preferably carbon atom, nitrogen atom, and oxygen atom, more preferably carbon atom and nitrogen atom, still more preferably carbon atom, and particularly preferably all carbon atoms. The atom may further be substituted,

and the substituent groups may bind to each other to form a ring, which may additionally be fused with another ring. The substituent groups then are, for example, the monovalent substituents R described above.

The ring formed from $X^a$, $X^b$, $Y^a$ to $Y^c$ and carbon atom and the ring formed from $X^c$, $X^d$, $Y^d$ to $Y^f$ and carbon atom (two rings bound to the aromatic heterocyclic residue represented by $Het^1$) each may have a double bond at any position. At least one of the two rings preferably has a fused ring. In addition, at least one of the two rings is preferably not a perimidine ring.

Specific examples of the compounds are shown in the following Tables 1 to 6, as the ring formed from $X^a$, $X^b$, $Y^a$ to $Y^c$ and carbon atom is designated as A, the aromatic heterocyclic residue represented by $Het^1$ as Het, and the ring formed from $X^c$, $X^d$, $Y^d$ to $Y^f$ and carbon atom as C.

[Table 1]

| A | Het | B |
|---|---|---|
| 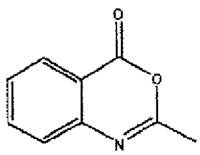 | 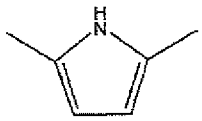 | 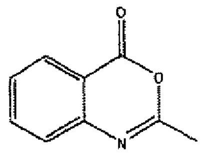 |
| 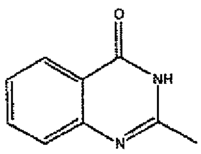 | 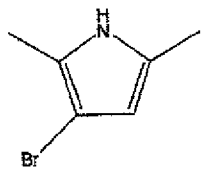 | 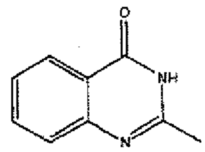 |
| 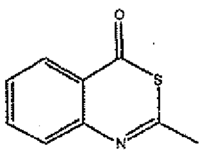 | 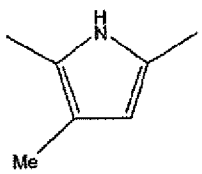 | 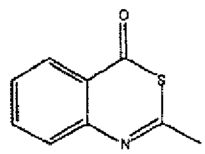 |
| 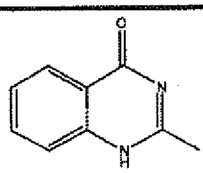 | 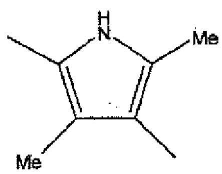 | 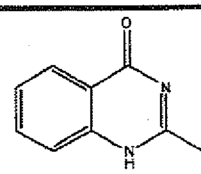 |
| 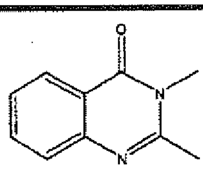 | 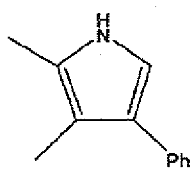 | 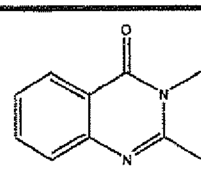 |
| 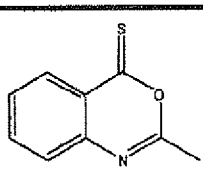 | 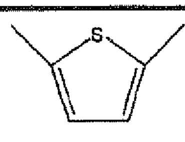 | 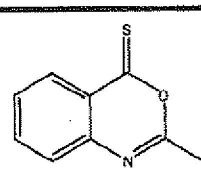 |
| 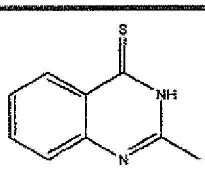 | 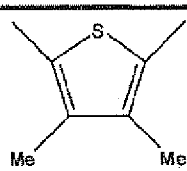 | 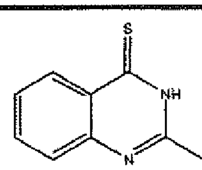 |

[Table 2]

| A | Het | B |
|---|---|---|
| 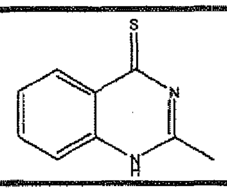 | 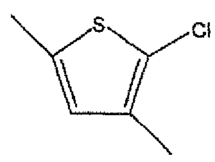 | 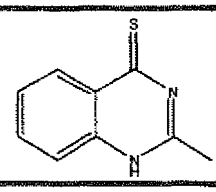 |
| 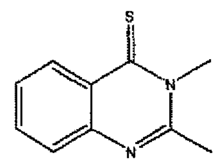 | 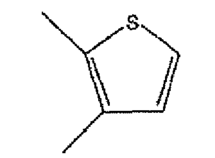 | 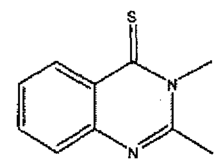 |
| 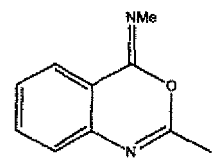 | 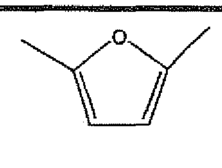 | 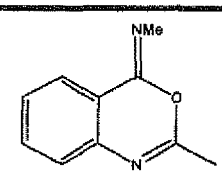 |
| 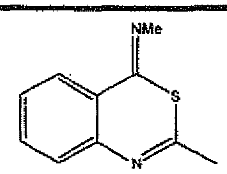 | 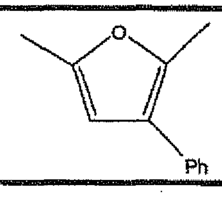 | 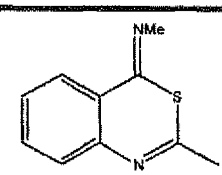 |
| 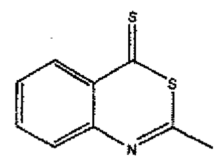 | 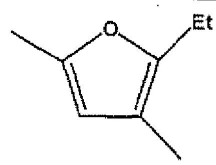 | 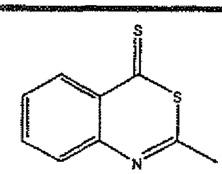 |
| 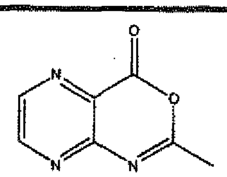 | 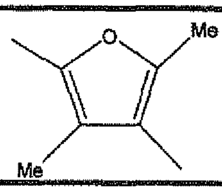 | 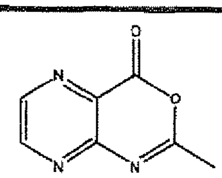 |
| 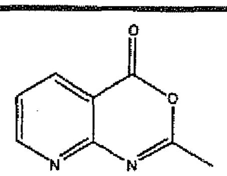 | 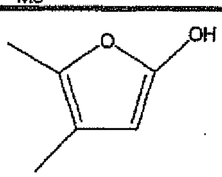 | 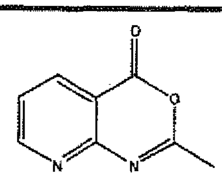 |
| 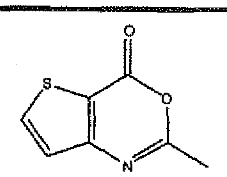 | 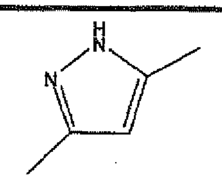 | 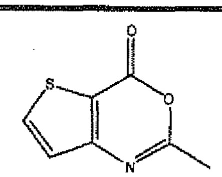 |
| 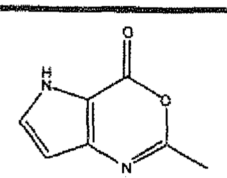 | 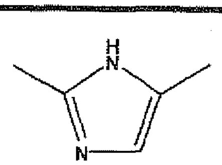 | 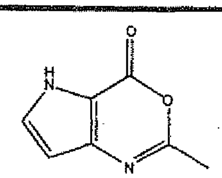 |

[Table 3]

| A | Het | B |
|---|---|---|
| 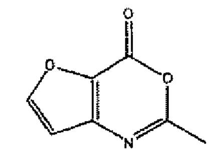 | 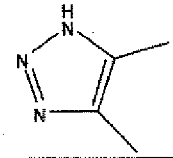 | 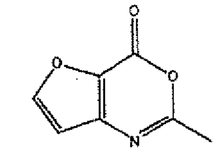 |
| 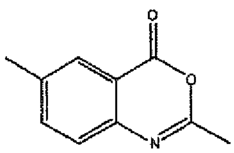 | 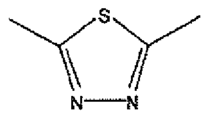 | 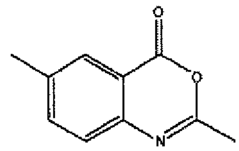 |
| 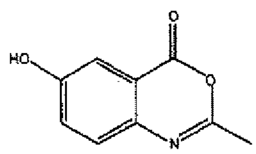 | 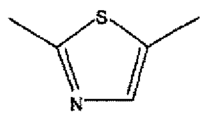 | 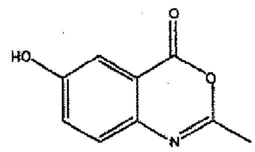 |
| 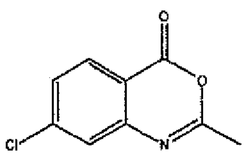 | 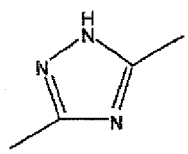 | 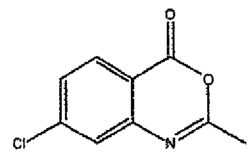 |
| 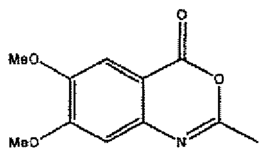 | 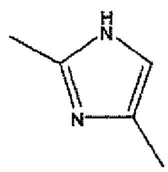 | 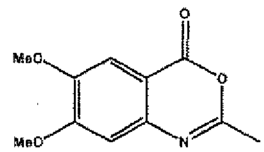 |
| 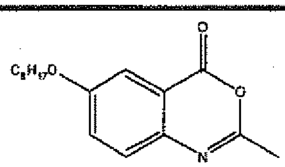 | 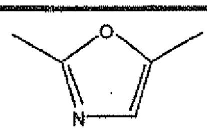 | 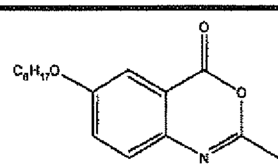 |
| 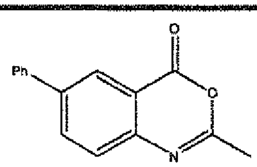 | 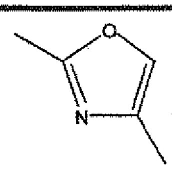 | 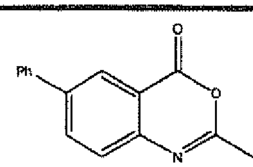 |
| 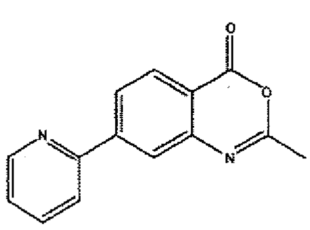 | 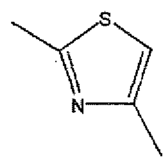 | 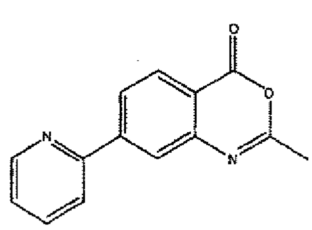 |

[Table 4]

| A | Het | B |
|---|---|---|
| 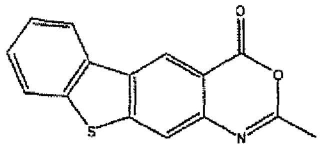 | 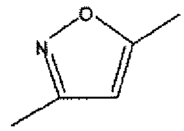 | 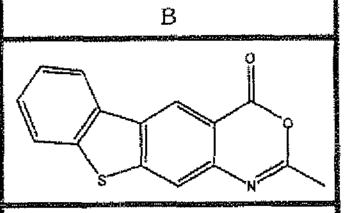 |
| 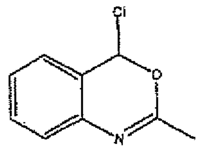 | 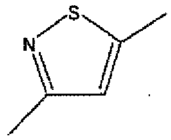 | 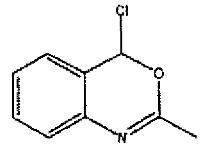 |
| 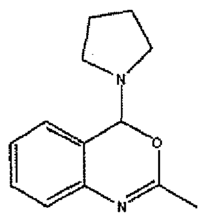 | 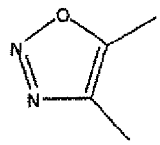 | 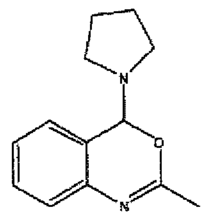 |
| 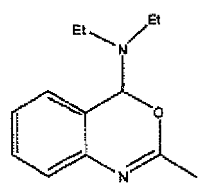 | 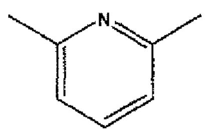 | 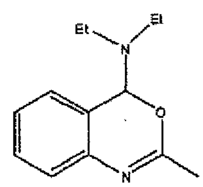 |
| 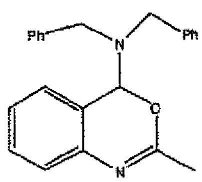 | 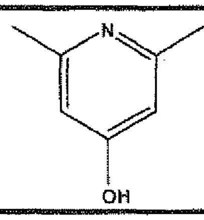 | 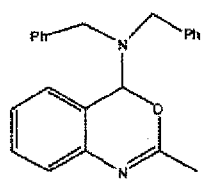 |
| 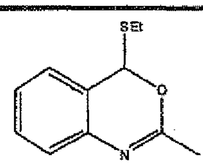 | 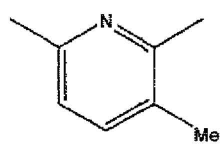 | 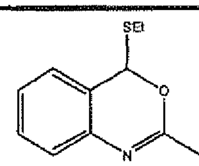 |
| 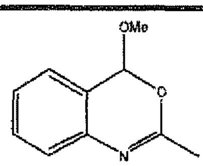 | 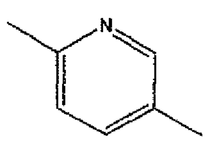 | 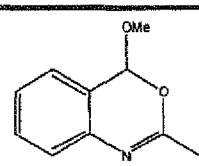 |
| 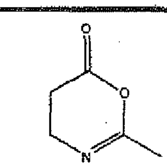 | 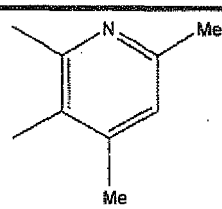 | 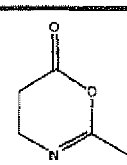 |

[Table 5]

| A | Het | B |
|---|---|---|
| 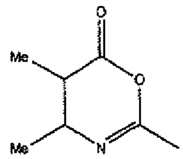 | 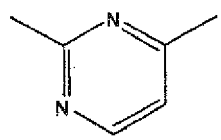 | 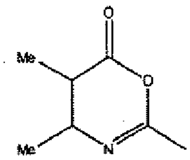 |
| 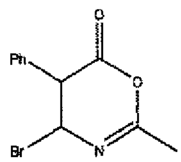 | 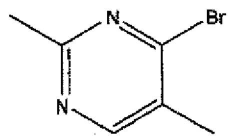 | 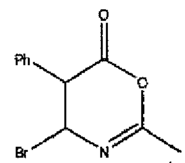 |
| 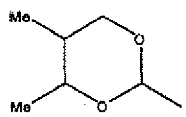 | 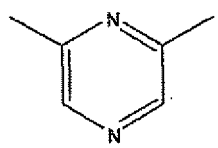 | 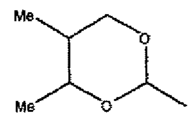 |
| 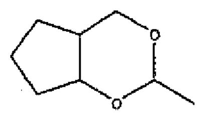 | 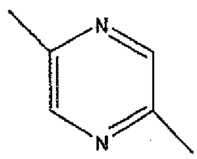 | 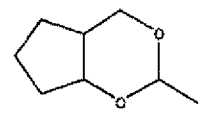 |
| 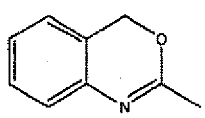 | 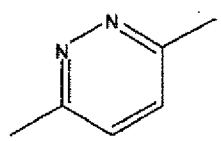 | 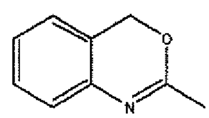 |
| 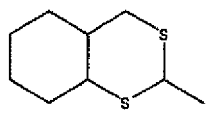 | 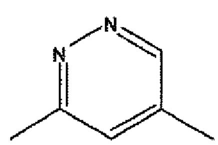 | 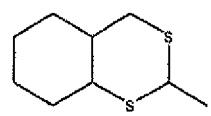 |
| 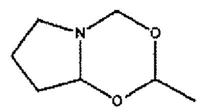 | 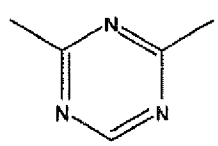 | 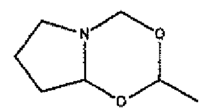 |
| 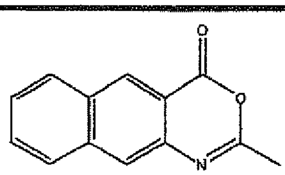 | 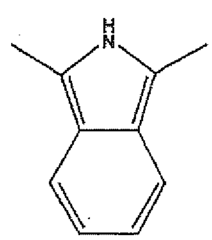 | 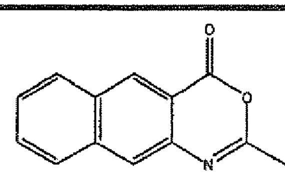 |

[Table 6]

| A | Het | B |
|---|---|---|
| 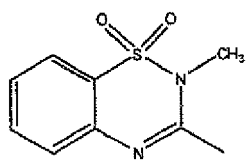 | 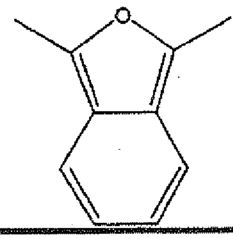 | 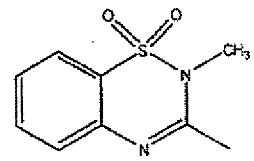 |
| 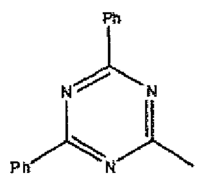 | 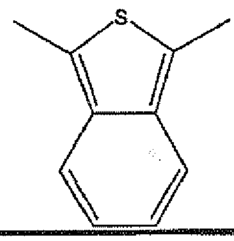 | 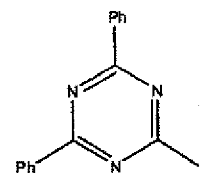 |
| 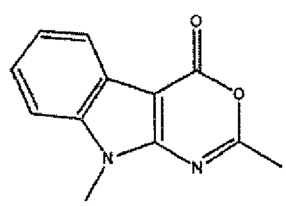 | 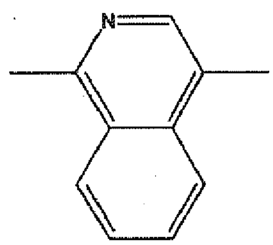 | 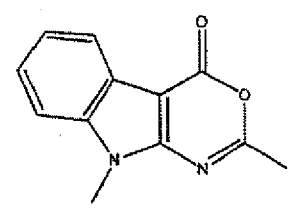 |
| 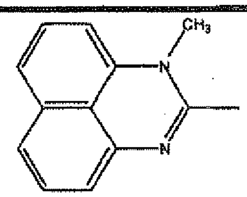 | 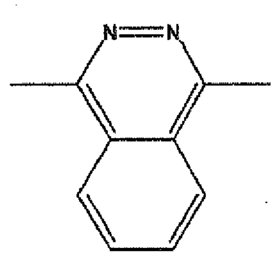 | 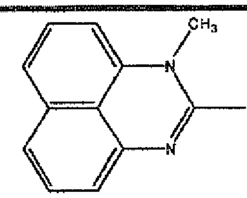 |

The compound represented by the above Formula (1) is preferably a compound represented by the above Formula (2). Hereinafter, the compound represented by the above Formula (2) is described in detail.

$Het^2$ is the same as $Het^1$ in the above Formula (1) and the favorable examples thereof are also the same.

$X^{2a}$, $X^{2b}$, $X^{2c}$ and $X^{2d}$ is the same as $X^a$, $X^b$, $X^c$ and $X^d$ in the above Formula (1) and the favorable examples thereof are also the same. $X^{2a}$, $X^{2b}$, $X^{2c}$ and $X^{2d}$ may be different from each other. It is more preferable that the combinations of $X^{2a}$ and $X^{2b}$, and $X^{2c}$ and $X^{2d}$ are the same as each other, and particularly preferable that $X^{2a}$ and $X^{2c}$ are oxygen atoms and $X^{2b}$ and $X^{2d}$ are nitrogen atoms.

$Y^{2b}$, $Y^{2c}$, $Y^{2e}$ and $Y^{2f}$ are the same as $Y^b$, $Y^c$, $Y^e$ and $Y^f$ in the above Formula (1), respectively, and the favorable examples thereof are also the same.

$L^1$ and $L^2$ each independently represent an oxygen atom or sulfur atom or $=NR^a$ ($R^a$ represents a hydrogen atom or a monovalent substituent group. The substituent group is, for example, the monovalent substituent R described above), preferably an oxygen atom or $=NR^a$, and more preferably an oxygen atom. $L^1$ and $L^2$ may be different from each other, but preferably the same. In particular, $L^1$ and $L^2$ are particularly favorably both oxygen atoms.

$Z^1$ and $Z^2$ each independently represent an atom group needed for forming a four- to eight-membered ring together with $Y^{2b}$ and $Y^{2c}$ or $Y^{2e}$ and $Y^{2f}$. These rings may have a substituent group(s), which may further have a fused ring. Examples of the rings formed include aliphatic hydrocarbon rings such as cyclohexane and cyclopentane; aromatic hydrocarbon rings such as benzene and naphthalene; and heterocycles such as pyridine, pyrrole, pyridazine, thiophene,

imidazole, furan, pyrazole, oxazole, triazole, thiazole, or the benzo-fused rings thereof, and the like. Preferable examples are aromatic hydrocarbon rings and heterocycles. Aromatic hydrocarbon rings are more preferable, and a benzene ring is particularly preferable..

Further, the compound represented by Formula (2) is preferably a compound represented by the above Formula (3). Hereinafter, the compound represented by the above Formula (3) is described in detail.

$Het^3$ is the same as $Het^2$ in the above Formula (2) and the favorable examples thereof are also the same.

$X^{3a}$, $X^{3b}$, $X^{3c}$ and $X^{3d}$ are the same as $X^{2a}$, $X^{2b}$, $X^{2c}$ and $X^{2d}$ in the above Formula (2), respectively, and the favorable examples thereof are also the same. $X^{3a}$, $X^{3b}$, $X^{3c}$ and $X^{3d}$ may be different from each other. It is preferble that the combinations of $X^{3a}$ and $X^{3b}$, and $X^{3c}$ and $X^{3d}$ are the same as each other, and particularly preferable that $X^{3a}$ and $X^{3c}$ are oxygen atoms and $X^{3b}$ and $X^{3d}$ are nitrogen atoms.

$R^{3a}$, $R^{3b}$, $R^{3c}$, $R^{3d}$, $R^{3e}$, $R^{3f}$, $R^{3g}$ and $R^{3h}$ each independently represent a hydrogen atom or a monovalent substituent group. The substituent groups then are, for example, the monovalent substituents R described above. Any two substituent groups among $R^{3a}$ to $R^{3d}$ and $R^{3e}$ to $R^{3h}$ may bind to each other to form a ring, which may be a fused ring. $R^{3a}$ to $R^{3h}$ each preferably represent a hydrogen atom, an alkyl group having 10 or less carbon atoms, an alkoxy group having 10 or less carbon atoms, or a hydroxy group, more preferably a hydrogen atom or an alkoxy group having 10 or less carbon atoms, still more preferably a hydrogen atom, and particularly preferably, $R^{3a}$ to $R^{3h}$ are all hydrogen atoms.

Further, the compound represented by the above Formula (3) is preferably a compound represented by the above Formula (4). Hereinafter, the compound represented by the above Formula (4) is described in detail.

$Het^4$ is the same as $Het^3$ in the above Formula (3) and the favorable examples thereof are also the same.

$R^{4a}$, $R^{4b}$, $R^{4c}$, $R^{4d}$, $R^{4e}$, $R^{4f}$, $R^{4g}$ and $R^{4h}$ are the same as $R^{3a}$, $R^{3b}$, $R^{3c}$, $R^{3d}$, $R^{3e}$, $R^{3f}$, $R^{3g}$ and $R^{3h}$ in the above Formula (3), respectively, and the favorable examples thereof are also the same.

Further, the compound represented by the above Formula (4) is preferably a compound represented by the above Formula (5). Hereinafter, the compound represented by the above Formula (5) is described in detail.

$Het^5$ is the same as $Het^4$ in the above Formula (4) and the favorable examples thereof are also the same.

$R^{5a}$, $R^{5b}$, $R^{5c}$, $R^{5d}$, $R^{5e}$, $R^{5f}$, $R^{5g}$ and $R^{5h}$ are the same as $R^{4a}$, $R^{4b}$,$R^{4c}$, $R^{4d}$, $R^{4e}$, $R^{4f}$, $R^{4g}$ and $R^{4h}$ in the above Formula (4), respectively, and the favorable examples thereof are also the same.

$R^{5i}$ and $R^{5j}$ each independently represent a hydrogen atom or a monovalent substituent group. The monovalent substituent groups are, for example, the monovalent substituents R described above. $R^{5i}$ and $R^{5j}$ may bind to each other to form a ring, which may be a fused ring. $R^{5i}$ and $R^{5j}$ each preferably represent a hydrogen atom, an alkyl group having 10 or less carbon atoms, an alkoxy group having 10 or less carbon atoms, or a hydroxy group, more preferably a hydrogen atom or an alkoxy group having 10 or less carbon atoms, still more preferably a hydrogen atom, and particularly preferably, $R^{5i}$ and $R^{5j}$ are both hydrogen atoms.

The compound represented by any one of the above Formulae (1) to (5) may be prepared by any method. Examples of the methods include those disclosed in known patent documents and non-patent documents, for example, Examples of JP-A-2000-264879, p.4. left line 43 to right line 8; in the Examples of JP-A-2003-155375, p.4, right column lines 5 to 30; "Bioorganic & Medicinal Chemistry", 2000, vol. 8, p.2095-2103, "Bioorganic & Medicinal Chemistry Letters", 2003, vol. 13, p.4077-4080, and others. For example, exemplary compound (15) can be prepared in reaction of 3,5-pyrazole dicarbonyl dichloride with anthranilic acid. Alternatively, exemplary compound (32) can be prepared in reaction of 2,5-thiophenedicarbonyl dichloride with 4,5-dimethoxyanthranilic acid.

Hereinafter, specific examples of the compounds (exemplary compounds) represented by any one of the above Formulae (1) to (5) is described below, but the present invention is not limited thereby.

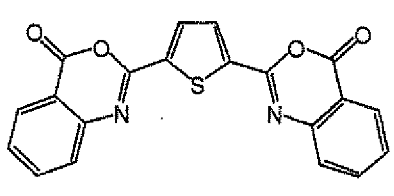

(1)

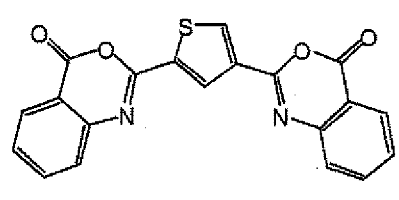

(2)

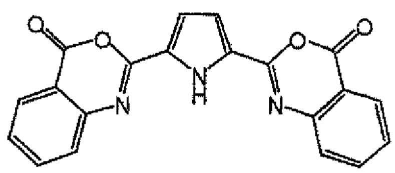
(3)

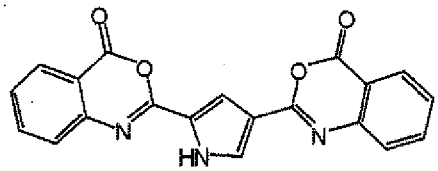
(4)

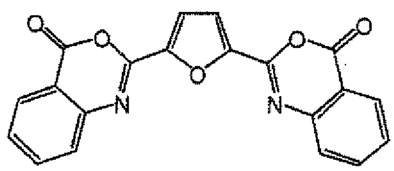
(5)

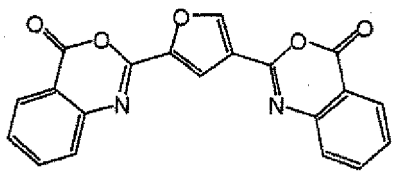
(6)

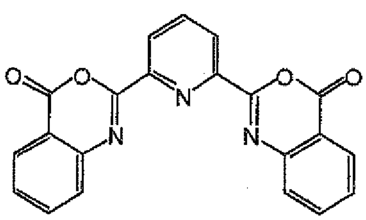
(7)

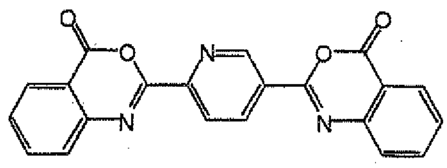
(8)

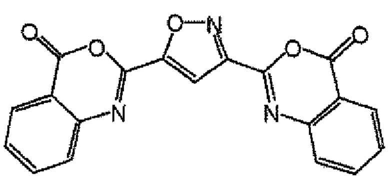
(9)

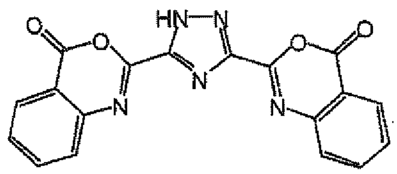
(10)

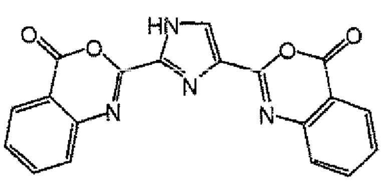
(11)

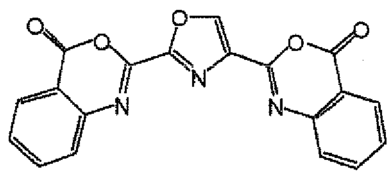
(12)

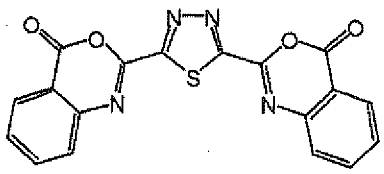

(13)

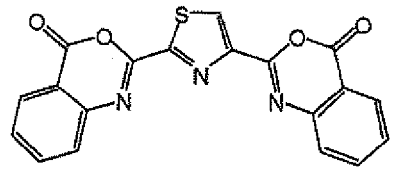

(14)

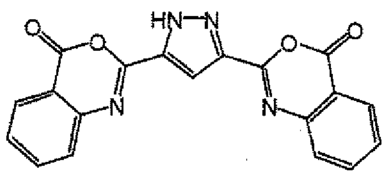

(15)

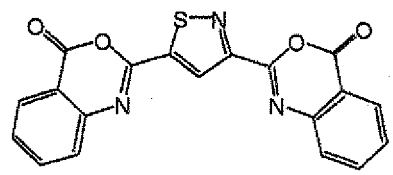

(16)

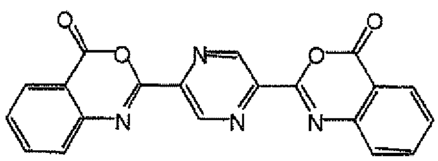

(17)

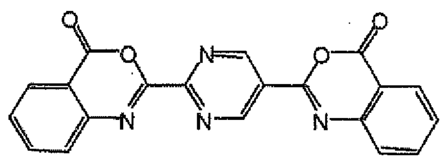

(18)

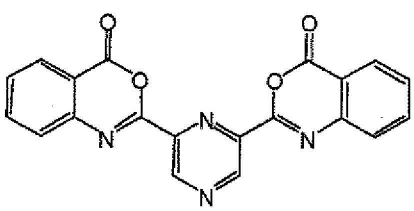

(19)

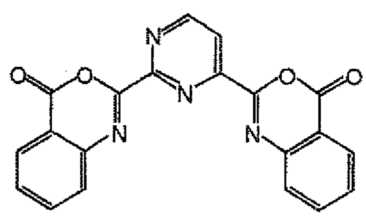

(20)

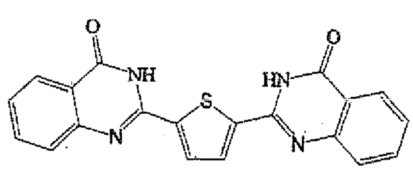

(21)

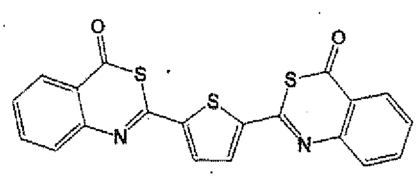

(22)

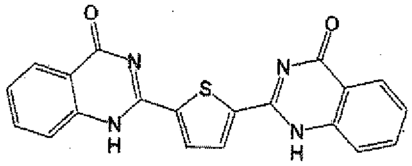 (23) 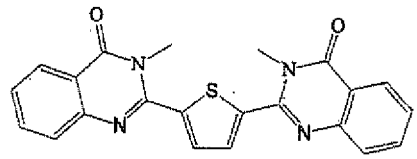 (24)

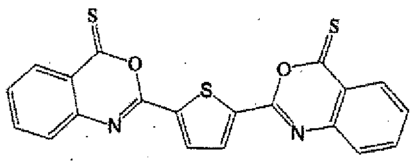 (25) 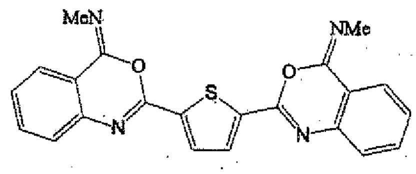 (26)

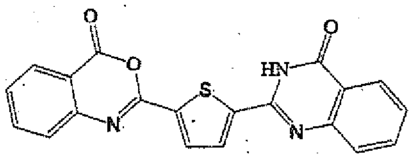 (27) 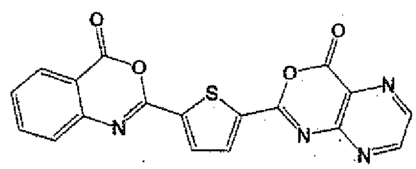 (28)

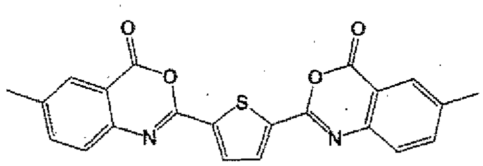 (29) 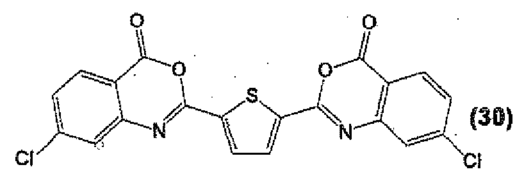 (30)

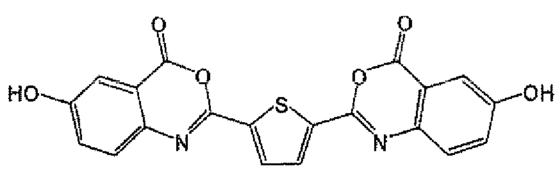 (31)

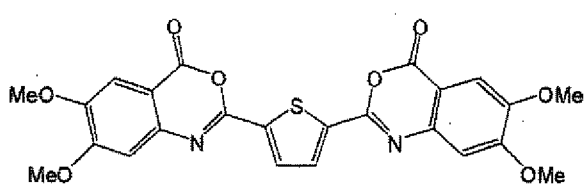 (32)

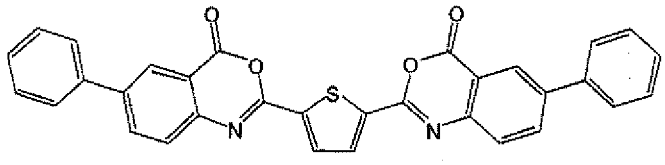

(33)

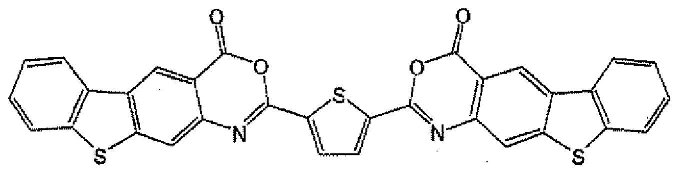

(34)

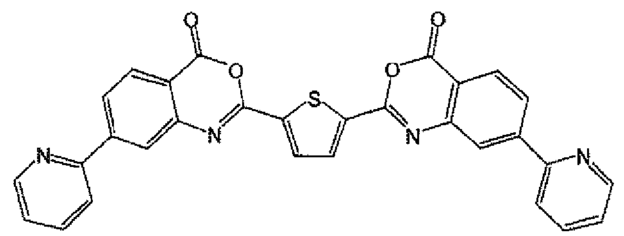

(35)

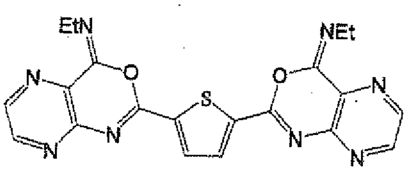

(36)

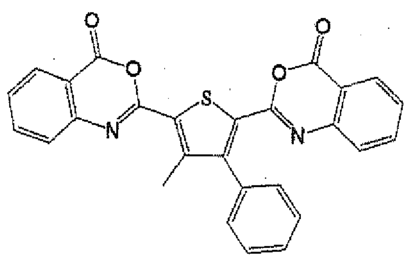

(37)

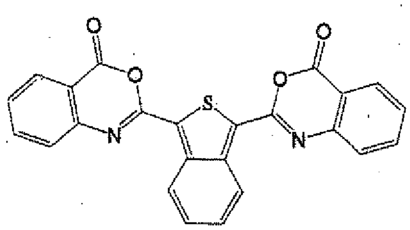

(38)

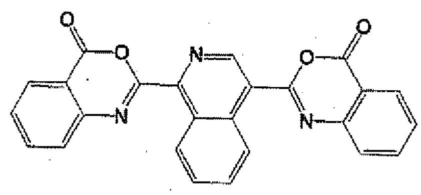

(39)

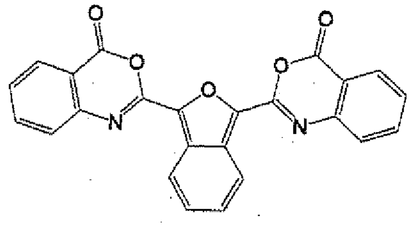 (40)

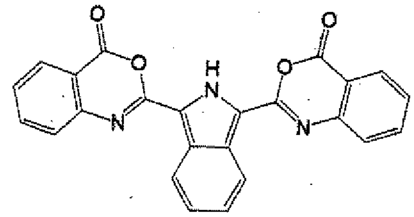 (41)

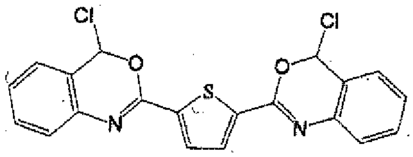 (42)

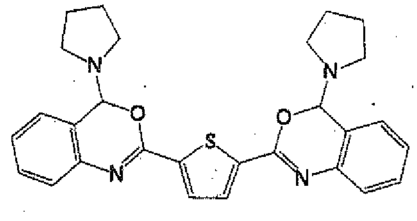 (43)

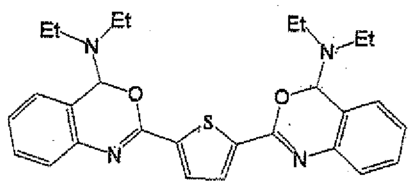 (44)

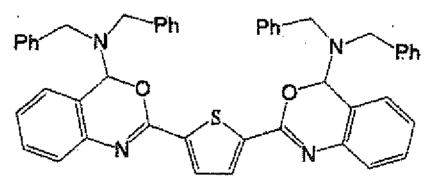 (45)

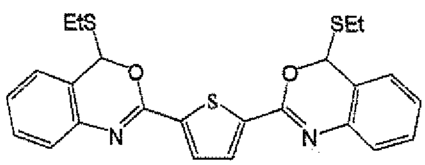 (46)

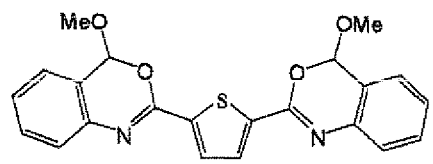 (47)

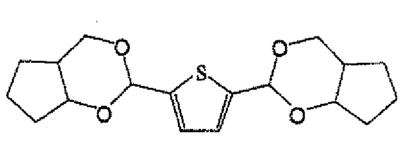 (48)

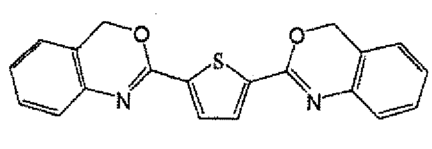 (49)

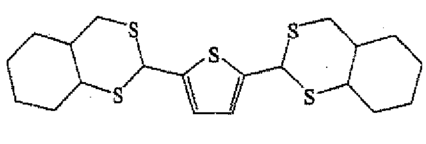 (50)

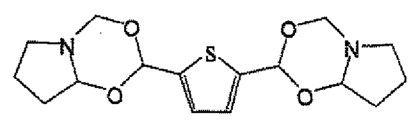 (51)

The compound represented by any one of the above Formulae (1) to (5) may have tautomers depending on the structure and the environment where the compound is located. A typical form thereof is described here in the present specification, but the tautomers different from that described in the present specification are also included in the compound of the present invention.

The compound represented by any one of the above Formulae (1) to (5) may have an isotopic element (such as $^{2}H$, $^{3}H$, $^{13}C$, $^{15}N$, $^{17}O$, or $^{18}O$).

A polymer having the structure of the compound represented by any one of the above Formulae (1) to (5) in its recurring unit can also be used favorably in the present invention. The polymer may be a homopolymer or a copolymer having two or more kinds of recurring units. It may be a copolymer having another recurring unit additionally. Examples of the polymers having an ultraviolet absorbent structure in the recurring unit are described in each bulletin of JP-B-1-53455 ("JP-B" means examined Japanese patent publication) and JP-A-61-189530, and the specification of EP Patent No. 27242. The polymer can be prepared with reference to the methods described in these patent documents.

Next, the ultraviolet absorbent (B) having a specific absorption-spectral shape is explained in detail. The ultraviolet absorbent (B) is **characterized in that** absorbance at 320 nm is 20% or more of the absorbance at the wavelength of maximum absorption in the range of from 270 nm to 400 nm, and the wavelength of maximum absorption is 380 nm or less. If the absorbance at 320 nm is less than 20% of the absorbance at the wavelength of maximum absorption, a wavelength range which can not be covered by both the ultraviolet absorbent (A) and the ultraviolet absorbent (B) occurs. Especially, absorbance of the ultraviolet absorbent (B) at 320 nm is preferably 30% or more of the absorbance at the wavelength of maximum absorption, more preferably 40% or more, and most preferably 50% or more. Further, absorbance of the ultraviolet absorbent (B) at 320 nm is suitably less than 100%, preferably 99% or less, more preferably 95% or less, and most preferably 90% or less, of the absorbance at the wavelength of maximum absorption in the range of from 270 nm to 400 nm. Further, the wavelength of maximum absorption is preferably 380 nm or less, more preferably 370 nm or less, further more preferably 365 nm or less, and most preferably 350 nm or less.

The ultraviolet absorbent (B) represents a material in which absorbance at 320 nm is 20% or more of the absorbance at the wavelength of maximum absorption and the wavelength of maximum absorption is 380 nm or less. As is shown in Fig. 1, the ultraviolet absorbent (B) is classified into ultraviolet absorbent B-(1) in which the wavelength of absorption maximum is less than 320 nm and ultraviolet absorbent B-(2) in which the wavelength of maximum absorption is in the range of from 320 nm to 380 nm, which may be suitably selected in accordance with their intended use.

For example, the ultraviolet absorbent B-(1) is especially preferably used when other element capable of absorbing a short-wave ultraviolet is not present as in the case of kneading an ultraviolet absorbent into a plastic molding or a polymer. In the case of kneading an ultraviolet absorbent into a plastic molding or a polymer, since other element capable of absorbing a short-wave ultraviolet of 300 nm or less is not present, the use of the ultraviolet absorbent B-(1) capable of effectively absorbing light in a short-wave ultraviolet range makes it possible to prevent the plastic molding itself and its content from ultraviolet light without using another short-wave ultraviolet range-absorbing filter. Further, such an unexpected effect that compatibility with a polymer and light fastness can be improved by using the ultraviolet absorbent B-(1) in combination with the ultraviolet absorbent (A) used in the present invention.

For example, the ultraviolet absorbent B-(2) is especially preferably used when other element capable of absorbing a short-wave ultraviolet is present as in the case of coating an ultraviolet absorbent dissolved in a film or polymer on a glass substrate, In the case of using the ultraviolet absorbent B-(2), blocking capability of ultraviolet near 320 nm is excellent. Although a short-wave ultraviolet range of 300 nm or less can be absorbed efficiently by the ultraviolet absorbent B-(2), sometimes difficulty occurs. As a result, it is preferred that the ultraviolet absorbent B-(2) is used by coating it on a polymer or glass substrate that functions as a filter that blocks a short-wave side of ultraviolet. Further, use of the ultraviolet absorbent B-(2) in combination with the ultraviolet absorbent (A) used in the present invention achieves such unexpected effects that solubility with respect to solvents (ethyl acetate, methyl ethyl ketone, toluene, and the like) that are used when a coating film is used in a solvent coating system, and light fastness are improved.

The ultraviolet absorbent (B) may have any suitable structure, as long as the structure satisfies the conditions that absorbance at 320 nm is 20% or more of the absorbance at the wavelength of maximum absorption and the wavelength of maximum absorption is 380 nm or less. Examples of the ultraviolet absorbent (B) include benzotriazole-series, triazine-series, benzophenone-series, merocyanine-series, cyaine-series, dibenzoylmethane-series, cinnamic acid-series, acrylate-series, benzoic acid ester-series, oxalic acid diamide-series, formamidine-series, and benzoxadinone-series compounds, all of which are known as a structure of the ultraviolet absorbent. Of these, benzotriazole-series, triazine-series, benzophenone-series, dibenzoylmethane-seies, formamidine-series, and benzoxadinone-series compounds are preferred. More preferable compounds are benzotriazole-series, triazine-series, benzophenone-series, formamidine-series, and benzoxadinone-series compounds. Most preferable compounds are benzotriazole-series, triazine-series, and benzoxadinone-series compounds. These ultraviolet absorbents are described, for example, in Fine Chemical (in English), May in 2004, pp. 28-38, Kobunshi-yo Kinoseitenkazai no Shin Tenkai (New Developments of Functional Additives for Polymers), published by Toray Research Center, Division of Investigation Research (Toray Research Center, 1999), pp. 96-140, Kobunshi Tenkazai no Kaihatsu to kankyo Taisaku (Developments and Environmental Measures for Polymer Additives), supervised by Seiichi Okatsu (published by C M C Shuppan, 2003), pp. 54-64, and Kobunshi no Rekka · Henshoku mekanizumu to sono Anteika Gijutsu-Nohausyu- (Mechanism of Deterioration Discoloration of Polymers and Their Stabilization Technique-Collection of Know-how), published by Kabushiki kaisha Gijutsu Jyoho Kyokai (Gijutsu Jyoho Kyokai, 2006).

The benzotriazole-series compounds have an effective absorption wavelength of approximately 270 to 380 nm, and is preferably represented by the following Formulae (IIa) and (IIb). Hereinafter, (IIa) and (IIb) are described deteil.

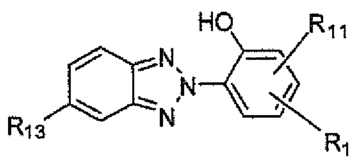
(IIa)

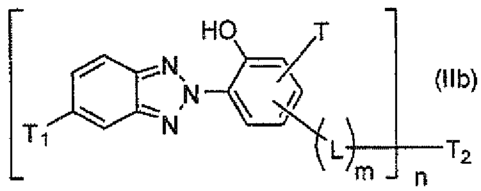
(IIb)

[In Formula (IIa),

$R_{11}$ represents a hydrogen atom, a substituted or unsubstituted alkyl group, a substituted or unsubstituted cycloalkyl group, or a substituted or unsubstituted aryl group;
$R_{12}$ represents a hydrogen atom, a halogen atom, a substituted or unsubstituted alkyl group, or a substituted or unsubstituted aryl group; and
$R_{13}$ represents a hydrogen atom, a halogen atom, a substituted or unsubstituted alkyl group, a substituted or unsubstituted alkoxy group, or -$COOR_{14}$ group (herein, $R_{14}$ represents a hydrogen atom, a substituted or unsubstituted alkyl group or a substituted or unsubstituted aryl group.)]

[In Formula (IIb),

T represents a hydrogen atom or a substituted or unsubstituted alkyl group;
$T_1$ represents a hydrogen atom, a halogen atom, a substituted or unsubstituted alkyl group, a substituted or unsubstituted aryl group, or a substituted or unsubstituted alkoxy group;
L represents a divalent linking group or a single bond;
m represents 0 or 1;
n represents an integer of 1 to 4; and
when n is 1, $T_2$ represents a halogen atom, a substituted or unsubstituted alkyl group or a substituted or unsubstituted aryl group; when n is 2, $T_2$ represents a divalent substituent; when n is 3, $T_2$ represents a trivalent substituent; and when n is 4, $T_2$ represents a tetravalent substituent.]

(Formula (IIa))

$R_{11}$ represents a hydrogen atom, a substituted or unsubstituted alkyl group, a substituted or unsubstituted cycloalkyl group or a substituted or unsubstituted aryl group.
$R_{11}$ is preferably a substituted or unsubstituted alkyl group having 1 to 18 carbon atoms, a substituted or unsubstituted cycloalkyl group having 5 to 18 carbon atoms, or a substituted or unsubstituted aryl group having 6 to 24 carbon atoms; and particularly preferably a substituted or unsubstituted alkyl group having 1 to 18 carbon atoms, or a substituted or unsubstituted alkyl group having 1 to 18 carbon atoms.

The substituted alkyl group, the substituted cycloalkyl group and the substituted aryl group each are referred to as an alkyl group, a cycloalkyl group and an aryl group, each of which has a monovalent substituent at an arbitrary position thereof, respectively. Examples of the monovalent substituent include a halogen atom (e.g., a fluorine atom, a chlorine atom, a bromine atom and an iodine atom), a straight-chain or branched alkyl group having 1 to 20 carbon atoms (preferably 1 to 10 carbon atoms) (e.g., methyl, ethyl), an aryl group having 6 to 20 carbon atoms (preferably 6 to 10 carbon atoms) (e.g., phenyl, naphthyl), a cyano group, a carboxyl group, an alkoxycarbonyl group having 1 to 20 carbon atoms (preferably 1 to 10 carbon atoms) (e.g., methoxycarbonyl), an aryloxycarbonyl group having 6 to 20 carbon atoms (preferably 6 to 10 carbon atoms) (e.g., phenoxycarbonyl), a substituted or unsubstituted carbamoyl group having 0 to 20 carbon atoms (preferably 0 to 10 carbon atoms) (e.g., carbamoyl, N-phenylcarbamoyl, N,N-dimethylcarbamoyl), an alkylcarbonyl group having 1 to 20 carbon atoms (preferably 1 to 10 carbon atoms) (e.g., acetyl), an arylcarbonyl group having 6 to 20 carbon atoms (preferably 6 to 10 carbon atoms) (e.g., benzoyl), a nitro group, a substituted or

unsubstituted amino group having 0 to 20 carbon atoms (preferably 0 to 10 carbon atoms) (e.g., amino, dimethylamino, anilino), an acylamino group having 1 to 20 carbon atoms (preferably 1 to 10 carbon atoms) (e.g., acetamido, ethoxycarbonylamino),

a sulfonamido group having 0 to 20 carbon atoms (preferably 0 to 10 carbon atoms) (e.g., methanesulfonamido), an imido group having 2 to 20 carbon atoms (preferably 2 to 10 carbon atoms) (e.g., succinimido, phthalimido), an imino group having 1 to 20 carbon atoms (preferably 1 to 10 carbon atoms) (e.g., benzylideneamino), a hydroxy group, an alkoxy group having 1 to 20 carbon atoms (preferably 1 to 10 carbon atoms) (e.g., methoxy), an aryloxy group having 6 to 20 carbon atoms (preferably 6 to 10 carbon atoms) (e.g., phenoxy), an acyloxy group having 1 to 20 carbon atoms (preferably 1 to 10 carbon atoms) (e.g., acetoxy), an alkylsulfonyloxy group having 1 to 20 carbon atoms (preferably 1 to 10 carbon atoms) (e.g., methanesulfonyloxy), an arylsulfonyloxy group having 6 to 20 carbon atoms (preferably 6 to 10 carbon atoms) (e.g., benzenesulfonyloxy), a sulfo group, a substituted or unsubstituted sulfamoyl group having 0 to 20 carbon atoms (preferably 0 to 10 carbon atoms) (e.g., sulfamoyl, N-phenylsulfamoyl), an alkylthio group having 1 to 20 carbon atoms (preferably 1 to 10 carbon atoms) (e.g., methylthio), an arylthio group having 6 to 20 carbon atoms (preferably 6 to 10 carbon atoms) (e.g., phenylthio), an alkylsulfonyl group having 1 to 20 carbon atoms (preferably 1 to 10 carbon atoms) (e.g., methansulfonyl), an arylsulfonyl group having 6 to 20 carbon atoms (preferably 6 to 10 carbon atoms) (e.g., benzenesulfonyl) and a four- to seven-membered (preferably five- to six-membered) heterocyclic group (e.g., pyridyl, morpholino).

$R_{12}$ represents a hydrogen atom, a halogen atom, a substituted or unsubstituted alkyl group or a substituted or unsubstituted aryl group. $R_{12}$ is preferably a hydrogen atom, a chlorine atom, a substituted or unsubstituted alkyl group having 1 to 18 carbon atoms, a substituted or unsubstituted cycloalkyl group having 5 to 18 carbon atoms, or a substituted or unsubstituted aryl group having 6 to 24 carbon atoms; and particularly preferably a hydrogen atom, a chlorine atom, a substituted or unsubstituted alkyl group having 1 to 18 carbon atoms, or a substituted or unsubstituted aryl group having 6 to 24 carbon atoms.

$R_{13}$ represents a hydrogen atom, a halogen atom, a substituted or unsubstituted alkyl group, a substituted or unsubstituted alkoxy group or $-COOR_{14}$ group (herein, $R_{14}$ represents a hydrogen atom, a substituted or unsubstituted alkyl group, or a substituted or unsubstituted aryl group). $R_{13}$ is preferably a hydrogen atom, a chlorine atom, a substituted or unsubstituted alkyl group having 1 to 18 carbon atoms, a substituted or unsubstituted alkoxy group having 1 to 18 carbon atoms, or $-COOR_{14}$ group (herein, $R_{14}$ represents a hydrogen atom, a substituted or unsubstituted alkyl group having 1 to 18 carbon atoms or a substituted, or unsubstituted aryl group having 6 to 24 carbon atoms).

$R_{11}$ and $R_{12}$ may be substituted at an arbitrary position of the benzene ring. The substitution at 2- or 4- position to the hydroxyl group is preferable.

(Formula (IIb))

T represents a hydrogen atom or a substituted or unsubstituted alkyl group. T is preferably a hydrogen atom or a substituted or unsubstituted alkyl group having 1 to 18 carbon atoms.

$T_1$ represents a hydrogen atom, a halogen atom, a substituted or unsubstituted alkyl group, a substituted or unsubstituted aryl group or a substituted or unsubstituted alkoxy group. $T_1$ is preferably a hydrogen atom, a chlorine atom, a substituted or unsubstituted alkyl group having 1 to 18 carbon atoms, an aryl group having 6 to 24 carbon atoms or an alkoxy group having 1 to 18 carbon atoms.

-L- represents a divalent linking group or a single bond. m represents 0 or 1.

The case where m is 0 (zero) means that $T_2$ directly bonds with the benzene ring without involving L, that is -L- represents a single bond.

The divalent linking group -L- is explained. -L- is a divalent substituent represented by the following Formula (a).

Formula (a) $-(L_1)_{m1}-(L_2)_{m2}-(L_3)_{m3}-(L_4)_{m4}-(L_5)_{m5}-$

In Formula (a), m1, m2, m3, m4 and m5 each represent an integer of 0 to 2. $L_1$, $L_2$, $L_3$, $L_4$ and $L_5$ each independently represent -CO-, -O-, $-SO_2-$, -SO-, $-NR_L-$, a substituted or unsubstituted divalent alkyl group, a substituted or unsubstituted divalent alkenyl group, or a substituted or unsubstituted divalent aryl group. $R_L$ represents a hydrogen atom, a substituted or unsubstituted alkyl group, or a substituted or unsubstituted aryl group.

Examples of $R_L$ include a hydrogen atom, a methyl group, an ethyl group, a propyl group, a hexyl group, an octyl group, a phenyl group, and a naphthyl group. The group may be substituted with one or more monovalent substituents at any position of the alkyl or aryl group. The monovalent substituent is, for example, the monovalent substituent described above. $R_L$ is preferably a substituted or unsubstituted alkyl group having 3 to 20 carbon atoms or a substituted or unsubstituted aryl group having 6 to 14 carbon atoms; and more preferably a substituted or unsubstituted alkyl group having 6 to 12 carbon atoms or a substituted or unsubstituted aryl group having 6 to 10 carbon atoms.

Preferred examples of the divalent substituent -L- include $-O-CO-C_2H_4-CO-O-$, $-O-CO-C_3H_6-$, -NH-CO-

$C_3H_6$-CO-NH-, -NH-CO-$C_4H_8$-, -$CH_2$-, -$C_2H_4$-, -$C_3H_6$-, -$C_4H_8$-, -$C_5H_{10}$-, -$C_8H_{16}$-, -$C_4H_8$-CO-O-, -$C_6H_4$-$C_6H_4$- and -NH-$SO_2$-$C_3H_6$-.

In Formula (IIb), n represents an integer of 1 to 4.

When n is 1, $T_2$ represents a halogen atom, a substituted or unsubstituted alkyl group or a substituted or unsubstituted aryl group. When n is 1, $T_2$ is preferably a chlorine atom, a substituted or unsubstituted alkyl group having 1 to 18 carbon atoms, or a substituted or unsubstituted aryl group having 6 to 24 carbon atoms.

When n is 2, $T_2$ represents a divalent substituent. When n is 2, examples of $T_2$ include the same examples as the above-described divalent substituent -L-. When n is 2, $T_2$ is preferably -$CH_2$-, -O-CO-$C_2H_4$-CO-O-, or -NH-CO-$C_3H_6$-CO-NH-.

When n is 3, $T_2$ represents a trivalent substituent. The trivalent substituent is explained. The trivalent substituent is a trivalent alkyl group, a trivalent aryl group or a substituent represented by the following formula.

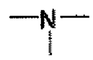

The trivalent substituent is preferably a trivalent alkyl group having 1 to 8 carbon atoms, a trivalent aryl group having 6 to 14 carbon atoms or a substituent represented by the following formula.

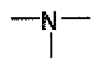

When n is 4, $T_2$ represents a tetravalent substituent. The tetravalent substituent is explained. The tetravalent substituent is a tetravalent alkyl group or a tetravalent aryl group. Among the tetravalent substituents, a tetravalent alkyl group having 1 to 8 carbon atoms and a tetravalent aryl group having 6 to 14 carbon atoms are preferable.

In Formula (IIb), it is especially preferable that n is 1 or 2. Specifically, the components of the Formula (IIb) are preferably combined as follows:

When n is 1, T is a hydrogen atom, or a substituted or unsubstituted alkyl group having 1 to 18 carbon atoms; $T_1$ is a hydrogen atom, a chlorine atom, a substituted or unsubstituted alkyl group having 1 to 18 carbon atoms, a substituted or unsubstituted aryl group having 6 to 24 carbon atoms, or an alkoxy group having 1 to 18 carbon atoms; L is -O-CO-$C_3H_6$-, -$CH_2$-, -$C_3H_6$-, -$C_5H_{10}$-, -$C_8H_{16}$-, -NH-CO-$C_4H_8$- or a single bond; and $T_2$ is a chlorine atom, a substituted or unsubstituted alkyl group having 1 to 18 carbon atoms, or a substituted or unsubstituted aryl group having 6 to 24 carbon atoms.

When n is 2, a preferable combination is that T is a hydrogen atom, or a substituted or unsubstituted alkyl group having 1 to 18 carbon atoms; $T_1$ is a hydrogen atom, a chlorine atom, a substituted or unsubstituted alkyl group having 1 to 18 carbon atoms, an aryl group having 6 to 24 carbon atoms, or an alkoxy group having 1 to 18 carbon atoms; L is -$CH_2$- or a single bond; and $T_2$ is -$CH_2$-, -O-CO-$C_2H_4$-CO-O- or NH-CO-$C_3H_6$-CO-NH-.

Typical examples of the compound represented by Formula (IIa) or (IIb) include 2-(2'-hydroxy-5'-methylphenyl) benzotriazole, 2-(2'-hydroxy-5'-t-butylphenyl)benzotriazole, 2-(2'-hydroxy-3'-t-butyl-5'-methylphenyl)-5-chlorobenzotriazole, 2-(2'-hydroxy-3',5'-di-t-butylphenyl)-5-chlorobenzotriazole, 2-(2'-hydroxy- 3'-dodecyl-5'methylphenyl)-5-chlorobenzotriazole, 2-(2'-hydroxy-3',5'-di-t-amylphenyl)benzotriazole,2-(2'-hydroxy-5'-(1,1,3,3-tetramethylbutyl)phenyl) benzotriazole, 2-(2'-hydroxy-4-octyloxyphenyl)benzotriazole, 2-(2'-hydroxy-3'-(3,4,5,6-tetrahydrophthalimidylmethyl)-5'-methylbenzyl)phenyl)benzotriazole, 2-(3'-sec-butyl-5'-t-butyl-2'-hydroxyphenyl)benzotriazole, 2-(3',5'-bis-($\alpha$,$\alpha$-dimethylbenzyl)-2'-hydroxyphenyl)benzotriazole, 2-(3'-t-butyl-2'-hydroxy-5'-(2-octyloxycarbonylethyl)phenyl)-5-chlorobe-nzotriazole, 2-(3'-t-butyl-5'-[2-(2-ethylhexyloxy)-carbonylethyl]-2'-hydroxyphenyl)-5-chloro-benzotriazole, 2-(3'-t-butyl-2'-hydroxy-5'-(2-methoxycarbonylethyl)phenyl)-5-chloro-benzotriazole, 2-(3'-t-butyl-2'-hydroxy-5'-(2-methoxycarbonylethyl)phenyl)benzotriazole, 2-(3'-t-butyl-2'-hydroxy-5'-(2-octyloxycarbonylethyl)phenyl)benzotriazole, 2-(3'-t-butyl-5'-[2-(2-ethylhexyloxy)carbonylethyl]-2'-hydroxyphenyl)benzotriazole, 2-(3'-dodecyl-2'-hydroxy-5'-methylphenyl) benzotriazole, 2-(3'-t-butyl-2'-hydroxy-5'-(2-isooctyloxycarbonylethyl)phenylbenzotriazole, 2,2'-methylene-bis [4-(1,1,3,3-tetramethylbutyl)-6-benzotriazole-2-ylphenol], ester exchange products of 2-[3'-t-butyl-5'-(2-methoxycarbonylethyl)-2'-hydroxyphenyl]-2H-benzotriazole and polyethylene glycol 300; and the compound represented by the following formula:

$$[R-CH_2CH_2-COO-CH_2CH_2]_2$$

(wherein, R represents 3'-tert-butyl-4'-hydroxy-5'-2H-benzotriazol-2-ylphenyl, 2-[2'-hydroxy-3'-(α,α-dimethylbenzyl)-5'-(1,1,3,3-tetramethylbutyl)-phenyl]benzotriazole; 2-[2'-hydroxy-3'-(1,1,3,3-tetramethylbutyl)-5'-(α,α-dimethylbenzyl)-phenyl]benzotriazole and the like).

The triazine-based compound is preferably a compound having an effective absorption wavelength of approximately 270 to 380 nm that is represented by Formula (III).

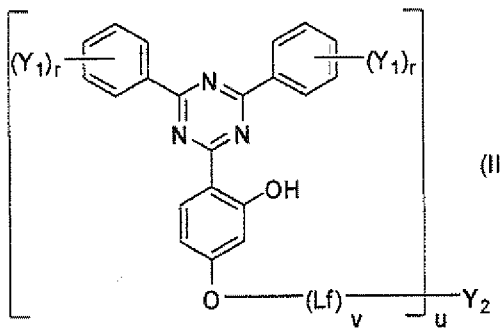

(III)

[In Formula (III),

the substituent $Y_1$'s each independently represent a hydrogen atom, a hydroxyl group, a substituted or unsubstituted alkyl group, a substituted or unsubstituted aryl group, or a substituted or unsubstituted alkoxy group;
Lf represents a divalent linking group or a single bond;
u represents 1 or 2;
v represents 0 or 1;
r represents an integer of 1 to 3; and
when u is 1, $Y_2$ represents a hydrogen atom, a substituted or unsubstituted alkyl group, or a substituted or unsubstituted aryl group; and when u is 2, $Y_2$ represents a divalent substituent.

$Y_1$'s each independently represent a hydrogen atom, a hydroxyl group, a substituted or unsubstituted alkyl group, a substituted or unsubstituted aryl group, or a substituted or unsubstituted alkoxy group. $Y_1$ is preferably a hydrogen atom, a hydroxyl group, a substituted or unsubstituted alkyl group having 1 to 18 carbon atoms, a substituted or unsubstituted aryl group having 6 to 24 carbon atoms, or a substituted or unsubstituted alkoxy group having 1 to 18 carbon atoms.

Lf represents a divalent linking group or a single bond. u represents 1 or 2. r represents an integer of 1 to 3. v represents 0 or 1. When v is 0, Lf represents a single bond.
The divalent linking group -Lf- is explained. The divalent linking group -Lf-is a divalent substituent represented by the following Formula (b).

Formula (b) $-(Lf_1)_{mf1}-(Lf_2)_{mf2}-(Lf_3)_{mf3}-(Lf_4)_{mf4}-(Lf_5)_{mf5}-$

In Formula (b), mf1 to mf5 each represents an integer of 0 to 2.

$Lf_1$, $Lf_2$, $Lf_3$, $Lf_4$ and $Lf_5$ each independently represent -CO-, -O-, $-SO_2-$, -SO-,
$-NRf_L-$, a substituted or unsubstituted divalent alkyl group, a substituted or unsubstituted divalent alkenyl group, or a substituted or unsubstituted divalent aryl group. $Rf_L$ represents a hydrogen atom, a substituted or unsubstituted alkyl group, or a substituted or unsubstituted aryl group.

Examples of $Rf_L$ include a hydrogen atom, a methyl group, an ethyl group, a propyl group, a hexyl group, an octyl group, a phenyl group, and a naphthyl group. The group may be substituted with one or more monovalent substituents at any position of the alkyl or aryl groups. The monovalent substituent is, for example, the monovalent substituent described above. $Rf_L$ is preferably a substituted or unsubstituted alkyl group having 3 to 20 carbon atoms or a substituted or unsubstituted aryl group having 6 to 14 carbon atoms; and more preferably a substituted or unsubstituted alkyl group having 6 to 12 carbon atoms or a substituted or unsubstituted aryl group having 6 to 10 carbon atoms.

Preferred examples of the divalent substituent -Lf- include $-O-CO-C_2H_4-CO-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$ and $-NH-SO_2-C_3H_6-$.

When u is 1, $Y_2$ represents a hydrogen atom, a substituted or unsubstituted alkyl group, or a substituted or

unsubstituted aryl group. When u is 1, $Y_2$ is preferably a hydrogen atom, a substituted or unsubstituted alkyl group having 1 to 18 carbon atoms, or a substituted or unsubstituted aryl group having 6 to 24 carbon atoms.

When u is 2, $Y^2$ represents a divalent substituent. Examples of the divalent substituent include the same examples as the aforementioned divalent substituent -L-. $Y_2$ is preferably a substituted or unsubstituted divalent alkyl group, a substituted or unsubstituted divalent alkenyl group, a substituted or unsubstituted divalent aryl group, $-CH_2CH(OH)CH_2-O-Y_{11}-OCH_2CH(OH)CH_2$, $-CO-Y_{12}-CO-$, $-CO-NH-Y_{13}-NH-CO-$, or $-(CH_2)_t-CO_2-Y_{14}-OCO-(CH_2)_t$.

Herein, t is 1, 2 or 3;

$Y_{11}$ represents a substituted or unsubstituted alkylene group, phenylene group, or -phenylene-M-phenylene- (wherein, M represents -O-, -S-, $-SO_2-$, $-CH_2-$ or $-C(CH_3)_2-$);

$Y_{12}$ represents a substituted or unsubstituted divalent alkyl group, a substituted or unsubstituted divalent alkenyl group, or a substituted or unsubstituted divalent aryl group;

$Y_{13}$ represents a substituted or unsubstituted divalent alkyl group, or a substituted or unsubstituted divalent aryl group; and $Y_{14}$ represents a substituted or unsubstituted divalent alkyl group, or a substituted or unsubstituted divalent aryl group.

Namely, when u is 2, $Y_2$ is preferably a substituted or unsubstituted divalent alkyl group having 1 to 18 carbon atoms, a substituted or unsubstituted divalent aryl group having 6 to 24 carbon atoms, $-CH_2CH(OH)CH_2-O-CH_2-OCH_2CH(OH)CH_2-$, $-CH_2CH(OH)CH_2-O-C(CH_3)_2-OC_8H_{16}-$, or $-(CH_2)_2-CO_2-C_2H_4-OCO-(CH_2)_2-$.

Typical examples of the compound represented by Formula (III) include 2-(4-butoxy-2-hydroxyphenyl)-4,6-di(4-butoxyphenyl)-1,3,5-triazine, 2-(4-butoxy-2-hydroxyphenyl)-4,6-di(2,4-dibutoxyphenyl)-1,3,5-triazine, 2,4-di(4-butoxy-2-hydroxyphenyl)-6-(4-butoxyphenyl)-1,3,5-triazine, 2,4-di(4-butoxy-2-hydroxyphenyl)-6-(2,4-dibutoxyphenyl)-1,3,5-triazine, 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxyl-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethyl)-1,3,5-triazine, 2-[2-hydroxy-4-(2-hydroxy-3-octyloxypropyloxy)phenyl]-4,6-bis(2,4-dimethyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3-dodecyloxypropoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris(2-hydroxy-4-(3-butoxy-2-hydroxypropoxy)phenyl)-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2-hydroxy-propyloxy]phenyl}-4,6-bis(2,4-dimethylphenyl)-1,3,5-triazine and 2-(2-hydroxy-4-(2-ethylhexyl)oxy)phenyl-4,6-di(4-phenyl)phenyl-1,3,5-triazine.

The benzophenone-based compound is preferably a compound having an effective absorption wavelength of approximately 270 to 380 nm that is represented by the following Formula (IVa) or (IVb).

[化 17]

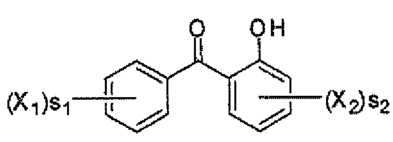 (IVa) 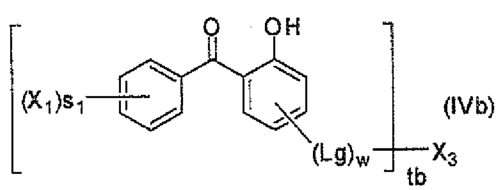 (IVb)

[In Formula (IV a), $X_1$ and $X_2$ each independently represent a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group or a substituted or unsubstituted amino group; and s1 and s2 each independently represent an integer of 1 to 3.]

[In Formula (IVb), $X_1$ represents a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group; s1 represents an integer of 1 to 3;

Lg represents a divalent substituent or a single bond; w represents 0 or 1;

tb represents 1 or 2; and when tb is 1, $X_3$ represents a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group; and when tb is 2, $X_3$ represents a divalent substituent.]

(Formula (IVa))

$X_1$ and $X_2$ each independently represent a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group.

$X_1$ and $X_2$ each are preferably a hydrogen atom, a chlorine atom, a hydroxyl group, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon atoms, an alkyloxycarbonyl group having 2 to 18 carbon atoms, an aryloxycarbonyl group having 7 to 24 carbon atoms, a sulfonic acid group or a substituted or unsubstituted amino group having 1 to 16 carbon atoms; and particularly preferably a hydrogen atom, a hydroxyl group, a substituted or unsubstituted alkoxy group having 1 to 18 carbon atoms, a sulfonic acid group or a substituted or unsubstituted amino group having 1 to 16 carbon atoms.

(Formula (IVb))

tb is 1 or 2, w is 0 or 1, and s1 is an integer of 1 to 3.

The substituent $X_1$ represents a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group.

$X_1$ is preferably a hydrogen atom, a chlorine atom, a hydroxyl group, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon atoms, an alkyloxycarbonyl group having 2 to 18 carbon atoms, an aryloxycarbonyl group having 7 to 24 carbon atoms, a sulfonic acid group or a substituted or unsubstituted amino group having 1 to 16 carbon atoms; and particularly preferably a hydrogen atom, a hydroxyl group, a substituted or unsubstituted alkoxy group having 1 to 18 carbon atoms, a sulfonic acid group or a substituted, or unsubstituted amino group having 1 to 16 carbon atoms.

-Lg- represents a divalent linking group or a single bond. w represents an integer of 0 or 1. The case where w is 0 (zero) means that $X_3$ directly bonds with the benzene ring without involving Lg, namely, -Lg- represents a single bond. The divalent linking group -Lg- is explained. The divalent linking group Lg is a divalent substituent represented by the following Formula (c).

Formula (c) $$-(Lg_1)_{mg1}-(Lg_2)_{mg2}-(Lg_3)_{mg3}-(Lg_4)_{mg4}-(Lg_5)_{mg5}-$$

In Formula (c), mg1, mg2, mug3, mg4 and mg5 each represent an integer of 0 to 2.

$Lg_1$, $Lg_2$, $Lg_3$, $Lg_4$ and $Lg_5$ each independently represent -CO-, -O-, $-SO_2-$, -SO-, $-NRg_L-$, a substituted or unsubstituted divalent alkyl group, a substituted or unsubstituted divalent alkenyl group, or a substituted or unsubstituted divalent aryl group. $Rg_L$ represents a hydrogen atom, a substituted or unsubstituted alkyl group or a substituted or unsubstituted aryl group.

Examples of $Rg_L$ include a hydrogen atom, a methyl group, an ethyl group, a propyl group, a hexyl group, an octyl group, a phenyl group and a naphthyl group.

The group may be substituted with one or more monovalent substituents at any position of the alkyl or aryl groups. The monovalent substituent is, for example, the monovalent substituent described above. $Rg_L$ is preferably a substituted or unsubstituted alkyl group having 3 to 20 carbon atoms, or a substituted or unsubstituted aryl group having 6 to 14 carbon atoms; and more preferably a substituted or unsubstituted alkyl group having 6 to 12 carbon atoms, or a substituted or unsubstituted aryl group having 6 to 10 carbon atoms.

Namely, preferred examples of the divalent substituent -Lg- include -O-, $-O-CO-C_2H_4-CO-O-$, $-O-C_4H_8-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$, and $-NH-SO_2-C_3H_6-$.

When tb is 1, $X_3$ represents a hydrogen atom, a halogen atom, a hydroxyl group, a substituted or unsubstituted phenyl group, a substituted or unsubstituted alkyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group or a substituted or unsubstituted amino group.

When tb is 1, $X_3$ is preferably a hydrogen atom, a hydroxyl group, a chlorine atom, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon atoms, alkyloxycarbonyl group having 2 to 18 carbon atoms, an aryloxycarbonyl group having 7 to 24 carbon atoms, a sulfonic acid group, or a substituted or unsubstituted amino group having 1 to 16 carbon atoms.

$X_3$ is particularly preferably a hydrogen atom, a hydroxyl group, a substituted or unsubstituted alkoxy group having 1 to 18 carbon atoms, a sulfonic acid group, or a substituted or unsubstituted amino group having 1 to 16 carbon atoms.

When tb is 2, $X_3$ represents a divalent substituent.

When tb is 2, examples of $X_3$ include the same examples as the above-described divalent substituent -L-. When tb is 2, $X_3$ is preferably $-CH_2-$, $-C_4H_8-$,$-O-C_4H_8-O-$, $-O-CO-C_2H_4-CO-O-$, or $-NH-CO-C_3H_6-CO-NH-$.

In Formula (IVb), tb is particularly preferably 1.

Namely, the component of Formula (IVb) is preferably combined as follows.

Specifically, when tb is 1, a preferable combination is that $X_1$ is a hydrogen atom, a hydroxyl group, a substituted or unsubstituted alkoxy group having 1 to 18 carbon atoms, a sulfonic acid group, or a substituted or unsubstituted amino group having 1 to 16 carbon atoms;

Lg is -O-, $-O-CO-C_2H_4-CO-O-$, $-O-C_4H_8-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$, $-NH-SO_2-C_3H_6-$, or a single bond; and

$X_3$ is a hydrogen atom, a hydroxyl group, a chlorine atom, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon atoms, an alkyloxycarbonyl group having 2 to 18 carbon atoms, an aryloxycarbonyl group having 7 to 24 carbon atoms, a sulfonic acid group, or a substituted or unsubstituted amino group having 1 to 16 carbon atoms.

When tb is 2, a preferable combination is that

$X_1$ is a hydrogen atom, a hydroxyl group, a substituted or unsubstituted alkoxy group having 1 to 18 carbon atoms, a sulfonic acid group, or a substituted or unsubstituted amino group having 1 to 16 carbon atoms;

Lg is -O-, $-O-CO-C_2H_4-CO-O-$, $-O-C_4H_8-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$, $-NH-SO_2-C_3H_6-$, or a single bond; and

$X_3$ is $-CH_2-$, $-C_4H_8-$, $-O-C_4H_8-O-$, $-O-CO-C_2H_4-CO-O-$, or $-NH-CO-C_3H_6-CO-NH-$.

Typical examples of the benzophenone-series compound include 2,4-dihydroxybenzophenone, 2-hydroxy-4-methoxybenzophenone, 2-hydroxy-4-octyloxybenzophenone, 2-hydroxy-4-decyloxybenzophenone, 2-hydroxy-4-dodecyloxybenzophenone, 2-hydroxy-4-benzyloxybenzophenone, 2-hydroxy-4-(2-hydroxy-3-methacryloxypropoxy)benzophenone, 2-hydroxy-4-methoxy-5-sulfobenzophenone, 2-hydroxy-4-methoxy-5-sulfobenzophenone trihydrate, 2-hydroxy-4-methoxy-2'-carboxybenzophenone, 2-hydroxy-4-octadecyloxybenzophenone, 2-hydroxy-4-diethylamino-2'-hexyloxycarbonylbenzophenone, 2,2'-dihydroxy-4-methoxybenzophenone, 2,2',4,4-tetrahydroxybenzophenone, 2,2'-dihydroxy-4,4'-dimethoxybenzophenone, and 1,4-bis(4-benzyloxy-3-hydroxyphenoxy)butane.

The benzoxazinone-series compound is preferably a compound having an effective absorption wavelength of approximately 270 to 380 nm, and is represented by the following Formula (V).

Formula (V)

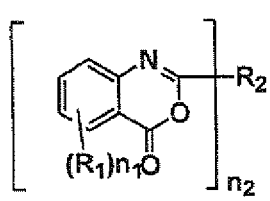

(In the above Formula (V), $R_1$ represents a substituent. $n_1$ is an integer of 0 to 4. $R_2$ represents a $n_2$-valent substituent or linking group. $n_2$ is an integer of 1 to 4.)

In the above Formula (V), $R_1$ represents a substituent. Examples of the substituent include the same as those recited as examples of the substituent involved in the above-described substituted alkyl group, substituted alkenyl group,

substituted alkynyl group, and substituent of the alkyl moiety of the above-described substituted aralkyl group. $R_1$ is preferably a halogen atom, an alkyl group, an alkenyl group, an alkynyl group, an aryl group, a cyano group, a hydroxy group, a nitro group, a carboxyl group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group, an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, an acyl group, an aryloxycarbonyl group, an alkoxycarbonyl group, a carbamoyl group, an imido group, a phosphino group, a phosphinyl group, a phosphinyloxy group, a phosphinylamino group, or a silyl group; more preferably a halogen atom, an alkyl group, an aryl group, a cyano group, a hydroxy group, a nitro group, a carboxyl group, an alkoxy group, an aryloxy group, a silyloxy group, a heterocyclic oxy group, an acyloxy group, a carbamoyloxy group, an amino group, an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, a carbamoyl group, an imido group, a phosphino group, a phosphinyl group, a phosphinyloxy group, a phosphinylamino group, or a silyl group; further preferably a halogen atom, an alkyl group, an aryl group, a hydroxy group, an alkoxy group, an aryloxy group, an amino group, a mercapto group, an alkylthio group, an arylthio group, a sulfamoyl group, a sulfo group, an alkyl- or aryl-sulfinyl group, or an alkyl- or aryl-sulfonyl group; further preferably a halogen atom, an alkyl group, an aryl group, an alkoxy group, an aryloxy group, an alkylthio group, or an arylthio group; further preferably a halogen atom, an alkyl group having 1 to 20 carbon atoms, an aryl group having 6 to 20 carbon atoms, an alkoxy group having 1 to 20 carbon atoms, an aryloxy group having 6 to 20 carbon atoms, an alkylthio group having 1 to 20 carbon atoms, or an arylthio group having 6 to 20 carbon atoms; further preferably a chlorine atom, a fluorine atom, a bromine atom, an alkyl group having 1 to 8 carbon atoms, an aryl group having 6 to 10 carbon atoms, an alkoxy group having 1 to 8 carbon atoms, an aryloxy group having 6 to 10 carbon atoms, an alkylthio group having 1 to 8 carbon atoms, or an arylthio group having 6 to 10 carbon atoms; and further preferably a chlorine atom, a fluorine atom, an alkyl group having 1 to 4 carbon atoms, or an alkoxy group having 1 to 4 carbon atoms.

$n_1$ is preferably an integer of 0 to 3, more preferably an integer of 0 to 2, and further more preferably 0 or 1. $n_1$ is most preferably 0, which means that the benzene ring has no substituent.

$R_2$ represents a $n_2$-valent substituent or linking group. Examples of the substituent include the same as those recited as examples of the substituent involved in the above-described substituted alkyl group, substituted alkenyl group, substituted alkynyl group, and substituent of the alkyl moiety of the above-described substituted aralkyl group. the linking group is a substituent further having one or more of linking bond. $R_2$ is preferably an aliphatic group, an aromatic group, or a linking group in which the aliphatic group and/or the aromatic group have (has) additional bond (s). $R_2$ is more preferably an alkyl group, an alkenyl group, an alkynyl group, an aryl group, or a divalent, trivalent, or tetravalent linking group each derived from these groups, still more preferably an alkyl group, an alkenyl group, an aryl group, and a divalent or trivalent linking group each derived from these groups, still more preferably an alkyl group having 1 to 20 carbon atoms, an alkenyl group having 2 to 20 carbon atoms, an aryl group having 6 to 20 carbon atoms, and a divalent or trivalent linking group each derived from these groups, still more preferably an alkyl group having 1 to 8 carbon atoms, an alkenyl group having 2 to 8 carbon atoms, an aryl group having 6 to 12 carbon atoms, and a divalent or trivalent linking group each derived from these groups, still more preferably an alkyl group having 1 to 8 carbon atoms, an aryl group having 6 to 12 carbon atoms, and a divalent or trivalent linking group each derived from these groups, still more preferably methyl, ethyl, propyl, butyl, isopropyl, 2-butyl, benzyl, phenyl, 2-naphthyl, ethylene, trimethylene, 1,2-propylene, tetramethylene, 1,2-phenylene, 1,3-phenylene, 1,4-phenylene, 2,6-naphthylene, and benzene-1,3,5,-yl, still more preferably methyl, ethyl, benzyl, phenyl, ethylene, trimethylene, 1,3-phenylene, 1,4-phenylene, and benzene-1,3,5,-yl, still more preferably ethylene, trimethylene, 1,3-phenylene, 1,4-phenylene, and benzene-1,3,5,-yl, and most preferably 1,4-phenylene.

$n_2$ is preferably an integer of 1 to 3, more preferably 2 or 3, and most preferably 2.

Representative examples of the above-described benzoxadinone-series compound include 2,2'-(p-phenylene) di-3,1-benzoxadine-4-on.

The salicylic acid-series compound above is preferably a compound having an effective absorption wavelength of approximately 290 to 330 nm, and typical examples thereof include phenyl salicylate, 4-t-butylphenyl salicylate, 4-octylphenyl salicylate, dibenzoylresorcinol, bis(4-t-butylbenzoyl)resorcinol, benzoylresorcinol, 2,4-di-t-butylphenyl 3,5-di-t-butyl-4-hydroxysalicylate, and hexadecyl 3,5-di-t-butyl-4-hydroxysalicylate.

The acrylate-series compound above is preferably a compound having an effective absorption wavelength of approximately 270 to 350 nm, and typical examples thereof include 2-ethylhexyl 2-cyano-3,3-diphenylacrylate, ethyl 2-cyano-3,3-diphenylacrylate, isooctyl 2-cyano-3,3-diphenylacrylate, hexadecyl 2-cyano-3-(4-methylphenyl)acrylate, methyl 2-cyano-3-methyl-3-(4-methoxyphenyl)cinnamate, butyl 2-cyano-3-methyl-3-(4-methoxyphenyl)cinnamate, methyl 2-carbomethoxy-3-(4-methoxyphenyl)cinnamate 2-cyano-3-(4-methylphenyl)acrylate salt, 1,3-bis(2'-cyano-3,3'-diphenylacryloyl)oxy)-2,2-bis(((2'-cyano-3,3'-diphenylacryloyl)oxy)methyl)propane, and N-(2-carbomethoxy-2-cyanovinyl)-2-methylindoline.

The oxalic diamide-series compound above is preferably a compound having an effective absorption wavelength of approximately 250 to 350 nm, and typical examples thereof include 4,4'-dioctyloxyoxanilide, 2,2'-dioctyloxy-5,5'-di-t-butyloxanilide, 2,2'-didodecyloxy-5,5'-di-t-butyloxanilide, 2-ethoxy-2'-ethyloxanilide, N,N'-bis(3-dimethylaminopropyl) oxamide, 2-ethoxy-5-t-butyl-2'-ethyloxanilide, and 2-ethoxy-2'-ethyl-5,4'-di-t-butyloxanilide.

The ultraviolet absorbent (B) is particular preferably a compound selected from the following compound group B. The compound group B includes the following compounds (II-1) to (V-1).

[1] Compound represented by Formula (IIa) described above

(II-1) 2-(2'-hydroxy-3',5'-di-t-amylphenyl)benzotriazole
(II-2) 2-(3-t-butyl-5-methyl-2-hydroxyphenyl)-5-chlorobenzotriazole
(II-3) 2-(2-hydroxy-5-t-octylphenyl)benzotriazole
(II-4) 2-ethylhexyl-3-[3-t-butyl-4-hydroxy-5-(5-chloro-2H-benzotriazole-2-yl)phenyl] propionate
(II-5) 2-(2H-benzotriazole-2-yl)-6-dodecyl-4-methyl-phenol
(II-6) 2-(2H-benzotriazole-2-yl)-3-t-butylphenol
(II-7) 2-(2H-benzotriazole-2-yl)-6-(1-methyl-1-phenylethyl)-4-(1,1-3,3-tetramethylbutyl)phenol
(II-8) 2-(2H-benzotriazole-2-yl)-3-methylphenol
(II-9) 2-(2H-benzotriazole-2-yl)-6-dodecyl-4-methyl-phenol

[2] Compound represented by Formula (IIb) described above

(II-10) 2,2'-methylene-bis[6-(2H-benzotriazole-2-yl)-4-(1,1,3,3-tetramethylbutyl)phenol]

[3] Compound represented by Formula (III) described above

(III-1) 2,4-bis(2-hydroxy-4-butoxyphenyl)-6-(2,4-dibutoxyphenyl)-1,3,5-triazine
(III-2) 2-[4-[(2-hydroxy-3-(2'-ethyl)hexyl)oxy]-2-hydroxyphenyl]-4,6-bis(2,4-dimethylphenyl)-1,3,5-triazine
(III-3) 2-[4-[(2-hydroxy-3-(2'-ethyl)hexyl)oxy]-2-hydroxyphenyl-4,6-bis(2,4-dimethylphenyl)-1,3,5-triazine
(III-4) 2-(4,6-diphenyl-1,3,5-triazin-2-yl)-5-hexyloxyphenol (III-5) bisethylhexyloxyphenol methoxyphenyltriazine

[4] Compound represented by Formula (IV) described above

(IV-1) hexyl 2-(4-diethylamino-2-hydroxybenzoyl)benzoate
(IV-2) 2,2'-dihydroxy-4,4'-dimethoxybenzophenone
(IV-3) 2-hydroxy-4-methoxybenzophenone
(IV-4) 1,4-bis(4-benzoyl-3-hydroxyphenoxy)butane
(IV-5) 2-hydroxy-4-octoxybenzophenone
(IV-6) 2-hydroxy-4-methoxybenzophenone-5-sulfonic acid
(IV-7) 2,2',4,4'-tetrahydroxybenzophenone

[5]Compound represented by Formula (V) described above

(V-1) 2,2'-(p-phenylene)di-3,1-benzoxazinone-4-on

The compound (II-1) has the following structure, and is commercially available as trade name Tinuvin 328 (manufactured by Ciba Specialty Chemicals).
The compound (II-2) has the following structure, and is commercially available as trade name Tinuvin 326 (manufactured

by Ciba Specialty Chemicals).
The compound (II-3) has the following structure, and is commercially available as trade name Tinuvin 329 (manufactured by Ciba Specialty Chemicals).
The compound (II-4) has the following structure, and is commercially available as trade name Tinuvin 109 (manufactured by Ciba Specialty Chemicals).
The compound (II-5) has the following structure, and is commercially available as trade name Tinuvin 171 (manufactured by Ciba Specialty Chemicals).
The compound (II-6) has the following structure, and is commercially available as trade name Tinuvin PS (manufactured by Ciba Specialty Chemicals).
The compound (II-7) has the following structure, and is commercially available as trade name Tinuvin 928 (manufactured by Ciba Specialty Chemicals).
The compound (II-8) has the following structure, and is commercially available as trade name Tinuvin P (manufactured by Ciba Specialty Chemicals).
The compound (II-9) has the following structure, and is commercially available as trade name Tinuvin 234 (manufactured by Ciba Specialty Chemicals).
The compound (II-10) has the following structure, and is commercially available as trade name Tinuvin 360 (manufactured by Ciba Specialty Chemicals).

The compound (III-1) has the following structure, and is commercially available as trade name Tinuvin 460 (manufactured by Ciba Specialty Chemicals).
The compound (III-2) has the following structure, and is commercially available as trade name Cyasorb UV-116 (manufactured by CYTEC Company Ltd.).
The compound (III-3) has the following structure, and is commercially available as trade name Tinuvin 405 (manufactured by Ciba Specialty Chemicals).
The compound (III-4) has the following structure, and is commercially available as trade name Tinuvin 1577 (manufactured by Ciba Specialty Chemicals).
The compound (III-5) has the following structure, and is commercially available as trade name Tinosorb S (manufactured by Ciba Specialty Chemicals).

The compound (IV-1) has the following structure, and is commercially available as trade name Uvinul A plus (manufactured by BASF Japan Ltd.).
The compound (IV-2) has the following structure, and is commercially available as trade name Uvinul 3049 (manufactured by BASF Japan Ltd.).
The compound (IV-3) has the following structure, and is commercially available as trade name Visorb 110 (manufactured by KYODO CHEMICAL CO., LTD.).
The compound (IV-4) has the following structure, and is commercially available as trade name Seesorb 151 (manufactured by SHIPRO KASEI KAISHA LTD.).
The compound (IV-5) has the following structure, and is commercially available as trade name Chimassorb 81 (manufactured by Ciba Specialty Chemicals).
The compound (IV-6) has the following structure, and is commercially available as trade name Uvinul MS40 (manufactured by BASF Japan Ltd.).
The compound (IV-7) has the following structure, and is commercially available as trade name Uvinul 3050 (manufactured by BASF Japan Ltd.).
The compound (V-1) has the following structure, and is commercially available as trade name Cyasorb UV-3638 (manufactured by CYTEC Company Ltd.).

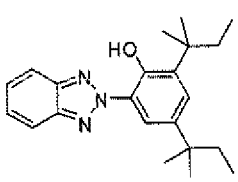
(II-1)

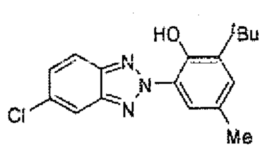
(II-2)

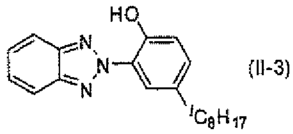
(II-3)

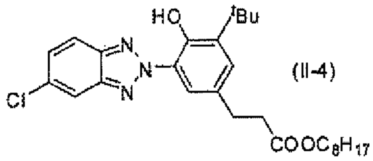
(II-4)

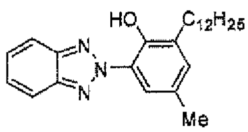
(II-5)

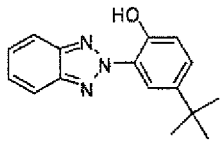
(II-6)

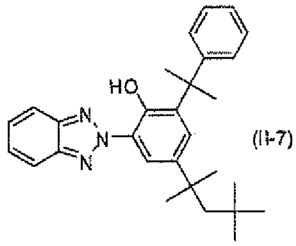
(II-7)

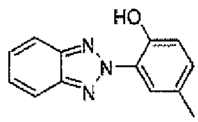
(II-8)

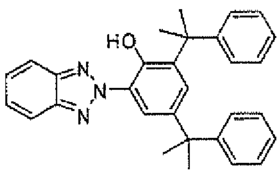
(II-9)

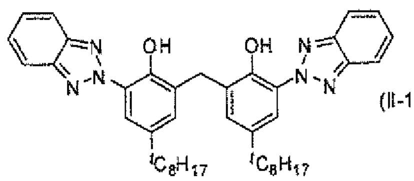
(II-10)

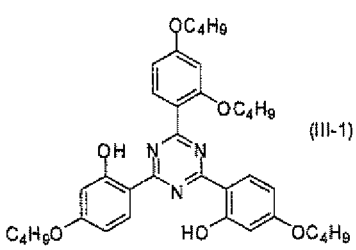
(III-1)

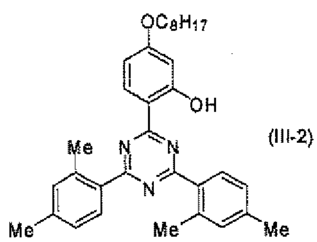
(III-2)

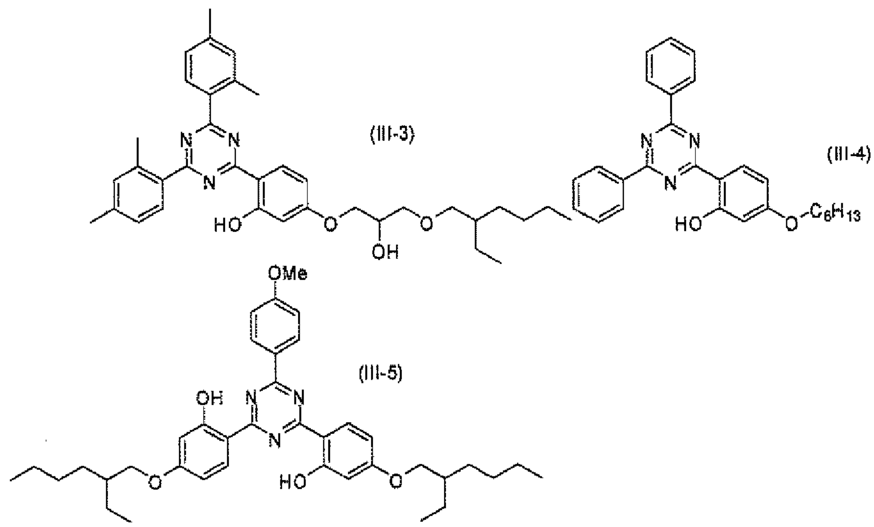

(III-3) (III-4)

(III-5)

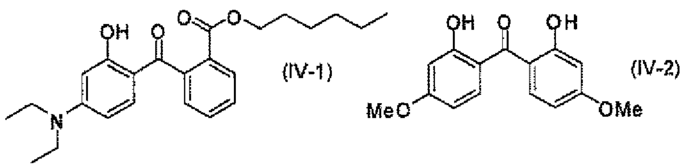

(IV-1) (IV-2)

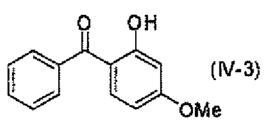

(IV-3)

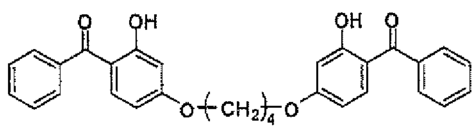

(IV-4)

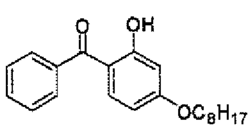

(IV-5)

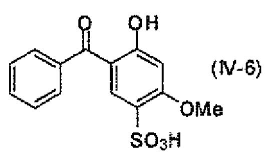

(IV-6)

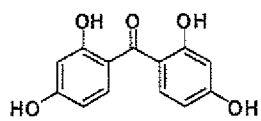

(IV-7)

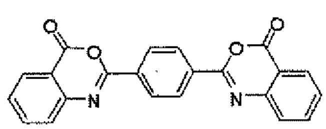

(V-1)

The ultraviolet absorbents (A) and (B) used in the present invention may be individually present, or may be connected to each other previously or by binding together with each other in a composition. Further, a polymerizable group may be bound with each of the ultraviolet absorbents (A) and (B) to form a polymerizable monomer, followed by polymerization of these monomers to form a copolymer including these monomers as a unit structure. Alternatively, these compounds may be used together with other monomers free of these ultraviolet absorbents (A) and (B) to form a copolymer. A preferable embodiment is that a composition is constructed by monomers, and a copolymer is formed by polymerization of the monomers at a desired stage.

The ultraviolet absorbent composition of the present invention may further contain a light stabilizer, or an antioxidant.
Preferable examples of the light stabilizer and the antioxidant include compounds described in JP-A-2004-117997. Specifically, compounds described on page 29, middle paragraph Nos. [0071] to [0111] of JP-A-2004-117997 are preferable. Especially, compounds represented by Formula (TS-I), (TS-II), (TS-IV), or (TS-V) described on the paragraph No. [0072] are preferable.
The ultraviolet absorbent composition of the present invention may be in any form, for example, liquid dispersion, solution, polymer material, and the like. The ultraviolet absorbent composition of the present invention may contain any other desirable components according to application, in addition to the ultraviolet absorbents (A) and (B).
The ultraviolet absorbent of the present invention is preferably in the state of dispersion in which the ultraviolet absorbent is dispersed in a dispersing medium. Hereinafter, the dispersion containing the ultraviolet absorbent of the present invention is described.
The medium for dispersing the ultraviolet absorbent of the present invention is arbitrary. Examples thereof include water, organic solvents, resins, resin solutions, and the like. These media may be used alone or in combination.
Examples of the organic solvents as the dispersing medium that can be used in the present invention include hydrocarbon-based solvents such as pentane, hexane and octane; aromatic-based solvents such as benzene, toluene and xylene; ether-based solvents such as diethylether and methyl-t-butylether; alcoholic-based solvents such as methanol, ethanol and isopropanol; ester-based solvents such as acetone, ethyl acetate and butyl acetate; ketone-based solvents such as methyl ethyl ketone; nitrile-based solvents such as acetonitrile and propionitrile; amide-based solvents such as N,N-dimethylformamide, N,N-dimethylacetamide and N-methylpyrrolidone; sulfoxide-based solvents such as dimethylsulfoxide; amine-based solvents such as triethylamine and tributylamine; carboxylic acid-based solvents such as acetic acid and propionic acid; halogen-based solvents such as methylene chloride and chloroform; and heterocycle-based solvents such as tetrahydrofuran and pyridine. These solvents may be used as a mixture at any rate.
Examples of the resins as the dispersing medium that can be used in the present invention include various known thermoplastic and thermosetting resins commonly used for production of molded article, sheet, film and others. Examples of the thermoplastic resins include polyethylene series resins, polypropylene series resins, poly(meth)acrylic ester series resins, polystyrene series resins, styrene-acrylonitrile series resins, acrylonitrile-butadiene-styrene series resins, polyvinyl chloride series resins, polyvinylidene chloride series resins, polyvinyl acetate series resins, polyvinylbutyral series resins, ethylene-vinyl acetate series copolymers, ethylene-vinylalcohol series resins, polyethylene terephthalate resins (PET), polybutylene terephthalate resins (PBT), liquid crystal polyester resins (LCP), polyacetal resins (POM), polyamide resins (PA), polycarbonate resins, polyurethane resins, and polyphenylene sulfide resins (PPS), and these resins may be used alone or as polymer blend or alloy of two or more. The resin may be used as a thermoplastic molding material containing a natural resin and additionally a filler such as glass fiber, carbon fiber, semi-carbonized fiber, cellulosic fiber or glass bead, a flame retardant, and the like. As needed, resin additives commonly used, such as polyolefin series resin fine powder, polyolefin series wax, ethylene bisamide wax, and metal soap, may be used alone or in combination.
When the ultraviolet absorbent is used together with a thermoplastic resin, the ultraviolet absorbent may be either added in a polymerization process of the thermoplastic resin or added after the polymerization. When the ultraviolet absorbent is added to the thermoplastic resin in a molten state after the polymerization, the ultraviolet absorbent may be added singly, or may be added in dispersed condition with a solvent, etc. In this situation, it may be appropriate that the solvent to be used does not make the resin being kneaded deteriorate but make the ultraviolet absorbent disperse.
Such a melt blending is possible by adding the ultraviolet absorbent at the temperature higher than a melting temperature of the polymer employing a melt blending apparatus such as uniaxis or dual axis extruder. In a case where a dispersion liquid is used for the melt blending, the blending is executable by removing an organic solvent after adding the dispersion liquid while pressurizing.
The ultraviolet absorbent may be added to the thermoplastic resin in the molten state followed by being kneaded in a film formation process. This method is preferable since it is possible to suppress a deterioration of the thermoplastic resin by reducing heat history.
When the thermoplastic resin is melt polymerizable thermoplastic polyester such as polyethylene terephthalate, polyethylenenaphthalate or so, the dispersion liquid of the ultraviolet absorbent may be added either before the polymerization or during the polymerization. The ultraviolet absorbent may be added singly, or may be added in preliminarily dispersed condition using a solvent. Regarding the solvent in this case, a solvent material for the polymer is preferable. The polymerization reaction may be executed in accordance with usual polymerization condition of the polymer.
The aimed ultraviolet absorbent containing polymer can be also obtained by adopting the thermoplastic resin containing the ultraviolet absorbent in relatively high concentration of 0.5 to 50% by mass prepared with the above-mentioned method as a masterbatch, and by further allowing the masterbatch to be kneaded into the thermoplastic resin to which the ultraviolet absorbent is not added yet.
Examples of the thermosetting resins include epoxy resins, melamine resins, and unsaturated polyester resins,

and these resins may be used as a thermosetting molding material containing a natural resin and additionally a filler, such as glass fiber, carbon fiber, semi-carbonized fiber, cellulosic fiber or glass bead, and a flame retardant.

The ultraviolet absorbent dispersion of the present invention may contain a dispersant, an antifoam, a preservative, an antifreezing agent, a surfactant or others, or combinations thereof. The dispersion may contain any other compounds additionally. Examples of them include dye, pigment, infrared absorbent, flavoring agent, polymerizable compound, polymer, inorganic material, metal and the like.

For example, a high-speed-agitation dispersing machine providing a high-sharing force or a dispersing machine imparting a high-strength ultrasonic may be used as the apparatus for preparation of the ultraviolet absorbent dispersion of the present invention. Specific examples thereof include colloid mill, homogenizer, capillary emulsifier, liquid siren, electromagnetic-distortion ultrasonic wave generator, emulsifier having a Pallmann whistle, and the like. The high-speed-agitation dispersing machine favorably used in the present invention is a dispersing machine in which a dispersing part is revolving in liquid at high speed (500 to 15,000 rpm, preferably 2,000 to 4,000 rpm) such as dissolver, polytron, homomixer, homoblender, keddy mill, or jet agitator. The high-speed-agitation dispersing machine that can be used in the present invention is also called a dissolver or a high-speed impeller dispersing machine, and, as described in JP-A-55-129136, a dispersing machine having impellers of saw-teeth shaped plate alternately bent in the vertical direction that are connected to the shaft revolving at high speed is also a favorable example.

Various methods may be used in preparation of an emulsified dispersion containing a hydrophobic compound. For example, in dissolving a hydrophobic compound in an organic solvent, the hydrophobic compound is dissolved in a solvent or a mixture of two or more arbitrarily selected from high-boiling point organic materials, water-immiscible low boiling point organic solvents and water-miscible organic solvents, and the solution is then dispersed in water or an aqueous hydrophilic colloid solution in the presence of a surfactant compound. The water-insoluble phase containing the hydrophobic compound and the aqueous phase may be mixed by the so-called normal mixing method of adding the water-insoluble phase into the agitated aqueous phase or by the reverse mixing method of adding the phases reversely.

The content of the ultraviolet absorbent in the dispersion containing the ultraviolet absorbent of the present invention may not be determined specifically, because it varies according to application and type of usage, and is thus arbitrary according to application. Preferably, the content is 0.001 to 50 mass%, more preferably 0.01 to 20 mass%, with respect to the total amount of the ultraviolet absorbent dispersion.

The ultraviolet absorbent composition of the present invention is preferably used in the state of a solution dissolved in a liquid medium. Hereinafter, the ultraviolet absorbent solution of the present invention is described. The liquid dissolving the ultraviolet absorbent composition of the present invention is arbitrary. It is, for example, water, an organic solvent, a resin, a resin solution, or the like. Examples of the organic solvent, the resin, and the resin solution include those described above as the dispersing medium. These may be used alone or in combination.

The solution of the ultraviolet absorbent composition of the present invention may contain any other compounds additionally. Examples thereof include dye, pigment, ultraviolet absorbent, infrared absorbent, flavoring agent, polymerizable compound, polymer, inorganic material, metal and the like. Components other than the ultraviolet absorbent composition of the present invention may not necessarily be dissolved.

The content of the ultraviolet absorbent composition in the ultraviolet absorbent solution of the present invention may not be determined specifically, because it varies according to application and type of usage, and thus the concentration is arbitrary according to application. The concentration is preferably 0.001 to 30 mass% with respect to the entire solution, more preferably 0.01 to 10 mass%. A solution at higher concentration may be prepared in advance and diluted at a desired stage before use. The dilution solvent can be selected arbitrarily from the solvents described above.

The polymer composition is used in preparation of the polymer material of the present invention. The polymer composition used in the preesent invention contains a polymer substance described below and the ultraviolet absorbent according to the present invention.

The ultraviolet absorbent composition of the present invention can be contained in the polymer substance in various methods. When the ultraviolet absorbent composition of the present invention is compatible with the polymer substance, the ultraviolet absorbent composition of the present invention may be added to the polymer substance directly. The ultraviolet absorbent composition of the present invention may be dissolved in an auxiliary solvent compatible with the polymer substance, and then the obtained solution be added to the polymer substance. The ultraviolet absorbent composition of the present invention may be dispersed in a high-boiling point organic solvent or a polymer, and the obtained dispersion be added to the polymer substance.

The boiling point of the high-boiling point organic solvent is preferably 180˚C or higher, more preferably 200˚C or higher. The melting point of the high-boiling point organic solvent is preferably 150˚C or lower, more preferably 100˚C or lower. Examples of the high-boiling point organic solvents include phosphoric esters, phosphonic esters, benzoic esters, phthalic esters, fatty acid esters, carbonate esters, amides, ethers, halogenated hydrocarbons, alcohols and paraffins. Phosphoric esters, phosphonic esters, phthalic ester, benzoic esters and fatty acid esters are preferable.

The method of adding the ultraviolet absorbent according to the present invention is determined, by reference to the description in JP-A-58-209735, JP-A-63-264748, JP-A-4-191851, JP-A-8-272058, and British Patent No. 2016017A.

The content of the ultraviolet absorbent composition in the ultraviolet absorbent solution of the present invention may not be determined specifically, because it varies according to application and type of usage, and thus the concentration is arbitrary according to application. The concentration in the polymer material is preferably 0.001 to 30 mass%, more preferably 0.01 to 10 mass%.

The ultraviolet absorbent composition of the present invention is preferably used for a polymer material. Hereinafter, the polymer material of the present invention is described.

In the present invention, although practically sufficient ultraviolet-shielding effect is obtained only with the ultraviolet absorbent compound of the present invention, a white pigment which has higher hiding power such as titanium oxide may be used in the case where further strictness is demanded. In addition, a trace (0.05 mass% or less) amount of colorant may be used additionally, if the appearance or the color tone is of a problem or as needed. Alternatively, a fluorescent brightener may be used additionally for applications demanding transparency or whiteness. Examples of the fluorescent brighteners include commercialized products, the compounds represented by Formula [1] and typical exemplary compounds 1 to 35 described in JP-A-2002-53824, and the like.

Hereinafter, the polymer substance that can be used in the polymer composition is described. The polymer substance is a natural or synthetic polymer or copolymer. Examples thereof include the followings:

<1> Monoolefinic and diolefinic polymers such as polypropylene, polyisobutylene, polybut-1-ene, poly-4-methyl pent-1-ene, polyvinylcyclohexane, polyisoprene and polybutadiene; cycloolefin polymers such as of cyclopentene or norbornene; polyethylenes (crosslinkable as needed) such as high-density polyethylene (HDPE), high-density and high-molecular weight polyethylene (HDPE-HMW), high-density and ultrahigh molecular weight polyethylene (HDPE-UHMW), medium-density polyethylene (MDPE), low-density polyethylene (LDPE), and linear low-density polyethylene (LLDPE), (VLDPE) and (ULDPE).

Polyolefins, namely, polymers of the monoolefins exemplified in the paragraph above, preferably polyethylene and polypropylene, may be prepared by various methods, particularly by the following methods:

a) Radical polymerization (normally under high pressure and elevated temperature), and

b) Catalytic polymerization normally by using one or more metals in the groups IVb, Vb, VIb and VIII of the Periodic Table. The metal is normally bound to one or more ligands, typically π- or σ-coordinating groups such as oxide, halide, alcoholate, ester, ether, amine, alkyl, alkenyl and/or aryl. The metal complex is in the free state or immobilized on a base material such as activated magnesium chloride, titanium (III) chloride, alumina or silicon oxide. The catalyst may be soluble or insoluble in the polymerization medium. The catalyst may be used as it is in polymerization or in combination with another activating agent, such as metal alkyl, metal hydride, metal alkyl halide, metal alkyl oxide or metal alkyloxane, the metal being an element in the groups Ia, IIa and/or IIIa of the Periodic Table. The activating agent may be modified properly with an other ester, ether, amine or silylether group. Such a catalyst system is normally called Philips, Standard Oil-Indiana, Ziegler (Natta), TNZ (Du Pont), metallocene or single site catalyst (SSC).

<2> Mixture of the polymers described in <1> above such as a mixture of a polypropylene and a polyisobutylene, a mixture of a polypropylene and a polyethylene (such as PP/HDPE and PP/LDPE), and a mixture of different type of polyethylenes (such as LDPE/HDPE).

<3> Copolymers of a monoolefin and a diolefin, or copolymers of a monoolefin or diolefin with another vinyl monomer such as ethylene/propylene copolymer, a mixture of linear low-density polyethylene (LLDPE) and its low-density polyethylene (LDPE), propylene/but-1-ene copolymer, propylene/isobutylene copolymer, ethylene/but-1-ene copolymer, ethylene/hexene copolymer, ethylene/methylpentene copolymer, ethylene/heptene copolymer, ethylene/octene copolymer, ethylene/vinylcyclohexane copolymer, ethylene/cycloolefin copolymer (for example, ethylene/norbomene such as COC (Cyclo-Olefin Copolymer), ethylene/1-olefin copolymer producing 1-olefin in situ, propylene/butadiene copolymer, isobutylene/isoprene copolymer, ethylene/vinylcyclohexene copolymer, ethylene/alkyl acrylate copolymer, ethylene/alkyl methacrylate copolymer, ethylene/vinyl acetate copolymer or ethylene/acrylic acid copolymer and the salts thereof (ionomers); and terpolymers of ethylene and propyrene with diene such as hexadiene, dicyclopentadiene or ethylidene-norbornene; and mixtures of such copolymers and the polymer described in the above 1) such as polypropylene/ethylene-propylene copolymer, LDPE/ethylene-vinyl acetate copolymer (EVA), LDPE/ethylene-acrylic acid copolymer (EAA), LLDPE/EVA, LLDPE/EAA and alternating or random polyalkylene/carbon monoxide copolymer and the mixture thereof with other polymer such as polyamide.

<4> Mixtures of hydrocarbon resins (for example, having 5 to 9 carbon atoms) containing hydrogenated derivatives (such as tackifier) and polyalkylene and starch.

The homopolymers and copolymers described in <1> to <4> above may have any steric structure, such as syndiotactic, isotactic, hemiisotactic and atactic; and atactic polymers are preferable. Stereoblock polymers are also included.

<5> Polystyrene, poly(p-methylstyrene), and poly($\alpha$-methylstyrene).

<6> Aromatic homopolymers and copolymers prepared from aromatic vinyl monomers including all isomers of styrene, $\alpha$-methylstyrene, and vinyltoluene, in particular all isomers of p-vinyltoluene, ethylstyrene, propylstyrene, vinyl biphenyl, vinylnaphthalene, and vinylanthracene, and the mixture thereof. The homopolymers and copolymers may have any steric structure, such as syndiotactic, isotactic, hemiisotactic and atactic; and atactic polymers are preferable. Stereoblock polymers are also included.

<6a> Copolymers selected from ethylene, propylene, dienes, nitriles, acids, maleic anhydride, maleimide, vinyl acetate and vinyl chloride or its acryl derivative and the mixture thereof containing the aromatic vinyl monomers or comonomers. Examples thereof include styrene/butadiene, styrene/acrylonitrile, styrene/ethylene (copolymer), styrene/alkyl methacrylate, styrene/butadiene/alkyl acrylate, styrene/butadiene/alkyl methacrylate, styrene/maleic anhydride, and styrene/acrylonitrile/methyl acrylate; styrene copolymers and other polymers including high shock-resistant mixtures such as polyacrylate, diene polymer, and ethylene/propylene/diene terpolymer; and styrene block copolymers such as styrene/butadiene/styrene, styrene/isoprene/styrene, styrene/ethylenelbutylene/styrene and styrene/ethylene/propylene/styrone.

<6b> Hydrogenated aromatic polymers prepared from the hydrogenated polymers described in <6>, in particular polycyclohexylethylene (PCHE), often called polyvinylcyclohexane (PVCH), prepared by hydrogenation of atactic polystyrene.

<6c> Hydrogenated aromatic polymers prepared by hydrogenation of the polymers described in <6a> above.

The homopolymers and copolymers may have any steric structure, such as syndiotactic, isotactic, hemiisotactic and atactic, and atactic polymers are preferable. Stereoblock polymers are also included.

<7> Graft copolymers of an aromatic vinyl monomer such as styrene or $\alpha$-methylstyrene, including graft copolymers of polybutadiene/styrene; polybutadienestyrene or polybutadiene-acrylonitrile copolymer/styrene; polybutadiene/styrene and acrylonitrile (or methacrylonitrile); polybutadiene/styrene, acrylonitrile and methyl methacrylate; polybutadiene/styrene and maleic anhydride; polybutadiene/styrene, acrylonitrile and maleic anhydride or maleimide; polybutadiene/styrene and maleimide; polybutadiene/styrene and alkyl acrylate or methacrylate; ethylene/propylene/diene terpolymer/styrene and acrylonitrile; polyalkyl acrylate or polyalkyl methacrylate/styrene and acrylonitrile; acrylate/butadiene copolymer/styrene and acrylonitrile; and mixtures thereof with the copolymers described in <6> above such as known copolymer mixtures of ABS, SAN, MBS, ASA and AES polymer.

<8> Halogen-containing polymers such as polychloroprene, chlorinated rubber, chlorinated or brominated copolymers of isobutylene-isoprene (halobutyl rubbers), chlorinated or sulfochlorinated polyethylene, ethylene-chlorinated ethylene copolymer, and epichlorohydrin homopolymer and copolymers; in particular, polymers of a halogen-containing vinyl compound such as polyvinyl chloride, polyvinylidene chloride, polyvinyl fluoride, polyvinylidene fluoride, and copolymers thereof such as vinyl chloride/vinylidene chloride, vinyl chloride/vinyl acetate or vinylidene chloride/vinyl acetate copolymer.

<9> Polymers derived from α,β-unsaturated acid and the derivatives thereof such as polyacrylates and polymethacrylates; polymethyl methacrylate, polyacrylamide and polyacrylonitrile modified with butyl acrylate to improve high-impact resistance.

<10> Copolymers of the monomers described in <9> above or with another unsaturated monomer such as acrylonitrile/butadiene copolymer, acrylonitrile/alkyl acrylate copolymer, acrylonitrile/alkoxyalkyl acrylate or acrylonitrile/vinyl halide copolymer and acrylonitrile/alkyl methacrylate/butadiene terpolymer.

<11> Polymers derived from an unsaturated alcohol and an amine, and acyl derivatives or acetals thereof such as polyvinylalcohol, polyvinyl acetate, polyvinyl stearate, polyvinyl benzoate, polyvinyl maleate, polyvinylbutyral, polyallyl phthalate and polyallylmelamine; and copolymers thereof with the olefin described in <1> above.

<12> Homopolymers and copolymers of cyclic ether such as polyalkylene glycols, polyethyleneoxide, polypropyleneoxide or bisglycidylether, and the copolymers thereof.

<13> Polyoxymethylene and polyacetals such as a polyoxymethylene containing ethyleneoxide as the comonomer; a thermoplastic polyurethane, polyacetals modified with acrylate or MBS.

<14> Mixtures of polyphenyleneoxide and sulfide, and those of polyphenyleneoxide and styrene polymer or polyamide.

<15> Polyurethanes derived from a polyether, polyester or polybutadiene having a hydroxyl group terminal and an aliphatic or aromatic polyisocyanate, and the precursors thereof.

<16> Polyamides and copolyamides derived from a diamine and a dicarboxylic acid and/or aminocarboxylic acid or the corresponding lactam, such as polyamide 4, polyamide 6, polyamides 6/6, 6/10, 6/9, 6/12, 4/6 and 12/12, polyamide 11, polyamide 12, and an aromatic polyamide from m-xylenediamine and adipic acid; polyamides prepared from hexamethylenediamine and isophthalic and/or terephthalic acid, in the presence or absence of a modifying agent elastomer such as poly-2,4,4-trimethylhexamethylene terephthalamide and poly-m-phenylene isophthalamide; block copolymers of the polyamides above with polyolefin, olefin copolymer, ionomer or chemically bonded or grafted elastomer; block copolymers with polyether such as polyethylene glycol, polypropylene glycol or polytetramethylene glycol; polyamides or copolyamides modified with EPDM or ABS; and polyamides condensed during processing (RIM polyamides).

<17> Polyurea, polyimide, polyamide-imide, polyether imide, polyester-imide, polyhydantoin and polybenzimidazole.

<18> Polyesters derived from a dicarboxylic acid and a diol and/or a hydroxycarboxylic acid or the corresponding lactone such as polyethylene terephthalate, polybutylene terephthalate, poly-1,4-dimethylolcyclohexane terephthalate, polyalkylene naphthalate (PAN) and polyhydroxybenzoate; block copolyether esters derived from hydroxyl terminal polyethers; and polyesters modified with polycarbonate or MBS; the polyesters and polyester copolymers specified in U.S. Patent No. 5,807,932 (2nd column, line 53) are also incorporated herein by reference.

<19> Polycarbonates and polyester carbonates.

<20> Polyketones.

<21> Polysulfones, polyether sulfones and polyether ketones.

<22> Crosslinked polymers derived from aldehyde as one component and phenol, urea and melamine as the other component such as phenol/formaldehyde resin, urea/formaldehyde resin and melamine/formaldehyde resin.

<23> Dry and non-dry alkyd resins.

<24> Unsaturated polyester resins derived from saturated and unsaturated dicarboxylic acids, a polyvalent alcohol, and vinyl compound as a crosslinking agent, and less flammable halogen-containing derivatives thereof.

<25> Substituted acrylates, for example, crosslinkable acrylic resins derived from epoxy acrylate, urethane acrylate or polyester acrylate.

<26> Alkyd, polyester and acrylate resins crosslinked with a melamine resin, urea resin, isocyanate, isocyanurate, polyisocyanate or epoxy resin.

<27> Crosslinked epoxy resins derived from an aliphatic, alicyclic, heterocyclic or aromatic glycidyl compound, for example, glycidyl ether products of bisphenol A or bisphenol F crosslinked with a common curing agent such as anhydride or amine in the presence or absence of an accelerator.

<28> Natural polymers such as cellulose, rubber, gelatin and chemically modified derivatives of their homologous series such as cellulose acetate, cellulose propionate and cellulose butyrate, and cellulose ethers such as methylcellulose; and rosins and the derivatives thereof.

<29> Polymer blends (polyblends) of the polymers described above such as PP/EPDM, polyamide/EPDM or ABS, PVCIEVA, PVC/ABS, PVC/MBS, PC/ABS, PBTP/ABS, PC/ASA, PC/PBT, PVC/CPE, PVC/acrylate, POM/thermoplastic PUR, PClthezanoplastic PUR, POM/acrylate, POM/MBS, PPO/HIPS, PPO/PA6.6 and copolymer, PA/HDPE, PA/PP, PA/PPO, PBT/PC/A-BS and PBT/PET/PC.

<30> Natural and synthetic organic materials of a pure monomeric compound or a mixture of the compounds such as mineral oils, animal and vegetable fats, oils and waxes, or synthetic ester (such as phthalate, adipate, phosphate or trimellitate)-based oils, fats and waxes, and mixtures thereof with a synthetic ester and mineral oil at any rate, mixtures typically used as a fiber-spinning composition, and the aqueous emulsions thereof.

<31> Aqueous emulsions of natural or synthetic rubber, for example, a natural latex or latexes of a carboxylated styrene/butadiene copolymer.

<32> Polysiloxanes, for example, the soft hydrophilic polysiloxane described in U.S. Patent No. 4,259,467 and the hard polyorganosiloxane described in U.S. Patent No. 4,355,147.

<33> Polyketimines in combination with an unsaturated acrylpolyacetoacetate resin or an unsaturated acrylic resin including urethane acrylate, polyester acrylate, vinyl or acrylic copolymers having a pendant unsaturated group, and acrylated melamines. The polyketimine is prepared from a polyamine and a ketone in the presence of an acid catalyst.

<34> Radiant ray-hardening compositions containing an ethylenically unsaturated monomer or oligomer and a polyunsaturated aliphatic oligomer.

<35> Epoxy melamine resins such as photostabilized epoxy resins crosslinked with a coetherified high-solid content melamine resin sensitive to epoxy groups, such as LSE-4103 (trade name, manufactured by Nionsanto).

The polymer substance for use in the present invention is preferably a synthetic polymer, more preferably a polyolefin, an acrylic polymer, polyester, polycarbonate, or a cellulose ester. Among them, polyethylene, polypropylene, poly(4-methylpentene), polymethyl methacrylate, polycarbonate, polyethylene terephthalate, polyethylene naphthalate, polybutylene terephthalate, and triacetylcellulose are particularly preferable.
The polymer substance for use in the present invention is preferably a thermoplastic resin.
The polymer material of the present invention may contain any additives such as antioxidant, photostabilizer, processing stabilizer, anti-aging agent, and compatibilizer, as needed in addition to the above polymer substance and the ultraviolet absorbent according to the present invention.
The polymer material of the present invention contains the above polymer substance. The polymer material of the present invention may be made only of the polymer substance, or may be formed by using the polymer substance dissolved in an arbitrary solvent.
The polymer material of the present invention is applicable to any application where synthetic resin is used, and particularly favorable to applications where there is possibility of exposure to light such as sunlight or ultraviolet light. Specific examples thereof include glass alternatives and their surface-coating agent; coating agents for the window glass, lighting glass and light-source-protecting glass of house, facility, vehicle, etc.; interior and exterior materials of house, facility vehicle, etc. and paints for them; materials for ultraviolet-emission sources such as fluorescent lamp and mercury lamp; materials for precision machines and electric and electronic devices; materials for shielding electromagnetic and other

waves emitted from various displays; containers or packaging materials for foods, chemicals, drugs etc.; agricultural and industrial sheet or film; anti-discoloring agent for print, dyed thing, colorant etc.; cosmetics such as anti-sunburn cream, shampoo, rinse, and hair dressing; apparel fiber products and the fibers; home interior products such as curtain, carpet and wall paper; medical devices such as plastic lens, contact lens and artificial eye; optical materials such as optical filter, prism, mirror, and photographic material; stationery products such as tape and ink; indicator display plates and devices and the surface-coating agents thereof, and the like.

The shape of the polymer material of the present invention may be flat, powder, spherical particle, crushed particle, masive continuous body, fiber, tube, hollow fiber, granule, plate, porous particle, or the other.

Since the polymer material of the present invention contains the ultraviolet absorbent composition, the polymer material is superior in light resistance (ultraviolet fastness), causing no precipitation or bleed out of the ultraviolet absorbent during long-term use. In addition, the polymer material of the present invention, which has superior long-wavelength ultraviolet absorption capacity, can be used as an ultraviolet-absorbing filter or container, for protection, for example, of an ultraviolet-sensitive compound therein. It is possible to obtain a molded article (such as container) of the polymer material of the present invention, for example, by molding the polymer substance by any molding method such as extrusion molding or injection molding. It is also possible to prepare a molded article coated with an ultraviolet-absorbing film made of the polymer material of the present invention, by coating and drying a solution of the polymer substance on a separately prepared molded article.

When the polymer material of the present invention is used as an ultraviolet-absorbing filter or film, the polymer substance is preferably transparent. Examples of the transparent polymer materials include cellulose esters (such as diacetylcellulose, triacetylcellulose (TAC), propionylcellulose, butyrylcellulose, acetyl propionyl cellulose, and nitrocellulose), polyamides, polycarbonates, polyesters (such as polyethylene terephthalate, polyethylene naphthalate, polybutylene terephthalate, poly-1,4-cyclohexane dimethylene terephthalate, polyethylene-1,2-diphenoxyethane-4,4'-dicarboxylate, and polybutylene terephthalate), polystyrenes (such as syndiotactic polystyrene), polyolefins (such as polyethylene, polypropylene, and polymethylpentene), polymethyl methacrylate, syndiotactic polystyrene, polysulfones, polyether sulfones, polyether ketones, polyether imides, polyoxyethylene, and the like. Preferable are cellulose esters, polycarbonates, polyesters, polyolefins, and acrylic resins. The polymer material of the present invention may be used as a transparent support, and the transmittance of the transparent support is preferably 80% or more, more preferably 86% or more.

Hereinafter, the packaging material containing the ultraviolet absorbent composition of the present invention is described. The packaging material containing the ultraviolet absorbent composition of the present invention may be a packaging material of any kind of polymer, as long as it contains the above-desribed ultraviolet absorbents (A) and (B). Examples thereof include the thermoplastic resins described in JP-A-8-208765; the polyvinylalcohols described in JP-A-8-151455; the polyvinyl chlorides described in JP-A-8-245849; the polyesters described in JP-A-10-168292 and JP-A-2004-285189; the heat-shrinkable polyesters described in JP-A-2001-323082; the styrene-based resins described in JP-A-10-298397; the polyolefins described in JP-A-11-315175, JP-A-2001-26081, and JP-A-2005-305745; the ROMP's described in JP-T-2003-524019; and the like. It may be, for example, the resin having a vapor-deposition thin film of an inorganic compound described in JP-A-2004-50460 or JP-A-2004-243674. It may be, for example, the paper coated with a resin containing an ultraviolet absorbent described in JP-A-2006-240734.

The packaging material containing the ultraviolet absorbent composition of the present invention may be that for packaging anything such as food, beverage, medicine, cosmetics, or individual health care product. Examples thereof include the food packaging materials described in JP-A-11-34261 and JP-A-2003-237825; the colored liquid packaging materials described in JP-A-8-80928; the liquid preparation-packaging materials described in JP-A-2004-51174; the medicine container packaging materials described in JP-A-8-301363 and JP-A-11-276550; the packaging materials for medical sterilization described in JP-A-2006-271781; the photographic photosensitive material packaging materials described in JP-A-7-287353; the photograph film packaging materials described in JP-A-2000-56433; the UV-hardening ink packaging materials described in JP-A-2a05-178832; the shrink labels described in JP-A-2003-200966 and JP-A-2006-323339; and the like.

The packaging material containing the ultraviolet absorbent composition of the present invention may be the transparent packaging material described, for example, in JP-A-2004-51174 or the light-shielding packaging material described, for example, in JP-A-2006-224317.

The packaging material containing the ultraviolet absorbent composition of the present invention may have ultraviolet light-shielding property as well as other properties, as described, for example, in JP-A-2001-26081 and JP-A-2005-305745. Examples thereof include the packaging materials having gas-barrier property described, for example, in JP-A-2002-160321; those containing an oxygen indicator as described, for example, in JP-A-2005-156220; those containing both an ultraviolet absorbent and a fluorescent brightener described, for example, in JP-A-2005-146278; and the like.

The packaging material containing the ultraviolet absorbent composition of the present invention may be prepared by any method. Examples of the method include the method of forming an ink layer described, for example, in

JP-A-2006-130807; the method of melt-extruding and laminating a resin containing an ultraviolet absorbent described, for example, in JP-A-2001-323082 and JP-A-2005-305745; the method of coating on a base film described, for example, in JP-A-9-142539; the method of dispersing an ultraviolet absorbent in an adhesive described, for example, in JP-A-9-157626; and the like.

Hereinafter, the container containing the ultraviolet absorbent composition of the present invention is described. The container containing the ultraviolet absorbent composition of the present invention may be a container of any kind of polymer, as long as it contains the ultraviolet absorbents (A) and (B). Examples thereof include the thermoplastic resin containers described in JP-A-8-324572; the polyester containers described in JP-A-2001-48153, JP-A-2005-105004, and JP-A-2006-1568; the polyethylene naphthalate containers described in JP-A-2000-238857; the polyethylene containers described in JP-A-2001-88815; the cyclic olefin-based resin composition containers described in JP-A-7-216152; the plastic containers described in JP-A-2001-270531; the transparent polyamide containers described in JP-A-2004-83858; and the like. It may be the paper container containing a resin described, for example, in JP-A-2001-114262 or JP-A-2041-213427. It may be, alternatively, the glass container having an ultraviolet-absorbing layer described, for example, in JP-A-7-242444, JP-A-8-133787, or JP-A-2005-320408.

The container containing the ultraviolet absorbent composition of the present invention is used as containers in various applications including food, beverage, medicine, cosmetics, individual health care product, shampoo and the like. Examples thereof include the liquid fuel-storing containers described in JP-A-5-139434; the golf ball containers described in JP-A-7-289665; the food containers described in JP-A-9-295664 and JP-A-2003-237825; the liquor containers described in JP-A-9-58687; the medicine-filling containers described in JP-A-8-155007; the beverage containers described in JP-A-8-324572 and JP-A-2006-298456; the oily food containers described in JP-A-9-86570; the analytical reagent solution containers described in JP-A-9-113494; the instant noodle containers described in JP-A-9-239910; the light-resistant cosmetic preparation containers described in JP-A-11-180474, JP-A-2002-68322, and JP-A-2005-278678; the medicine containers described in JP-A-11-276550; the high-purity chemical solution containers described in JP-A-11-290420; the liquid agent containers described in JP-A-2001-106218; the UV-hardening ink containers described in JP-A-2005-178832; the plastic ampoules described in WO 04/93775 pamphlet; and the like.

The container containing the ultraviolet absorbent composition of the present invention may have ultraviolet-shielding property as well as other properties, as described, for example, in JP-A-5-305975 and JP-A-7-40954. Examples of such containers include the antimicrobial containers described in JP-A-10-237312; the flexible containers described in JP-A-2000-152974; the dispenser containers described in JP-A-2002-264979; the biodegradable containers described in, for example, JP-A-2005-255736; and the like.

The container containing the ultraviolet absorbent composition of the present invention may be prepared by any method. Examples of the method include the two-layer stretching blow-molding method described in JP-A-2002-370723; the multilayer coextrusion blow-molding method described in JP-A-2001-88815; the method of forming an ultraviolet-absorbing layer on the external surface of an container described in JP-A-9-241407; the methods of using a shrinkable film described in JP-A-8-91385, JP-A-9-48935, JP-T-11-514387, JP-A-2000-66603, JP-A-2041-323082, JP-A-2005-105032, and WO 99/29490 pamphlet; the method of using a supercritical fluid described in JP-A-11-255925; and the like.

Hereinafter, the paint and the coated film containing the ultraviolet absorbent composition of the present invention is described. The paint containing the ultraviolet absorbent composition of the present invention may be a paint of any composition, as long as it contains the above-described ultraviolet absorbents (A) and (B). Examples thereof include those of acrylic resin series, urethane resin series, aminoalkyd resin series, epoxy resin series, silicone resin series, and fluororesin series. To these resins, a base compound, curing agent, diluent, leveling agent, cissing inhibitor or the like may be added.

For example, when an acrylic urethane resin or a silicon acrylic resin is selected as the transparent resin component, polyisocyanate etc. as the curing agent; a hydrocarbon-based solvent such as toluene or xylene, an ester-based solvent such as isobutyl acetate, butyl acetate and amyl acetate, and an alcohol-based solvent such as isopropyl alcohol and butyl alcohol as the diluent may be used. The acrylic urethane resin is an acrylic urethane resin obtained by reaction of a methacrylate, ester (typically, methyl methacrylate), hydroxyethyl methacrylate copolymer and a polyisocyanate. In such a case, the polyisocyanate is, for example, tolylene diisocyanate, diphenylmethane diisocyanate, polymethylene polyphenylene polyisocyanate, tolidine diisocyanate, naphthalene diisocyanate, hexamethylene diisocyanate, isophorone diisocyanate, xylylene diisocyanate, dicyclohexylmethane diisocyanate, hexamethylene diisocyanate and the like. Examples of other transparent resin components include polymethyl methacrylate, polymethyl methacrylate/styrene copolymer, polyvinyl chloride, polyvinyl acetate, and the like. In addition to these components, a leveling agent such as an acrylic or silicone resin, a silicone-based or acrylic cissing inhibitor, and others may be added as needed.

The paint containing the ultraviolet absorbent composition of the present invention may be used in any application. Examples thereof include the ultraviolet-shielding paints described in JP-A-7-26177, JP-A-9-169950, JP-A-9-221631, and JP-A-2002-80788; the ultraviolet- and near infrared-shielding paints described in JP-A-10-88039; the electromagnetic wave-shielding paints described in JP-A-2001-55541; the clear paints described in JP-A-8-81643; the

metallic paint compositions described in JP-A-2000-186234; the cationic electrodeposition paints described in JP-A-7-166112; the antimicrobial and lead-free cationic electrodeposition paints described in JP-A-2002-294165; the powder paints described in JP-A-2000-273362, JP-A-2001-279189, and JP-A-2002-271227; the aqueous intermediate-layer paints, aqueous metallic paints, and aqueous clear paints described in JP-A-2001-9357; the topcoat paints for automobile, construction, and civil work described in JP-A-2001-316630; the hardening paints described in JP-A-2002-356655; the coat-film forming compositions for use on plastic materials such as automobile bumper described in JP-A-2004-937; the paints for a metal plate described in JP-A-2004-2700; the hardening gradient coat films described in JP-A-2004-169182; the coating materials for an electric wire described in JP-A-2004-107700; the paints for automobile repair described in JP-A-6-49368; the anionic electrodeposition paints described in JP-A-2002-38084 and JP-A-2005-307161; the paints for an automobile described in JP-A-5-78606, JP-A-5-185031, JP-A-10-140089, JP-T-2000-509082, JP-T-2004-520284, and WO 2006/097201 pamphlet; the paints for a coated steel plate described in JP-A-6-1945; the paints for a stainless steel described in JP-A-6-313148; the lamp moth-repellent paints described in JP-A-7-3189; the UV-hardening paints described in JP-A-7-82454; the antimicrobial paints described in JP-A-7-118576; the eyestrain protection paints described in JP-A-2004-217727; the anti-fog paints described in JP-A-2005-314495; the ultra-weather-resistance paints described in JP-A-10-298493; the gradient paints described in JP-A-9-241534; the photocatalyst paints described in JP-A-2002-235028; the strippable paints described in JP-A-2000-345109; the concrete separation paints described in JP-A-6-346022; the anti-corrosion paints described in JP-A-2002-167545; the protective paints described in JP-A-8-324576; the water-repellent protective paints described in JP-A-9-12924; the anti-plate glass scattering paints described in JP-A-9-157581; the alkali-soluble protective paints described in JP-A-9-59539; the aqueous temporary protective paint compositions described in JP-A-2001-181558; the flooring paints described in JP-A-10-183057; the emulsion paints described in JP-A-2001-115080; the two-liquid aqueous paints described in JP-A-2001-262056; the one-liquid paints described in JP-A-9-263729; the UV-hardening paints described in JP-A-2001-288410; the electron beam-hardening paint compositions described in JP-A-2002-69331; the thermosetting paint compositions described in JP-A-2002-80781; the aqueous paints for baking lacquer described in JP-T-2003-525325; the powder paints and the slurry paints described in JP-A-2004-162021; the repair paints described in JP-A-2006-233010; the powder-paint aqueous dispersions described in JP-T-11-514689; the paints for a plastic article described in JP-A-2001-59068 and JP-A-2006-160847; the electron beam-hardening paints described in JP-A-2002-69331; and the like.

The paint containing the ultraviolet absorbent composition of the present invention generally contains a paint (containing a transparent resin component as the principal component) and an ultraviolet absorbent. The paint contains the ultraviolet absorbent preferably in an amount of 0 to 20 mass% with respect to the resin. The thickness of the film coated is preferably 2 to 1,000 μm, more preferably 5 to 200 μm. The method of coating the paint is arbitrary, and examples of the method include a spray method, a dipping method, a roller coating method, a flow coater method, a blow coating method, and the like. The drying after coating is preferably carried out at a temperature of approximately room temperature to 120°C for 10 to 90 minutes, although the condition may vary according to the paint composition.

The coated film containing the ultraviolet absorbent composition of the present invention is a coated film containing the ultraviolet absorbents (A) and (B), and formed by using the paint containing the ultraviolet absorbents.

Hereinafter, the ink containing the ultraviolet absorbent composition of the present invention is described. The ink containing the ultraviolet absorbent composition of the present invention may be any ink in any form, as long as it contains the above-described ultraviolet absorbents (A) and (B). For example, it may be dye ink, pigment ink, aqueous ink, solvent ink, or the like. It may be used in any application. Examples of the applications include the screen printing ink described in JP-A-8-3502; the flexographic printing ink described in JP-T-2006-521941; the gravure printing ink described in JP-T-2005-533915; the plane offset printing ink described in JP-T-11-504954; the letterpress printing ink described in JP-T-2005-533915; the UV ink described in JP-A-5-254277; the EB ink described in JP-A-2006-30596; and the like. Other examples thereof include the inkjet inks described in JP-A-11-199808, WO 99/67337 pamphlet, JP-A-2005-325150, JP-A-2005-350559, JP-A-2006-8811, and JP-T-2006-514130; the photochromic ink described in JP-A-2006-257165; the thermal transfer ink described in JP-A-8-108650; the masking ink described in JP-A-2005-23111; the fluorescence ink described in JP-A-2004-75888; the security ink described in JP-A-7-164729; the DNA ink described in JP-A-2006-22300; and the like.

Any product obtained by using the ink containing the ultraviolet absorbent composition of the present invention is also included in the present invention. Examples thereof include the print laminated films obtained by laminating the print, and the packaging materials and containers prepared by using the laminated film described in JP-A-2006-70190; the ink-receiving layer described in JP-A-2002-127596; and the like.

Hereinafter, the fiber containing the ultraviolet absorbent composition of the present invention is described. The fiber containing the ultraviolet absorbent composition of the present invention may be a fiber composed of any kind of polymer, as long as it contains the above-described ultraviolet absorbents (A) and (B). Examples thereof include the polyester fibers described in JP-A-5-117508, JP-A-7-119036, JP-A-7-196631, JP-A-8-188921, JP-A-10-237760, JP-A-2000-54287, JP-A-2006-299428, and JP-A-2006-299438; the polyphenylene sulfide fibers described in JP-A-2002-322360 and JP-A-2006-265770; the polyamide fibers described in JP-A-7-76580, JP-A-2001-348785, JP-A-2003-41434, and JP-A-2003-239136; the epoxy fibers described in WO 03/2661 pamphlet; the aramide fibers described in JP-A-10-251981; the polyurethane fibers described in JP-A-6-228816; the cellulosic fibers described in JP-T-2005-517822; and the like.

The fiber containing the ultraviolet absorbent composition of the present invention may be prepared by any method. Examples of the method include the method, as described in JP-A-6-228818, of processing a polymer previously containing the above-described ultraviolet absorbents (A) and (B) into fiber, and the methods, as described, for example, in JP-A-5-9870, JP-A-8-188921, and JP-A-10-1587, of processing a material processed in a fiber form with a solution containing the above-described ultraviolet absorbents (A) and (B). As described in JP-A-2002-212884 and JP-A-2006-16710, the fiber may be processed by using a supercritical fluid.

The fiber containing the ultraviolet absorbent composition of the present invention can be used in various applications. Examples thereof include the clothing described in JP-A-5-148703; the backing cloth described in JP-A-2004-285516; the underwear described in JP-A-2004-285517; the blanket described in JP-A-2003-339503; the hosiery described in JP-A-2004-11062; the synthetic leather described in JP-A-11-302982; the moth-repellent mesh sheet described in JP-A-7-289097; the mesh sheet for construction described in JP-A-10-1868; the carpet described in JP-A-5-256464; the moisture-permeable water-repellent sheet described in JP-A-5-193037; the nonwoven fabric described in JP-A-6-114991; the ultrafine fiber described in JP-A-11-247028; the fibrous sheet described in JP-A-2000-144583; the refreshing clothing described in JP-A-5-148703; the moisture-permeable water-repellent sheet described in JP-A-5-193037; the flame-resistant synthetic suede cloth structure described in JP-A-7-18584; the resin tarpaulin described in JP-A-8-41785; the filming agent, external wall material, and agricultural greenhouse described in JP-A-8-193136; the net and mesh for construction described in JP-A-8-269850; the filter substrate described in JP-A-8-284063; the stainproof filming agent described in JP-A-9-57889; the mesh fabric and land net described in JP-A-9-137335; the underwater net described in JP-A-10-165045; the ultrafine fibers described in JP-A-11-247027 and 11-247028; the textile fiber described in JP-A-7-310283 and JP-T-2003-528974; the air-bag base cloth described in JP-A-2001-30861; the ultraviolet-absorbing fiber products described in JP-A-7-324283, JP-A-8-20579, and JP-A-2003-147617; and the like.

Hereinafter, the construction material containing the ultraviolet absorbent composition of the present invention is described. The construction material containing the ultraviolet absorbent composition of the present invention may be a construction material of any kind of polymer, as long as it contains the above-described ultraviolet absorbents (A) and (B). Examples thereof include the vinyl chloride series described in JP-A-10-6451; the olefin series described in JP-A-10-16152; the polyester series described in JP-A-2002-161158; the polyphenylene ether series described in JP-A-2003-49065; the polycarbonate sesies described in JP-A-2003-160724; and the like.

The construction material containing the ultraviolet absorbent composition of the present invention may be prepared by any method. Examples of the method include the method, as described in JP-A-8-269850, of forming a material containing the above-described ultraviolet absorbents (A) and (B) into a desired shape; the methods, as described, for example, in JP-A-10-205056, of forming a laminate of a material containing the above-described ultraviolet absorbents (A) and (B); the methods, as described, for example, in JP-A-8-151457, of forming a coated layer using the above-described ultraviolet absorbents (A) and (B); and the methods, as described, for example, in JP-A-2001-172531, of forming it by coating a paint containing the above-described ultraviolet absorbents (A) and (B).

The construction material containing the ultraviolet absorbent composition of the present invention can be used in various applications. Examples thereof include the external construction materials described in JP-A-7-3955, JP-A-8-151457, and JP-A-2006-266042; the wood structure for construction described in JP-A-8-197511; the roofing material for construction described in JP-A-9-183159; the antimicrobial construction material described in JP-A-11-236734; the base construction material described in JP-A-10-205056; the antifouling construction material described in JP-A-11-300880; the flame-resistant material described in JP-A-2001-9811; the ceramic construction material described in JP-A-2001-172531; the decorative construction material described in JP-A-2003-328523; the painted articles for construction described in JP-A-2002-226764; the facing materials described in JP-A-10-6451, JP-A-10-16152, and JP-A-2006-306020; the construction net described in JP-A-8-269850; the moisture-permeable water-repellent sheet for construction described in JP-A-9-277414; the mesh sheet for construction described in JP-A-10-1868; the construction film described in JP-A-7-269016; the decorative film described in JP-A-2003-2111538; the coating materials for construction described in JP-A-9-239921, JP-A-9-254345, and JP-A-10-44352; the adhesive composition for construction described in JP-A-8-73825; the civil work construction structure described in JP-A-8-207218; the pathway coating material described in JP-A-2003-82608; the sheet-shaped photocuring resin described in JP-A-2001-139700; the wood-protecting paint described in JP-A-5-253559; the push-switch cover described in JP-A-2005-2941780; the bond-sheeting agent described in JP-A-9-183159; the base construction material described in JP-A-10-44352; the wall paper described in JP-A-2000-226778; the decorative polyester film described in JP-A-2003-211538; the decorative polyester film for molded material described in JP-A-2003-211606; the flooring material described in JP-A-2004-3191; and the like.

Hereinafter, the recording medium containing the ultraviolet absorbent composition of the present invention is described. The recording medium containing the ultraviolet absorbent composition of the present invention may be any medium, as long as it contains the above-described ultraviolet absorbents (A) and (B). Examples thereof include the

inkjet recording media described in JP-A-9-309260, JP-A-2002-178625, JP-A-2002-212237, JP-A-2003-266926, JP-A-2003-266927, and JP-A-2004-181813; the image-receiving medium for thermal transfer ink described in JP-A-8-108650; the image-receiving sheet for sublimation transfer described in JP-A-10-203033; the image-recording medium described in JP-A-2001-249430; the heat-sensitive recording medium described in JP-A-8-258415; the reversible heat-sensitive recording media described in JP-A-9-95055, JP-A-2003-145949, and JP-A-2006-167996; the information-photorecording medium described in JP-A-2002-367227; and the like.

Hereinafter, the image display device containing the ultraviolet absorbent composition of the present invention is described. The image display device containing the ultraviolet absorbent composition of the present invention may be any device, as long as it contains the above-described ultraviolet absorbents (A) and (B). Examples thereof include the image display device employing an electrochromic element described in JP-A-2006-301268; the image display device of so-called electronic paper described in JP-A-2006-293155; the plasma display described in JP-A-9-306344; the image display device employing an organic EL element described in JP-A-2000-223271; and the like. The ultraviolet absorbent according to the present invention may be contained, for example, in the ultraviolet-absorbing layer formed in the laminated structure described in JP-A-2000-223271 or in a necessary part such as the circularly polarizing plate described, for example, in JP-A-2005-189645.

Hereinafter, the solar cell cover containing the ultraviolet absorbent composition of the present invention is described. The solar cell in the present invention may be made of any kind of element. Examples thereof include a crystalline silicon solar cell, an amorphous silicon solar cell, and a dye-sensitized solar cell. As described in JP-A-2000-174296, a cover material has been used as a protective part for providing a crystalline silicon solar cell or an amorphous silicon solar cell with antifouling property, impact resistance, and durability. As described in JP-A-2006-282970, dye-sensitized solar batteries, which employ a metal oxide-based semiconductor that is activated by excitation of light (in particular, ultraviolet light) as its electrode material, have a problem of the photosensitizer colorant adsorbed being decomposed and thus the photovoltaic efficiency gradually declining, and for that reason, installation of an additional ultraviolet-absorbing layer was proposed.

The solar cell cover containing the ultraviolet absorbent composition of the present invention may be a cover of any kind of polymer, as long as it contains the above-described ultraviolet absorbents (A) and (B). Examples of the polymer include the polyester described in JP-A-2006-310461; the thermosetting transparent resin described in JP-A-2006-257144; the $\alpha$-olefin polymer described in JP-A-2006-210906; the polypropylene described in JP-A-2003-168814; the polyether sulfone described in JP-A-2005-129713; the acrylic resin described in JP-A-2004-227843; the transparent fluorine resin described in JP-A-2004-168057; and the like.

The solar cell cover containing the ultraviolet absorbent composition of the present invention may be prepared by any method. For example, the ultraviolet-absorbing layer described in JP-A-11-40833 may be formed; the layers respectively containing the ultraviolet absorbent may be laminated, as described in JP-A-2005-129926; it may be contained in the filler layer resin, as described in JP-A-2000-91611; or a film may be formed, together with the ultraviolet absorbent-containing polymer described in JP-A-2005-346999.

The solar cell cover containing the ultraviolet absorbent composition of the present invention may be in any form. Examples thereof include the film and sheet described in JP-A-2000-91610 and JP-A-11-261085; the laminate film described, for example, in JP-A-11-40833; the cover glass structure described in JP-A-11-214736; and the like. The ultraviolet absorbent may be contained in the sealer described in JP-A-2001-261904.

Other examples of applications include the illuminating device light source covers described in JP-A-8-102296, JP-A-2000-67629, and JP-A-2005-353554; the synthetic leathers described in JP-A-5-272076 and JP-A-2003-239181; the sport goggle described in JP-A-2006-63162; the deflection lens described in JP-A-2007-93649; the hardcoat for various plastic products described in JP-A-2001-214121, JP-A-2001-214122, JP-A-2001-315263, JP-A-2003-206422, JP-A-2003-25478, JP-A-2004-137457, and JP-A-2005-132999; the hardcoat for bonding on external window described in JP-A-2002-36441; the window film described in JP-A-10-250004; the high-definition antiglare hard-coat film described in JP-A-2002-36452; the antistatic hard-coat film described in JP-A-2003-39607; the permeable hard-coat film described in JP-A-2004-114355; the antiforgery ledger sheet described in JP-A-2002-113937; the turf purpura-preventing agent described in JP-A-2002-293706; the sealant for bonding resin film sheet described in JP-A-2006-274179; the optical guiding parts described in JP-A-2005-326761; the rubber-coating agent described in JP-A-2006-335855; the agricultural covering materials described in JP-A-10-34841 and JP-A-2002-114879; the color candles described in JP-T-2004-532306 and JP-T-2004-530024; the cloth-rinsing agent composition described in JP-T-2004-525273; the laminated glass described in JP-A-10-194796; the prism sheet described in JP-A-10-287804; the protective layer transfer sheet described in JP-A-2000-71626; the photocuring resin product described in JP-A-2001-139700; the flooring sheet described in JP-A-2001-159228; a drain anti-adhesion and heat ray-blocking glass plate described in JP-A-2002-127310; the light-blocking printing label described in JP-A-2002-189415; the fuel cup described in JP-A-2002-130591; the articles with hard-coat film described in JP-A-2002-307619; the intermediate transfer recording medium described in JP-A-2002-307845; the synthetic hair described in JP-A-2006-316395; the low-temperature heat-shrinkable films for label described in WO 99/29490 pamphlet and JP-A-2004-352847; the fishing goods described in JP-A-2000-224942; the

micro beads described in JP-A-8-208976; the precoated metal plate described in JP-A-8-318592; the thin film described in JP-A-2005-504735; the heat-shrinkable film described in JP-A-2005-105032; the in-mold molding label described in JP-A-2005-37642; the projection screen described in JP-A-2005-55615; the decorative sheets described in JP-A-9-300537, JP-A-2000-25180, JP-A-2003-19776, and JP-A-2005-74735; the hot-melt adhesive described in JP-A-2001-207144; the adhesives described in JP-T-2002-543265, JP-T-2002-543266 and U.S. Patent No. 6,225,384; the electrodeposited coat and the basecoat described in JP-A-2004-352783; the wood surface-protecting agent described in JP-A-7-268253; the light-controlling materials, light-controlling films, and light-controlling glasses described in JP-A-2003-253265, JP-A-2005-105131, JP-A-2005-300962, and Japanese Patent No. 3915339; the moth-repellent lamp described in JP-A-2005-304340; the touch panel described in JP-A-2405-44154; the sealant for bonding resin film sheet described in JP-A-2006-274197; the polycarbonate film coating material described in JP-A-2006-89697; the optical fiber tape described in JP-A-2000-231044; the solid wax described in JP-T-2002-527559; and the like.

Hereinafter, the method of evaluating the light stability of the polymer material is described. Methods of evaluating the light stability of the polymer material can be referred to the description, for example, in "Methods for Improving the Photostability of Polymers" (CMC Publishing, 2000) p. 85 to 107; "Basis and Physical Properties of High Functional Coatings" (CMC Publishing, 2003), p. 314 to 359; "Durability of Polymer Materials and Composite Material Products" (CMC Publishing, 2005); "Elongation of Lifetime of Polymer Materials and Environmental Measures" (CMC Publishing, 2000); H. Zweifel Ed., "Plastics Additives Handbook, 5th Edition" (Hanser Publishers), p. 238 to 244; and Tadahiko Kutsura, "Basic Seminar 2. Science of Plastic Packaging Container" (Society of packaging Science & Technology, Japan, 2003), Chapter 8.

In addition, the light stability in each application can be evaluated by the following known evaluation methods.

The photodegradation of polymer materials can be determined by the method described in JIS-K7105:1981, JIS-K7101:1981, JIS-K7102:1981, JIS-K7219:1998, JIS-K7350-1:1995, JIS-K7350-2:1995, JIS-K7350-3:1996, JIS-K7350-4:1996 or a method referring to those.

The light stability in the packaging or container application can be determined by the method of JIS-K7105 and a method referring to that. Typical examples thereof include the light transmittance and transparency evaluation of the bottle body and the functional test of the bottle content after ultraviolet irradiation by using a xenon light source described in JP-A-2006-298456; the haze value evaluation after xenon lamp irradiation described in JP-A-2000-238857; the haze value evaluation by using a halogen lamp as the light source described in JP-A-2006-224317; the yellowing evaluation after mercury lamp irradiation by using a blue wool scale described in JP-A-2006-240734; the haze value evaluation by using Sunshine Weather Meter and the visual observation of color development described in JP-A-2005-105004 and JP-A-2006-1568; the ultraviolet light transmittance evaluation described in JP-A-7-40954, JP-A-8-151455, JP-A-10-168292, JP-A-2001-323082, and JP-A-2005-146278; the ultraviolet-blocking evaluation described in JP-A-9-48935 and 9-142539; the light transmittance evaluation described in JP-A-9-241407, JP-A-2004-243674, JP-A-2005-320408, JP-A-2005-305745, and JP-A-2005-156220; the evaluation of the viscosity of the ink in ink container described in JP-A-2005-178832; the light transmittance evaluation, the visual observation of the container sample and the color difference ΔE evaluation after sunlight irradiation described in JP-A-2005-278678; the ultraviolet light transmittance evaluation, the light transmittance evaluation, and the color difference evaluation after white fluorescent lamp irradiation described in JP-A-2004-51174; the light transmittance evaluation, the haze value evaluation, and the color tone evaluation described in JP-A-2004-285189; the yellowness index evaluation described in JP-A-2003-237825; the light-blocking evaluation and the brightness evaluation by using the color difference Formula of the L*a*b* color system described in JP-A-2003-20966; the yellowing evaluation by using the color difference ΔEa*b* of a sample after irradiation of xenon lights of different in wavelength described in JP-A-2002-68322; the ultraviolet absorbance evaluation after ultraviolet light irradiation described in JP-A-2001-26081; the film tensile elongation test after photoirradiation by using Sunshine Weather Meter described in JP-A-10-298397; the antimicrobial evaluation after photoirradiation in a xenon weather meter described in JP-A-10-237312; the evaluation of discoloration of a package content after fluorescent lamp irradiation described in JP-A-9-239910; the evaluation of oil peroxide value and color tone after fluorescent lamp irradiation of a salad oil-filled bottle described in JP-A-9-86570; the evaluation of the difference in absorbance after chemical lamp irradiation described in JP-A-8-301363; the evaluation of surface glossiness retention rate and appearance after photoirradiation by using Sunshine Weather Meter described in JP-A-8-208765; the evaluation of color difference and bending strength after photoirradiation by using Sunshine Weather-O-meter described in JP-A-7-216152; the light-blocking rate evaluation and the evaluation of the peroxide generated in kerosene described in JP-A-5-139434; and the like.

The long-term durability thereof when the polymer material is used in the coating and coated film applications can be evaluated according to the method of JIS-K5400, JIS-K5600-7-5:1999, JIS-K5600-7-6:2002, JIS-KS600-7-7:1999, JIS-K5600-7-8:1999, or JIS-K874 or a method referring to those. Soecific examples thereof include the evaluation of the color density, the color difference ΔEa*b* in the CIE L*a*b* color coordinates, and the residual brilliance after photoirradiation in an xenon light-endurance test machine and an UVCON apparatus described in JP-T-2000-509082; the absorbance evaluation after photoirradiation on a film placed on a quartz slide in an xenon arc light-endurance test machine and the evaluation of the color density and the color difference ΔEa*b* in the CIE L*a*b* color coordinates after

fluorescent or UV lamp irradiation on wax described in JP-T-2004-520284; the color tone evaluation after photoirradiation in a Metalweather weather-resistance test machine described in JP-A-2006-160847; the evaluation of brilliance retention rate and the evaluation by using color difference ΔEa*b* after photoirradiation test by using a metal HID lamp, and the evaluation of glossiness after photoirradiation by a sunshine carbon arc light source described in JP-A-2005-307161; the evaluation by using color difference ΔEa*b*, the brilliance retention rate evaluation and the appearance evaluation after photoirradiation in a Metalweather weather-resistance test machine described in JP-A-2002-59331; the brilliance retention rate evaluation after photoirradiation by using Sunshine Weather-O-Meter described in JP-A-2002-38084; the evaluation by using the color difference ΔEa*b* and the brilliance retention rate evaluation after photoirradiation in a QUV weather-resistance test machine described in JP-A-2001-59068; the brilliance retention rate evaluation after photoirradiation by using Sunshine Weather-O-Meter described in JP-A-2001-115080, JP-A-6-49368, and JP-A-2001-262056; the evaluation of post-irradiation appearance after pbotoirradiation on a coated plate by using Sunshine Weather-O-Meter described in JP-A-8-324576, JP-A-9-12924, JP-A-9-169950, JP-A-9-241534, and JP-A-2001-181558; the evaluation of the brilliance retention rate and the change in brightness after photoirradiation by using Sunshine Weather-O-Meter described in JP-A-2000-186234; the evaluation of the appearance of the deteriorated coated film after dew cycle WOM photoirradiation on coated film described in JP-A-10-298493; the evaluation of the ultraviolet light transmittance of coated film described in JP-A-7-26177; the evaluation of the ultraviolet-blocking rate of coated film described in JP-A-7-3189 and JP-A-9-263729; the comparative evaluation of the period until the brilliance retention rate of the coated film declines to 80% by using Sunshine Weather-O-Meter as described in JP-A-6-1945; the evaluation of rusting after photoirradiation by using a Dewpanel Light Control Weather Meter described in JP-A-6-313148; the evaluation of the strength of a concrete to the coated formwork after external exposure described in JP-A-6-346022; the evaluation by using the color difference ΔEa*b*, the lattice adhesion test and the surface appearance evaluation after external photoirradiation described in JP-A-5-185031; the brilliance retention rate evaluation after external photoirradiation described in JP-A-5-78606; the evaluation of post-irradiation yellowing (ΔYI) by using a carbon arc light source described in JP-A-2006-63162; and the like.

The light stability when the polymer material is used in the ink application is determined by the method of JIS-K5701-1:2000, JIS-K7360-2, or ISO105-B02 or a method referring to those. Specific examples thereof include the evaluation of the color density and the measurement by the CIE L*a*b* color coordinates after photoirradiation by using an office fluorescent lamp or a discoloration tester described in JP-T-2006-514130; the electrophoretic evaluation after ultraviolet light irradiation by using an xenon arc light source described in JP-A-2006-22300; the print concentration evaluation with a xenon fade meter described in JP-A-2006-8811; the ink blurring evaluation by using a 100W chemical lamp described in JP-A-2005-23111; the evaluation of the dye residual ratio in the image-forming range by using a weather meter described in JP-A-2005-325150; the evaluation of print chalking and discoloration by using an Eye Super UV Tester described in JP-A-2002-127596; the evaluation of print by using the color difference ΔEa*b* in the CIE L*a*b* color coordinates after photoirradiation by a xenon fade meter described in JP-A-11-199808 and JP-A-8-108650; the reflectance evaluation after photoirradiation by using a carbon arc light source described in JP-A-7-164729; and the like.

The light stability of the solar cell module can be determined according to the method of JIS-C8917:1998 or JIS-C8938:1995 or a method referring to those.

Specific examples thereof include the I-V-measuring photovoltaic efficiency evaluation after photoirradiation by a xenon lamp light source having a sunlight-simulating compensation filter described in JP-A-2006-282970; and the evaluation of discoloration gray scale degree, color, and apparent adhesiveness after photoirradiation by using Sunshine Weather Meter or a fade mater described in JP-A-11-261085 and JP-A-2000-144583.

The light stability of fibers and fiber products can be evaluated according to the method of JIS-L1096:1999, JIS-A5905:2003, JIS-L0842, JIS-K6730, JIS-K7107, DIN75.202, SAEJ1885, SN-ISO-105-B02, or AS/NZS4399 or a method referring to those. Examples thereof include the ultraviolet light transmittance evaluation described in JP-A-10-1587, JP-A-2006-299428, and JP-A-2006-299438; the blue scale discoloration evaluation after phatoirradiation by using a xenon light source or a carbon arc light source described in JP-A-6-228816, JP-A-7-76580, JP-A-8-188921, JP-A-11-247028, JP-A-11-247027, JP-A-2000-144583, JP-A-2002-322360, JP-A-2003-339503, and JP-A-2004-11062; the UV-blocking rate evaluation described in JP-A-2003-147617; the ultraviolet-blocking property evaluation described in JP-A-2003-41434; the blue scale discoloration evaluation after dry cleaning and after irradiation by using a carbon are light source described in JP-A-11-302982; the evaluation of lightness index and color difference ΔE* according to chromaticness index after irradiation by using a Fade-O-meter described in JP-A-7-119036 and JP-A-10-251981; the tensile strength evaluation after photoirradiation by using a UV tester or Sunshine Weather Meter described in JP-A-9-57889, JP-A-9-137335, JP-A-10-1868, and JP-A-10-237760; the total transmission and strength retention evaluation described in JP-A-8-41785 and JP-A-8-193136; the ultraviolet protection factor (UPF) evaluation described in JP-T-2003-528974, JP-T-2005-517822, and JP-A-8-20579; the discoloration gray scale evaluation after irradiation by using a high-temperature fade meter described in JP-A- 6-228818, JP-A-7-324283, JP-A-7-196631, and JP-A-7-18584; the appearance evaluation after external photoirradiation described in JP-A-7-289097; the evaluation of yellowness index (YI) and yellowing degree (ΔYI) after ultraviolet irradiation described in JP-A-7-289665; the reflectance evaluation described in JP-T-2003-528974; and the like.

The light stability of the construction material can be evaluated according to the method of JIS-A1415:1999 or a method referring to that. Specific examples thereof include the surface color tone evaluation after photoirradiation by using Sunshine Weather-O-Meter described in JP-A-2006-266402; the appearance evaluation after irradiation by using a carbon arc light source, the post-irradiation appearance evaluation by using an Eye Super UV Tester, the post-irradiation absorbance evaluation, the post-irradiation chromaticity, the color difference evaluation, and the evaluation by using the color difference ΔEa*b* of CIE L*a*b* color coordinates after photoirradiation by using a metal HID lamp light source, and brilliance retention rate evaluation described in JP-A-2004-3191 and JP-A-2006-306020; the evaluation of the change in haze value after photoirradiation by using Sunshine Weather Meter and the elongation retention rate after photoirradiation by using a tensile test machine described in JP-A-10-44352, JP-A-2003-211538, JP-A-9-239921, JP-A-9-254345, and JP-A-2003-211606; the evaluation of ultraviolet transmittance after solvent dip-coating and the visual evaluation of post-irradiation appearance by using an Eye Super UV Tester described in JP-A-2002-161158; the evaluation of brilliance change after a QUV test described in JP-A-2002-226764; the brilliance retention rate evaluation after irradiation by using Sunshine Weather-O-Meter described in JP-A-2001-172531; the evaluation by using the color difference ΔEa*b* after ultraviolet irradiation by using a black light blue fluorescent lamp described in JP-A-11-300880; the evaluation ofpost-irradiation adhesion retention rate and ultraviolet-blocking property by using a UVCON acceleration test machine described in JP-A- 10-205056; the appearance evaluation, the total light transmittance evaluation, the haze change evaluation, and tensile shear adhesive strength evaluation after external exposure (JIS-A1410) described in JP-A-8-207218 and JP-A-9-183159; the evaluation of total light transmittance of the light in the entire wavelength range, the haze evaluation, and the yellowing degree evaluation after irradiation by using a xenon weather meter described in JP-A-8-151457; the evaluation of yellowing degree (ΔYI) and ultraviolet absorbent residual ratio after irradiation by using Sunshine Weather-O-Meter described in JP-A-7-3955; and the like.

The light stability when the polymer material is used in the recording medium application can be evaluated according to the method of JIS-K7350 or a method referring to that. Specific examples thereof include the evaluation of the difference in base color in the printing unit after fluorescent lamp irradiation described in JP-A-2006-167996; the evaluation of image density residual rate after irradiation by using a xenon weather meter described in JP-A-10-203033 and JP-A-2004-181813; the evaluation of the change in optical reflection density after irradiation by using a xenon weather meter described in JP-A-2002-207845; the yellowing degree evaluation based on the L*a*b* evaluation system after irradiation by using a Suntest CPS photodiscoloration tester described in JP-A-2003-266926; the post-irradiation discoloration evaluation by using a fade meter described in JP-A-2003-145949; the visual evaluation of post-irradiation discoloration by using a xenon fade meter described in JP-A-2002-212237; the color density retention rate evaluation after indoor sunlight irradiation and the post-irradiation color density retention rate evaluation by using a xenon weather meter described in JP-A-2002-178625; the evaluation of post-exposure C/N by using a fade meter described in JP-A-2002-367227; the fog density evaluation after fluorescent lamp irradiation described in JP-A-2001-249430; the optical reflection density evaluation and the erasability evaluation after irradiation by using a fluorescent lamp described in JP-A-9-95055; the evaluation of post-irradiation color difference ΔE* by using an Atlas fade meter described in JP-A-9-309260; the visual evaluation of post-irradiation discoloration by using a carbon arc fade meter described in JP-A-8-258415; the evaluation of the retention rate of organic EL element color-changing property described in JP-A-2000-223271; the measurement and evaluation of organic EL display brightness after photoirradiation by a xenon discoloration tester described in JP-A-2005-189645; and the like.

Other evaluation methods include those of JIS-K7103 and ISO/DIS9050 or a method referring to those. Specific examples thereof include the appearance evaluation of a polycarbonate coating film after irradiation by a UV tester described in JP-A-2006-89697; the blue scale evaluation of a synthetic hair after irradiation with ultraviolet light described in JP-A-2006-316395; the evaluation of water contact angle on a processing cloth for evaluation after irradiation by using an accelerated weather-resistance test machine described in JP-A-2006-335855; the visual evaluation of an image projected on a projection screen after irradiation by using a weather-resistance test machine described in JP-A-2005-55615; the evaluation of the deterioration of sample surface and visual evaluation of appearance after irradiation by using a Sunshine Weather Meter or a metal weather meter described in JP-A-2005-74735; the visual evaluation of appearance after photoirradiation by using a metal lamp reflector described in JP-A-2005-326761; the evaluation of the light transmittance of bottle label described in JP-A-2002-189415 and JP-A-2004-352847; the evaluation of polypropylene deterioration after irradiation by using a xenon weather meter under humid condition described in JP-A-2003-19776; the evaluation of the deterioration of a hard-coat film, the deterioration evaluation, the hydrophilicity evaluation and the abrasion resistance evaluation of the base material by using Sunshine Weather-O-Meter described in JP-A-2002-36441 and JP-A-2003-25478; the evaluation of the gray scale color difference of synthetic leather after irradiation by using a xenon lamp light described in JP-A-2003-239181; the evaluation of liquid crystal device characteristics after irradiation by using a mercury lamp described in JP-A-2003-253265; the post-irradiation adhesiveness evaluation by using Sunshine Weather-O-Meter described in JP-A-2002-307619; the evaluation of the degree of turf purpura described in JP-A-2002-293706; the evaluation of ultraviolet light transmittance and tensile strength after irradiation by using a xenon arc

light source described in JP-A-2002-114879, the concrete adhesion velocity evaluation described in JP-A-2001-139700; the appearance evaluation and the coated-film adhesiveness evaluation after irradiation by using Sunshine Weather-O-Meter described in JP-A-2001-315263; the evaluation of post-irradiation yellowing degree and adhesiveness by using a carbon arc light source described in JP-A-2001-214121 and JP-A-2001-214122; the adhesiveness evaluation by using an ultraviolet fade meter described in JP-A-2001-207144; the evaluation of insect-repellency when illumination is turned on described in JP-A-2000-67629; the evaluation of the laminated glass yellowing degree (ΔYI) by using Eye Super UV Tester described in JP-A-10-194796; the evaluation of the surface appearance and brilliance detention rate after QUV irradiation and humidity-resistance tests described in JP-A-8-318592; the evaluation of color difference over time by using a dew panel light control weather meter described in JP-A-8-208976; the evaluation of the glossiness (DI) and the yellowness index (YI) in the wood base-coated state after irradiation by using a xenon Weather-O-meter described in JP-A-7-268253; the ultraviolet absorbance evaluation after repeated processing of UV irradiation and storage in dark described in JP-T-2002-5443265 and JP-T-2002-543266; the evaluation of dye discoloration color difference ΔE after ultraviolet irradiation described in JP-T-2004-532306; and the like.

EXAMPLES

The present invention is described in more detail based on the following examples, but the invention is not intended to be limited thereby.

Example 1

(Preparation of ultraviolet absorbent composition sample)

Ultraviolet absorbent composition samples 1 to 27 containing ultraviolet absorbents (A) and (B) were prepared, as shown in the following Tables 7 and 8. In the following Tables 7 and 8, the ratio of the ultraviolet absorbents (A) to (B) (A:B) is expressed by molar ratio.

[Table 7]

| Sample | Ultraviolet absorbent | | A:B | General formula of ultraviolet absorbent (B) | Classification of ultraviolet absorbent (B) | Remarks |
|---|---|---|---|---|---|---|
| | (A) | (B) | | | | |
| 1 | Exemplary compound (1) | II-2 | 1:2 | (IIa), (IIb) | B-(1) | Present invention |
| 2 | Exemplary compound (1) | II-3 | 1:2 | (IIa), (IIb) | B-(1) | Present invention |
| 3 | Exemplary compound (1) | II-10 | 1:2 | (IIa), (IIb) | B-(1) | Present invention |
| 4 | Exemplary compound (1) | II-10 | 1:4 | (IIa), (IIb) | B-(1) | Present invention |
| 5 | Exemplary compound (1) | II-10 | 2:1 | (IIa), (IIb) | B-(1) | Present invention |
| 6 | Exemplary compound (1) | III-1 | 1:2 | (III) | B-(1) | Present invention |
| 7 | Exemplary compound (1) | III-1 | 1:4 | (III) | B-(1) | Present invention |
| 8 | Exemplary compound (1) | III-3 | 1:2 | (III) | B-(2) | Present invention |
| 9 | Exemplary compound (1) | III-4 | 1:2 | (III) | B-(1) | Present invention |
| 10 | Exemplary compound (1) | IV-2 | 1:2 | (IV) | B-(1) | Present invention |

(continued)

| Sample | Ultraviolet absorbent | | A:B | General formula of ultraviolet absorbent (B) | Classification of ultraviolet absorbent (B) | Remarks |
|---|---|---|---|---|---|---|
| | (A) | (B) | | | | |
| 11 | Exemplary compound (1) | IV-3 | 1:2 | (IV) | B-(2) | Present invention |
| 12 | Exemplary compound (1) | V-1 | 1:2 | (V) | B-(1) | Present invention |
| 13 | Exemplary compound (21) | II-2 | 1:2 | (IIa), (IIb) | B-(1) | Present invention |
| 14 | Exemplary compound (21) | II-10 | 1:2 | (IIa), (IIb) | B-(1) | Present invention |
| 15 | Exemplary compound (21) | II-10 | 1:4 | (IIa), (IIb) | B-(1) | Present invention |
| 16 | Exemplary compound (21) | III-1 | 1:2 | (III) | B-(1) | Present invention |
| 17 | Exemplary compound (21) | III-3 | 1:2 | (III) | B-(2) | Present invention |
| 18 | Exemplary compound (21) | III-4 | 1:2 | (III) | B-(1) | Present invention |
| 19 | Exemplary compound (21) | IV-2 | 1:2 | (IV) | B-(1) | Present invention |
| 20 | Exemplary compound (21) | V-1 | 1:2 | (V) | B-(1) | Present invention |

[Table 8] (Continuation of Table 7)

| Sample | Ultraviolet absorbent | | A:B | General formula of ultraviolet absorbent (B) | Classification of ultraviolet absorbent (B) | Remarks |
|---|---|---|---|---|---|---|
| | (A) | (B) | | | | |
| 21 | Exemplary compound (1) | III-1 | 1:12 | (III) | B-(1) | Present invention |
| 22 | Exemplary compound (1) | IV-2 | 12:1 | (IV) | B-(1) | Present invention |
| 23 | Exemplary compound (1) | - | 1:0 | - | - | Comparative example |
| 24 | Exemplary compound (21) | - | 1:0 | - | - | Comparative example |
| 25 | - | V-1 | 0:1 | - | - | Comparative example |
| 26 | Exemplary compound (1) | X-1 | 1:2 | (X) | B-(2) | Comparative example |
| 27 | X-2 | II-10 | 1:2 | (IIa), (IIb) | B-(1) | Comparative example |

The absorption maximum wavelength and the rate of the absorbance at 320 nm relative to that at the absorption maximum wavelength of each compound used in preparation of the samples 1 to 27 were determined by preparing an

ethyl acetate solution of each compound at a concentration of approximately $5 \times 10^{-5}$ mol/L and measuring the absorption spectrum of the solution in a 1-cm quartz cell by using a spectrophotometer (UV-4100 spectrophotometer (trade name) manufactured by Hitachi High-Technologies Corporation. The absorption maximum wavelength and the percentage of the absorbance at 320 nm relative to that at the absorption maximum wavelength were calculated from the spectral chart obtained. Results are shown in the following Tables 9. The following Table 10 shows the absorption maximum wavelength and the rate of the absorbance at 320 nm relative to that at the absorption maximum wavelength of compounds X-1 and X-2, which are used as comparative examples.

[Table 9]

| Classification of ultraviolet absorbent | Compound | Absorption maximum wavelength (nm) | Absorbance at 320 nm relative to absorbance at absorption maximum wavelength (%) |
|---|---|---|---|
| (A) | (1) | 376 | 33 |
| (A) | (21) | 375 | 50 |
| (B) | II-1 | 342 | 82 |
| (B) | II-2 | 350 | 83 |
| (B) | II-3 | 339 | 77 |
| (B) | II-4 | 346 | 83 |
| (B) | II-5 | 343 | 86 |
| (B) | II-6 | 337 | 79 |
| (B) | II-7 | 349 | 87 |
| (B) | II-8 | 341 | 69 |
| (B) | II-9 | 302 | 71 |
| (B) | II-10 | 349 | 84 |
| (B) | III-1 | 346 | 58 |
| (B) | III-2 | 287 | 48 |
| (B) | III-3 | 288 | 50 |
| (B) | III-4 | 274 | 41 |
| (B) | III-5 | 348 | 61 |
| (B) | IV-1 | 349 | 27 |
| (B) | IV-2 | 346 | 50 |
| (B) | IV-3 | 286 | 63 |
| (B) | IV-4 | 290 | 72 |
| (B) | IV-5 | 287 | 64 |
| (B) | IV-6 | 288 | 65 |
| (B) | IV-7 | 282 | 75 |
| (B) | V-1 | 346 | 64 |

[Table 10]

| Classification of ultraviolet absorbent | Compound | Absorption maximum wavelength (nm) | Absorbance at 320 nm relative to absorbance at absorption maximum wavelength (%) |
|---|---|---|---|
| Suitably (A) | X-2 | 371 | 25 |
| Suitably (B) | X-1 | 369 | 5 |

The compound X-1 is a known compound described in, for example, JP-A-51-56620 and the like. The compound X-2 is a known compound described in, for example, JP-A-2002-53824 and the like. Structures of the se compounds are shown below.

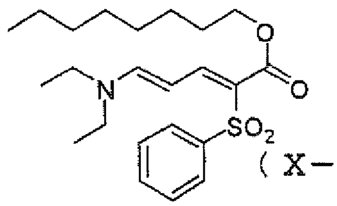

(X-1)

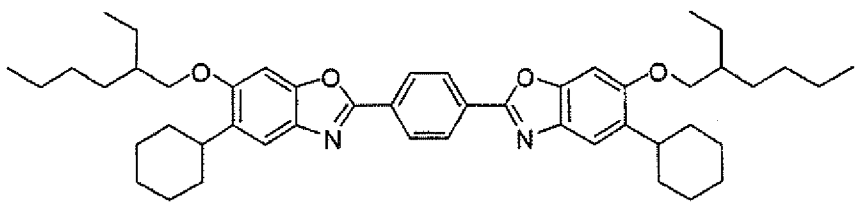

(X-2)

(Light stability test)

The ethyl acetate solutions of samples 1 to 26 (concentration of aproximately $2 \times 10^{-5}$ mol/L) were photoirradiated by a xenon lamp (manufactured by Eagle Engneering) at an illuminance of 170,000 lux for 200 hours, and the residual amount of the ultraviolet absorbents after irradiation was determined. The residual amount was calculated according to the following formula:

$$\text{Residual amount (\%)} = 100 \times (\text{Absorbance at 410nm after iradiation})/(\text{Absorbance at 410nm before iradiation})$$

[Table 11]

| Sample | Residual amount (%) | Sample | Residual amount ratio (%) | Sample | Residual amount ratio (%) |
|---|---|---|---|---|---|
| 1 | 98 | 10 | 99 | 19 | 98 |
| 2 | 99 | 11 | 98 | 20 | 97 |
| 3 | 98 | 12 | 97 | 21 | 98 |
| 4 | 97 | 13 | 99 | 22 | 99 |

(continued)

| Sample | Residual amount (%) | Sample | Residual amount ratio (%) | Sample | Residual amount ratio (%) |
|---|---|---|---|---|---|
| 5 | 99 | 14 | 98 | 23 | 80 |
| 6 | 98 | 15 | 98 | 24 | 82 |
| 7 | 98 | 16 | 97 | 25 | 79 |
| 8 | 97 | 17 | 99 | 26 | 78 |
| 9 | 99 | 18 | 98 | 27 | 79 |

As is apparent from Table 11, it was found that surprisingly, the light stabilities were improved when the ultraviolet absorbent compositions of the present invention (samples 1 to 22) were used, as compared to the cases (samples 23 and 24) in which the ultraviolet absorbent (A) was used alone. Further, it was found that light resistance-enhancing effects of the present invention were also notable, as compared to the case (sample 25) in which the ultraviolet absorbent (B) was used alone; the case (sample 26) in which the ultraviolet absorbent (B) was substituted by another ultraviolet absorbent that is not a preferable embodiment of the present invention; and the case (sample 27) in which the ultraviolet absorbent (A) is substituted by another ultraviolet absorbent that is not a preferable embodiment of the present invention.

Example 2

(Production or acrylic coating film samples)

30m of a solution prepared by dissolving 20g of polymethymethacrylate resin (PMMA resin) in 100m of dichloromethane was measured off. 20mg of the sample 1 was added to the solution. Then, the mixture was filtrated through a 45 μm filter and the filtrate was designated as paint sample 201. Further, paint samples 202,203, 204, 205, 206, 207,208, and 209 were prepared in the same manner as the paint sample 201, except that the sample 1 was substituted by samples 3, 4, 8, 10, 12, 15, 21, and 22, respectively. As comparative examples, comparative paint samples 210 and 211 were prepared in the same manner as the paint sample 201, except that the sample 1 was substituted by samples 23 and 26, respectively.
These samples were each coated on a 100 μm thick polyethyleneterephthalate (PET) film so as to become a thickness of about 30 μm, and then dried, thereby producing PET films (membranes 201 to 211) having an ultraviolet absorbent layer.

(Evaluation of light stability)

Each of these sample films was photoirradiated by a xenon lamp (manufactured by Eagle Engneering) at an illuminance of 170,000 lux for 100 hours, and the residual amount of the ultraviolet absorbent after irradiation was determined. The residual amount was calculated according to the following Formula:

$$\text{Residual amount (\%)} = 100 \times (\text{Absorbance at 410nm after iradiation})/(\text{Absorbance at 410nm before iradiation})$$

The results are shown in Table 12. Evaluation in Table 12 was performed according to the following criterion:

◎. Residual amount is 90% or more.
○: Residual amount is from 80% to less than 90%.
△: Residual amount is less than 80%.

[Table 12]

| Film | Sample | Residual amount (%) | Evaluation | Remarks |
|---|---|---|---|---|
| 201 | 1 | 93 | ◎ | Present invention |
| 202 | 3 | 94 | ◎ | Present invention |
| 203 | 4 | 93 | ◎ | Present invention |
| 204 | 8 | 94 | ◎ | Present invention |
| 205 | 10 | 94 | ◎ | Present invention |
| 206 | 12 | 94 | ◎ | Present invention |
| 207 | 15 | 95 | ◎ | Present invention |
| 208 | 21 | 93 | ◎ | Present invention |
| 209 | 22 | 89 | ○ | Present invention |
| 210 | 23 | 79 | △ | Comparative example |
| 211 | 26 | 79 | △ | Comparative example |

As is apparent from Table 12, it was found that surprisingly, the light stabilities were improved when the ultraviolet absorbent compositions of the present invention (membranes 201 to 209) were used, as compared to the case (membrane 210) in which the ultraviolet absorbent (A) was used alone. Further, it was found that light resistance-enhancing effects of the present invention were also notable, as compared to the case (membrane 211) in which the ultraviolet absorbent (B) was substituted by another ultraviolet absorbent that was not a preferable embodiment of the present invention.

Example 3

(Preparation of films 301 to 311)

One (1) kg of a polyethylene terephthalate resin (PET) and 150g of the composition sample 1 were agitated in a stainless steel tumbler for 1 hour. The resultant mixture was melted and blended by a biaxial extruder at 280˚C, and pellets for molding were prepared by an ordinary method. Film 301 having 100μm thickness was produced from the pellet using injection moulder.

Films 302 to 311 were produced in the same manner as the Film 301, except that the sample 1 was replaced with the samples 5,6,7,12,16,21,23,24,25 and 27, respectively.

(Evaluation of compatibility)

Evaluation of compatibility was conducted by visual observation of the produced film. The results are shown in Table 13.
In Table 13 below, ○ represents a transparent film, while × represents a film on which existence of a crystal is apparent.
[Table 13]

| Film | Sample | Visual observation (compatibility) | Remarks |
|---|---|---|---|
| 301 | 1 | ○ | Present invention |
| 302 | 5 | ○ | Present invention |
| 303 | 6 | ○ | Present invention |
| 304 | 7 | ○ | Present invention |
| 305 | 12 | ○ | Present invention |
| 306 | 16 | ○ | Present invention |
| 307 | 21 | ○ | Present invention |
| 308 | 23 | × | Comparative example |

(continued)

| Film | Sample | Visual observation (compatibility) | Remarks |
|---|---|---|---|
| 309 | 24 | × | Comparative example |
| 310 | 25 | × | Comparative example |
| 311 | 27 | × | Comparative example |

As is apparent from Table 13, it was found that compatibilities were notably increased when the ultraviolet absorbent compositions of the present invention (films 301 to 307) were used, as compared to the cases (films 308 and 309) in which the ultraviolet absorbent (A) was used alone. Further, it was found that compatibility-enhancing effects of the present invention were also notable, as compared to the case (film 310) in which the ultraviolet absorbent (B) was used alone, and the case (film 311) in which the ultraviolet absorbent (A) was substituted by another ultraviolet absorbent that was not a preferable embodiment of the present invention.

(Evaluation of ultraviolet-cutting property and low-staining property)

Measurement of optical transmittance of the produced film was conducted using a spectrophotometer U-4100 (trade name, manufactured by Shimadzu Corporation).
Evaluation of the measured transmittance was performed according to the following criterion:
<Measured wavelength: 320 nm>

○: Transmittance is less than 1%.
×: Transmittance is 1% or more.

<Measured wavelength: 410 nm>

◎: Transmittance is less than 1%.
○: Transmittance is from 1% to less than 3%.
×: Transmittance is 3% or more.

Staining properties were visually determined according to the following criterion:

○: yellow tinge is not conspicuous.
Δ: yellow tinge is apparently conspicuous.

The results are shown in Table 14.
[Table 14]

| Film | Transmittance (%T) | | Staining property |
|---|---|---|---|
| | 320nm | 410nm | |
| 301 | ○ | ◎ | ○ |
| 302 | ○ | ◎ | ○ |
| 303 | ○ | ◎ | ○ |
| 304 | ○ | ○ | ○ |
| 305 | ○ | ◎ | ○ |
| 306 | ○ | ○ | ○ |
| 307 | ○ | ○ | △ |
| 308 | ○ | ◎ | × |
| 309 | ○ | ◎ | × |
| 310 | ○ | × | ○ |

(continued)

| Film | Transmittance (%T) | | Staining property |
|---|---|---|---|
| | 320nm | 410nm | |
| 311 | ○ | ○ | × |

As is apparent from Table 14, it is understood that ultraviolet in a wide wavelength range can be cut when the ultraviolet absorbent compositions of the present invention (films 301 to 307) were used. However, in the cases (films 308 and 309) of using the ultraviolet absorbent (A) alone, the transmittance at the short wavelength side was insufficient. Further, in the case (film 310) of using the ultraviolet absorbent (B) alone, the ultraviolet-cutting capacity at the long wavelength side was insufficient.

(Evaluation of light stability)

First, initial transmittance of each produced films were measured. Then, each of the produced films was photoirradiated by a xenon lamp (manufactured by Eagle Engineering) at an illuminance of 170,000 lux for 50 hours, and the residual amount of the ultraviolet absorbent after irradiation was determined. The residual amount was calculated according to the following Formula:

$$\text{Residual amount (\%)} = 100 \times (100 - \text{Transmittance after irradiation})/(100 - \text{Transmittance before irradiation})$$

In calculation of residual amount, transmittance of each films 301-308, 310 and 311 was measured at 420 nm, while transmittance of film 309 was measured at 380 nm. The results are shown in Table 15.

[Table 15]

| Film | Residual amount (%) | Remarks |
|---|---|---|
| 301 | 93 | Present invention |
| 302 | 94 | Present invention |
| 303 | 93 | Present invention |
| 304 | 94 | Present invention |
| 305 | 94 | Present invention |
| 306 | 95 | Present invention |
| 307 | 90 | Present invention |
| 308 | 79 | Comparative example |
| 309 | 78 | Comparative example |
| 310 | 77 | Comparative example |
| 311 | 78 | Comparative example |

As is apparent from Table 15, it was found that surprisingly, the light stabilities were improved when the films each using the ultraviolet absorbent composition of the present invention were used, as compared to the cases (films 308 and 309) in which the ultraviolet absorbent (A) was used alone. Further, it was found that light resistance-enhancing effects of the present invention were also notable, as compared to the case (film 310) in which the ultraviolet absorbent (B) was used alone and the case (film 311) in which the ultraviolet absorbent (A) was substituted by another ultraviolet absorbent that was not a preferable embodiment of the present invention.

From the results described above, it is understood that the ultraviolet absorbent compositions of the present invention have not only an ultraviolet absorptive capacity in a wide wavelength range, but also low staining properties, high resistance to light, and excellent compatibility with a resin in combination.

Having described our invention as related to the present embodiments, it is our intention that the invention not be limited by any of the details of the description, unless otherwise specified, but rather be construed broadly within its spirit and scope as set out in the accompanying claims.

This non-provisional application claims priority under 35 U.S.C. § 119 (a) on Patent Application No. 2008-091837 filed in Japan on March 31, 2008, which is entirely herein incorporated by reference.

## Claims

1. An ultraviolet absorbent composition, comprising:

   at least one kind of ultraviolet absorbent (A) that is a compound represented by the following Formula (1); and at least one kind of ultraviolet absorbent (B) that is a compound where absorbance at 320 nm is 20% or more of absorbance at absorption maximum wavelength in the range of from 270 nm to 400 nm and the absorption maximum wavelength is 380 nm or less:

Formula (1)

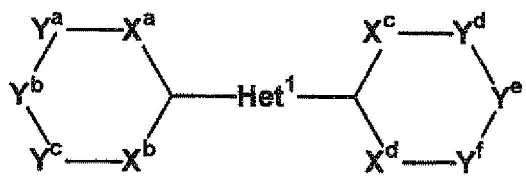

   wherein $Het^1$ represents a bivalent five- or six-membered aromatic heterocyclic residue; the aromatic heterocyclic residue may have a substituent;
   $X^a$, $X^b$, $X^c$ and $X^d$ each independently represent a heteroatom; $X^a$ to $X^d$ may have a substituent;
   $Y^a$, $Y^b$, $Y^c$, $Y^d$, $Y^e$ and $Y^f$ each independently represent a heteroatom or a carbon atom; $Y^a$ to $Y^f$ may have a substituent; and
   the rings bound to $Het^1$ may have a double bond at any position.

2. The ultraviolet absorbent composition according to Claim 1, wherein, in the above Formula (1), at least one of the ring formed by $X^a$, $X^b$, $Y^a$ to $Y^c$ and the carbon atom and the ring formed by $X^c$, $X^d$, $Y^d$ to $Y^f$ and the carbon atom is a fused ring.

3. The ultraviolet absorbent composition according to Claim 1 or 2, wherein, in the above Formula (1), at least one of the ring formed by $X^a$, $X^b$, $Y^a$ to $Y^c$ and the carbon atom and the ring formed by $X^c$, $X^d$, $Y^d$ to $Y^f$ and the carbon atom is not a perimidine ring.

4. The ultraviolet absorbent composition according to any one of Claims 1 to 3, wherein the compound represented by the above Formula (2) is a compound represented by the following Formula (2):

Formula (2)

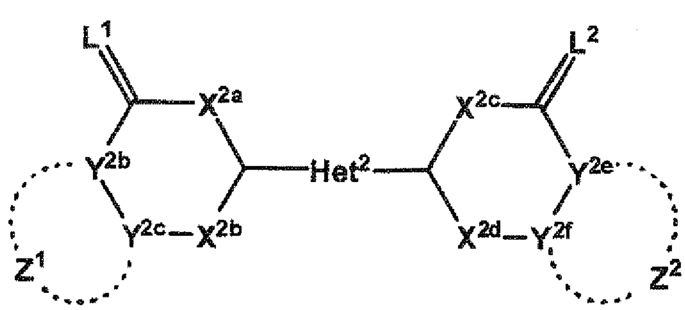

wherein $Het^2$ is the same as $Het^1$ in the above Formula (1);
$X^{2a}$, $X^{2b}$, $X^{2c}$ and $X^{2d}$ are the same as $X^a$, $X^b$, $X^c$ and $X^d$ in the above Formula (1), respectively;
$Y^{2b}$, $Y^{2c}$, $Y^{2e}$ and $Y^{2f}$ are the same as $Y^b$, $Y^c$, $Y^e$ and $Y^f$ in the above Formula (1), respectively;
$L^1$ and $L^2$ each independently represent an oxygen atom, a sulfur atom or $=NR^a$ ($R^a$ represents a hydrogen atom or a monovalent substituent); and
$Z^1$ and $Z^2$ each independently represent a group of atoms needed to form a four- to eight-membered ring together with $Y^{2b}$ and $Y^{2c}$ or $Y^{2e}$ and $Y^{2f}$.

**5.** The ultraviolet absorbent composition according to Claim 4, wherein the compound represented by the above Formula (2) is a compound represented by the following Formula (3):

Formula (3)

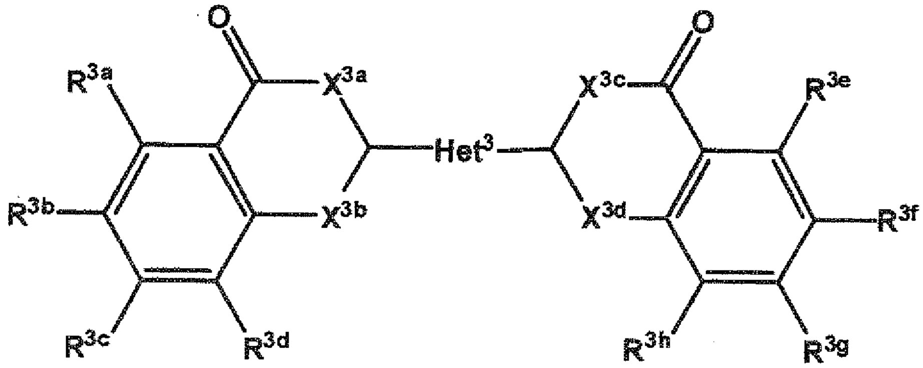

wherein $Het^3$ is the same as $Het^2$ in the above Formula (2);
$X^{3a}$, $X^{3b}$, $X^{3c}$ and $X^{3d}$ are the same as $X^{2a}$, $X^{2b}$, $X^{2c}$ and $X^{2d}$ in the above Formula (2), respectively; and
$R^{3a}$, $R^{3b}$, $R^{3c}$, $R^{3d}$, $R^{3e}$, $R^{3f}$, $R^{3g}$ and $R^{3h}$ each independently represent a hydrogen atom or a monovalent substituent.

**6.** The ultraviolet absorbent composition according to Claim 5, wherein the compound represented by the above Formula (3) is a compound represented by the following Formula (4):

Formula (4)

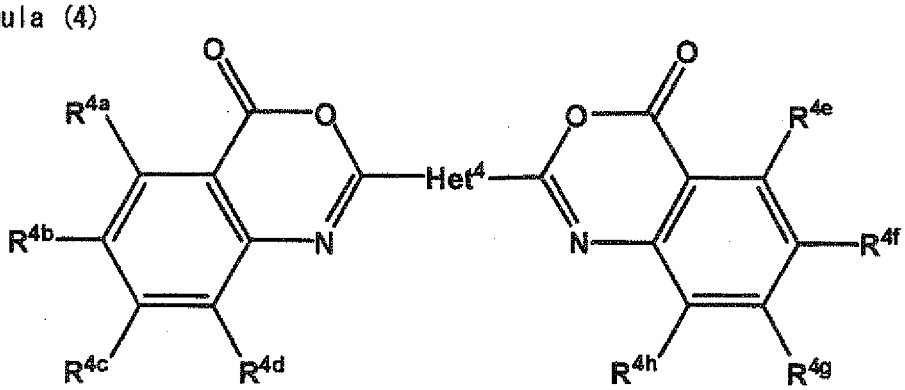

wherein $Het^4$ is the same as $Het^3$ in the above Formula (3); and
$R^{4a}$, $R^{4b}$, $R^{4c}$, $R^{4d}$, $R^{4e}$, $R^{4f}$, $R^{4g}$ and $R^{4h}$ are the same as $R^{3a}$, $R^{3b}$, $R^{3c}$, $R^{3d}$, $R^{3e}$, $R^{3f}$, $R^{3g}$ and $R^{3h}$ in the above Formula (3), respectively.

**7.** The ultraviolet absorbent composition according to Claim 6, wherein the compound represented by the above Formula (4) is a compound represented by the following Formula (5):

Formula (5)

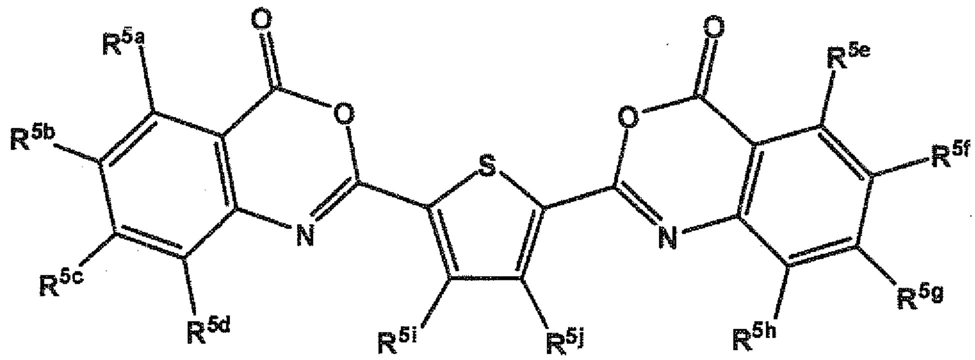

wherein $R^{5a}$, $R^{5b}$,$R^{5c}$, $R^{5d}$, $R^{5e}$, $R^{5f}$, $R^{5g}$ and $R^{5h}$ are the same as $R^{4a}$, $R^{4b}$, $R^{4c}$, $R^{4d}$, $R^{4e}$, $R^{4f}$, $R^{4g}$ and $R^{4h}$ in the above Formula (4), respectively; and
$R^{5i}$ and $R^{5j}$ each independently represent a hydrogen atom or a monovalent substituent.

**8.** The ultraviolet absorbent composition according to any one of Claims 1 to 7, wherein a ratio of the ultraviolet absorbent (A) and the ultraviolet absorbent (B) is in the range of from 10:1 to 1:10.

**9.** An ultraviolet absorbent dispersion, comprising the ultraviolet absorbent composition according to any one of Claims 1 to 8.

**10.** An ultraviolet absorbent solution, comprising the ultraviolet absorbent composition according to any one of Claims 1 to 8.

**11.** A polymer material, comprising the ultraviolet absorbent composition according to any one of Claims 1 to 8.

[Fig.1]

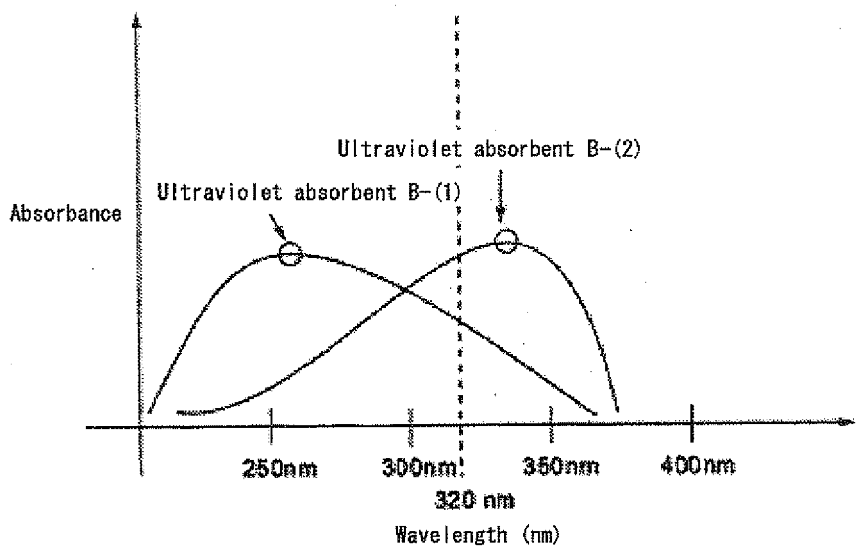
Ultraviolet absorbent B-(2)
Ultraviolet absorbent B-(1)
Absorbance
250nm
300nm
350nm
400nm
320 nm
Wavelength (nm)

**INTERNATIONAL SEARCH REPORT**

International application No.
PCT/JP2009/056542

A. CLASSIFICATION OF SUBJECT MATTER
*C09K3/00*(2006.01)i, *C07D409/14*(2006.01)i, *C07D413/14*(2006.01)i, *C08K5/45*(2006.01)i, *C08L101/00*(2006.01)i

According to International Patent Classification (IPC) or to both national classification and IPC

B. FIELDS SEARCHED

Minimum documentation searched (classification system followed by classification symbols)
C09K3/00, C07D409/14, C07D413/14, C08K5/45, C08L101/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2003-526650 A (Merck Patent GmbH),<br>09 September, 2003 (09.09.03),<br>Claims; Par. Nos. [0084] to [0103]; table 3<br>& US 2003/0207886 A1 & EP 1267819 A2<br>& WO 2001/068047 A2 | 1-3,9-11<br>4-8 |
| A | JP 2003-177235 A (Eastman Kodak Co.),<br>27 June, 2003 (27.06.03),<br>The entire specification<br>& US 2003/0080326 A1 & EP 1300700 A1 | 1-11 |
| A | JP 11-218602 A (Asahi Optical Co., Ltd.),<br>10 August, 1999 (10.08.99),<br>The entire specification<br>(Family: none) | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 June, 2009 (15.06.09) | 30 June, 2009 (30.06.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/056542

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-501339 A (Ciba Specialty Chemicals Holding Inc.), 12 January, 2006 (12.01.06), The entire specification & US 2006/0052491 A1 & EP 1308084 A1 & EP 1549713 A1 & WO 2004/031294 A1 | 1-11 |
| E,A | JP 2009-96974 A (Fujifilm Corp.), 07 May, 2009 (07.05.09), The entire specification (Family: none) | 1-11 |
| P,A | JP 2008-273927 A (Fujifilm Corp.), 13 November, 2008 (13.11.08), The entire specification (Family: none) | 1-11 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5339033 A
- JP 5345639 A
- JP 6056466 A
- JP 2006316107 A
- JP 6145387 A
- JP 2003177235 A
- JP 2005517787 T
- JP 7285927 A
- JP 2000 A
- JP 264879 A
- JP 2003155375 A
- JP 1053455 B
- JP 61189530 A
- EP 27242 A
- JP 2004117997 A
- JP 55129136 A
- JP 58209735 A
- JP 63264748 A
- JP 4191851 A
- JP 8272058 A
- GB 2016017 A
- JP 2002053824 A
- US 5807932 A
- US 4259467 A
- US 4355147 A
- JP 8208765 A
- JP 8151455 A
- JP 8245849 A
- JP 10168292 A
- JP 2004285189 A
- JP 2001323082 A
- JP 10298397 A
- JP 11315175 A
- JP 2001026081 A
- JP 2005305745 A
- JP 2003524019 T
- JP 2004050460 A
- JP 2004243674 A
- JP 2006240734 A
- JP 11034261 A
- JP 2003237825 A
- JP 8080928 A
- JP 2004051174 A
- JP 8301363 A
- JP 11276550 A
- JP 2006271781 A
- JP 7287353 A
- JP 2000056433 A
- JP 2003 A
- JP 200966 A
- JP 2006323339 A
- JP 2006224317 A
- JP 2002160321 A
- JP 2005156220 A
- JP 2005146278 A
- JP 2006130807 A
- JP 9142539 A
- JP 9157626 A
- JP 8324572 A
- JP 2001048153 A
- JP 2005105004 A
- JP 2006001568 A
- JP 2000238857 A
- JP 2001088815 A
- JP 7216152 A
- JP 2001 A
- JP 270531 A
- JP 2004083858 A
- JP 2001114262 A
- JP 2041213427 A
- JP 7242444 A
- JP 8133787 A
- JP 2005320408 A
- JP 5139434 A
- JP 7289665 A
- JP 9295664 A
- JP 9058687 A
- JP 8155007 A
- JP 2006298456 A
- JP 9086570 A
- JP 9113494 A
- JP 9239910 A
- JP 11180474 A
- JP 2002068322 A
- JP 2005278678 A
- JP 11290420 A
- JP 2001106218 A
- JP 2005178832 A
- WO 0493775 A
- JP 5305975 A
- JP 7040954 A
- JP 10237312 A
- JP 2000152974 A
- JP 2002264979 A
- JP 2005255736 A
- JP 2002370723 A
- JP 9241407 A
- JP 8091385 A

- JP 9048935 A
- JP 11514387 T
- JP 2000066603 A
- JP 2041323082 A
- JP 2005 A
- JP 105032 A
- WO 9929490 A
- JP 11255925 A
- JP 7026177 A
- JP 9169950 A
- JP 9221631 A
- JP 2002080788 A
- JP 10088039 A
- JP 2001055541 A
- JP 8081643 A
- JP 2000186234 A
- JP 7166112 A
- JP 2002 A
- JP 294165 A
- JP 2000273362 A
- JP 2001279189 A
- JP 2002271227 A
- JP 2001009357 A
- JP 2001316630 A
- JP 2002356655 A
- JP 2004000937 A
- JP 2004002700 A
- JP 2004169182 A
- JP 2004107700 A
- JP 6049368 A
- JP 2002038084 A
- JP 2005307161 A
- JP 5078606 A
- JP 5185031 A
- JP 10140089 A
- JP 2000509082 T
- JP 2004520284 T
- WO 2006097201 A
- JP 6001945 A
- JP 6313148 A
- JP 7003189 A
- JP 7082454 A
- JP 7118576 A
- JP 2004217727 A
- JP 2005314495 A
- JP 10298493 A
- JP 9241534 A
- JP 2002235028 A
- JP 345109 A
- JP 6346022 A
- JP 2002167545 A
- JP 8324576 A
- JP 9012924 A
- JP 9157581 A
- JP 9059539 A
- JP 2001181558 A
- JP 10183057 A
- JP 2001115080 A
- JP 2001262056 A
- JP 9263729 A
- JP 2001288410 A
- JP 2002069331 A
- JP 2002080781 A
- JP 2003525325 T
- JP 2004162021 A
- JP 2006233010 A
- JP 11514689 T
- JP 2001059068 A
- JP 2006160847 A
- JP 8003502 A
- JP 2006521941 T
- JP 2005533915 T
- JP 11504954 T
- JP 5254277 A
- JP 2006030596 A
- JP 11199808 A
- WO 9967337 A
- JP 2005325150 A
- JP 2005350559 A
- JP 2006008811 A
- JP 2006514130 T
- JP 2006257165 A
- JP 8108650 A
- JP 23111 A
- JP 2004075888 A
- JP 7164729 A
- JP 2006022300 A
- JP 2006070190 A
- JP 2002127596 A
- JP 5117508 A
- JP 7119036 A
- JP 7196631 A
- JP 8188921 A
- JP 10237760 A
- JP 2000054287 A
- JP 2006299428 A
- JP 2006299438 A
- JP 2002322360 A
- JP 2006265770 A
- JP 7076580 A
- JP 2001348785 A
- JP 2003041434 A
- JP 2003239136 A
- WO 032661 A
- JP 10251981 A
- JP 6228816 A
- JP 2005517822 T
- JP 6228818 A
- JP 5009870 A
- JP 10001587 A
- JP 2002212884 A
- JP 2006016710 A
- JP 5148703 A
- JP 2004285516 A
- JP 2004285517 A
- JP 339503 A

- JP 2004011062 A
- JP 11302982 A
- JP 7289097 A
- JP 10001868 A
- JP 5256464 A
- JP 5193037 A
- JP 6114991 A
- JP 11247028 A
- JP 2000144583 A
- JP 7018584 A
- JP 8041785 A
- JP 8193136 A
- JP 8269850 A
- JP 8284063 A
- JP 9057889 A
- JP 9137335 A
- JP 10165045 A
- JP 11247027 A
- JP 7310283 A
- JP 2003528974 T
- JP 2001030861 A
- JP 7324283 A
- JP 8020579 A
- JP 2003147617 A
- JP 10006451 A
- JP 10016152 A
- JP 2002161158 A
- JP 2003049065 A
- JP 2003160724 A
- JP 10205056 A
- JP 8151457 A
- JP 2001172531 A
- JP 7003955 A
- JP 2006266042 A
- JP 8197511 A
- JP 9183159 A
- JP 11236734 A
- JP 11300880 A
- JP 2001009811 A
- JP 2003328523 A
- JP 2002226764 A
- JP 2006306020 A
- JP 9277414 A
- JP 7269016 A
- JP 20032111538 A
- JP 9239921 A
- JP 9254345 A
- JP 10044352 A
- JP 8073825 A
- JP 8207218 A
- JP 2003082608 A
- JP 2001139700 A
- JP 5253559 A
- JP 20052941780 A
- JP 2000226778 A
- JP 211538 A
- JP 2003211606 A
- JP 2004003191 A
- JP 9309260 A
- JP 178625 A
- JP 2002212237 A
- JP 2003266926 A
- JP 2003266927 A
- JP 2004 A
- JP 181813 A
- JP 10203033 A
- JP 2001249430 A
- JP 8258415 A
- JP 9095055 A
- JP 2003145949 A
- JP 2006167996 A
- JP 2002367227 A
- JP 2006301268 A
- JP 2006293155 A
- JP 9306344 A
- JP 2000223271 A
- JP 2005189645 A
- JP 2000174296 A
- JP 2006 A
- JP 282970 A
- JP 2006310461 A
- JP 2006257144 A
- JP 2006210906 A
- JP 2003168814 A
- JP 2005129713 A
- JP 2004227843 A
- JP 2004168057 A
- JP 11040833 A
- JP 129926 A
- JP 2000091611 A
- JP 2005346999 A
- JP 2000091610 A
- JP 11261085 A
- JP 11214736 A
- JP 2001261904 A
- JP 8102296 A
- JP 2000067629 A
- JP 2005353554 A
- JP 5272076 A
- JP 2003239181 A
- JP 2006063162 A
- JP 2007093649 A
- JP 2001214121 A
- JP 214122 A
- JP 2001315263 A
- JP 2003206422 A
- JP 2003025478 A
- JP 2004137457 A
- JP 2005132999 A
- JP 2002036441 A
- JP 10250004 A
- JP 2002036452 A
- JP 2003039607 A
- JP 114355 A
- JP 2002113937 A
- JP 2002293706 A

- JP 2006274179 A
- JP 2005326761 A
- JP 2006335855 A
- JP 10034841 A
- JP 2002114879 A
- JP 2004532306 T
- JP 2004530024 T
- JP 2004525273 T
- JP 10194796 A
- JP 10287804 A
- JP 2000071626 A
- JP 2001159228 A
- JP 2002127310 A
- JP 2002189415 A
- JP 130591 A
- JP 2002307619 A
- JP 2002307845 A
- JP 2006316395 A
- JP 2004352847 A
- JP 2000224942 A
- JP 8208976 A
- JP 8318592 A
- JP 2005504735 A
- JP 2005105032 A
- JP 2005037642 A
- JP 2005055615 A
- JP 9300537 A
- JP 2000025180 A
- JP 2003019776 A
- JP 2005074735 A
- JP 207144 A
- JP 2002543265 T
- JP 2002543266 T
- US 6225384 B
- JP 2004352783 A
- JP 7268253 A
- JP 2003253265 A
- JP 2005105131 A
- JP 2005300962 A
- JP 3915339 B
- JP 2005304340 A
- JP 240544154 A
- JP 2006274197 A
- JP 89697 A
- JP 2000231044 A
- JP 2002527559 T
- JP 320408 A
- JP 2003020966 A
- JP 2002059331 A
- JP 2005023111 A
- JP 2006282970 A
- JP 2003339503 A
- JP 147617 A
- JP 2006266402 A
- JP 2003211538 A
- JP 167996 A
- JP 2004181813 A
- JP 2002207845 A
- JP 2002178625 A
- JP 2006089697 A
- JP 2001214122 A
- JP 2001207144 A
- JP 20025443265 T
- JP 51056620 A
- JP 2008091837 A

**Non-patent literature cited in the description**

- *Bioorganic & Medicinal Chemistry,* 2000, vol. 8, 2095-2103
- *Bioorganic & Medicinal Chemistry Letters,* 2003, vol. 13, 4077-4080
- *Fine Chemical,* May 2004, 28-38
- Kobunshi-yo Kinoseitenkazai no Shin Tenkai. Toray Research Center, 1999, 96-140
- **Seiichi Okatsu.** Kobunshi Tenkazai no Kaihatsu to kankyo Taisaku. C M C Shuppan, 2003, 54-64
- Kobunshi no Rekka · Henshoku mekanizumu to sono Anteika Gijutsu-Nohausyu. Kabushiki kaisha Gijutsu Jyoho Kyokai, 2006
- Methods for Improving the Photostability of Polymers. CMC Publishing, 2000, 85-107
- Basis and Physical Properties of High Functional Coatings. CMC Publishing, 2003, 314-359
- Durability of Polymer Materials and Composite Material Products. CMC Publishing, 2005
- Elongation of Lifetime of Polymer Materials and Environmental Measures. CMC Publishing, 2000
- Plastics Additives Handbook. Hanser Publishers, 238-244
- **Tadahiko Kutsura.** Basic Seminar 2. Science of Plastic Packaging Container. Society of packaging Science & Technology, 2003